# EUROPEAN PATENT APPLICATION

(11) **EP 4 530 361 A2**
(43) Date of publication of application: **02.04.2025**
(21) Application number: 25156516.4
(22) Date of filing: 25.07.2022
(51) Int. Cl.: C12Q 1/6851

(54) **NUCLEIC ACID PROCESSING VIA CIRCULARIZATION**

(30) Priority: 26.07.2021 US 202163225913 P
(62) Divisional of application: 22755048.0
(71) Applicant: 10x Genomics, Inc., Pleasanton, CA 94588-3260 (US)
(72) Inventor: SHASTRY, Shankar, Pleasanton, 94588-3260 (US)
(74) Representative: J A Kemp LLP

(57) **Abstract**

The present disclosure provides methods of processing or analyzing a sample. A method for processing a sample can comprise barcoding a nucleic acid molecule, circularizing the resultant product, and performing an extension reaction, without the need of a template switching molecule. One or more processes of the methods described herein can be performed within a partition, such as a droplet or well.

## Description

### RELATED APPLICATION

This application claims priority to U.S. Provisional Patent Application Serial No. 63/225,913, filed on July 26, 2021, which is incorporated herein by reference in its entirety for all purposes.

### BACKGROUND

A sample can be processed for various purposes, such as identification of a type of moiety within the sample. The sample can be a biological sample. Biological samples can be processed, such as for detection of a disease (*e.g*., cancer) or identification of a particular species. There are various approaches for processing samples, such as polymerase chain reaction (PCR) and sequencing.

Biological samples can be processed within various reaction environments, such as partitions. Partitions can be wells or droplets. Droplets or wells can be employed to process biological samples in a manner that enables the biological samples to be partitioned and processed separately. For example, such droplets can be fluidically isolated from other droplets, enabling accurate control of respective environments in the droplets.

Biological samples in partitions can be subjected to various processes, such as chemical processes or physical processes. Samples in partitions can be subjected to heating or cooling, or chemical reactions, such as to yield species that can be qualitatively or quantitatively processed.

Samples can be processed by barcoding nucleic acid molecules. Such barcoding can be performed using various approaches and can involve the use of nucleic acid barcode molecules and template switching molecules.

### SUMMARY

Recognized herein is a need for barcoding of nucleic acid molecules in a facile manner. The methods, compositions, and kits described herein provide approaches for barcoding nucleic acid molecules via circularization, without the need of template switching molecules. Such approaches can improve depth of long read sequencing.

In one aspect, provided herein is a method comprising: (a) providing (i) a first nucleic acid barcode molecule and a second nucleic acid barcode molecule, and (ii) a first nucleic acid molecule and a second nucleic acid molecule, wherein the first nucleic acid barcode molecule comprises, in a 5' to 3' direction, a first barcode sequence, a first primer sequence, and a second primer sequence, and wherein the second nucleic acid barcode molecule comprises, in a 5' to 3' direction, the second primer sequence, the first primer sequence, and a second barcode sequence; (b) generating (i) a first barcoded molecule using the first nucleic acid molecule and the first nucleic acid barcode molecule, wherein the first barcoded molecule comprises a first sequence complementary to at least a portion of the first nucleic acid molecule and (ii) a second barcoded molecule using the second nucleic acid molecule and the second nucleic acid barcode molecule, wherein the second barcoded molecule comprises a second sequence complementary to at least a portion of the second nucleic acid molecule; (c) circularizing (i) the first barcoded molecule to generate a first circularized molecule, and (ii) the second barcoded molecule to generate a second circularized molecule; and (d) generating (i) a first extension molecule using the first circularized molecule and a first primer molecule comprising a first primer sequence complementary to the first primer sequence, and (ii) a second extension molecule using the second circularized molecule and a second primer molecule comprising a second primer sequence complementary to the second primer sequence and, wherein the first extension molecule comprises a sequence corresponding to the first barcode sequence 5' to the sequence corresponding to the first nucleic acid molecule, and wherein the second extension molecule comprises a sequence corresponding to the second barcode sequence 3' to a sequence corresponding to the second nucleic acid molecule. In some embodiments, the first nucleic acid barcode molecule and the second nucleic acid barcode molecule in (a) are coupled to a bead. In some embodiments, the first barcode sequence and the second barcode sequence comprise a common barcode sequence. In some embodiments, the first nucleic acid barcode molecule comprises a first unique molecular identifier (UMI) sequence and the second nucleic acid barcode molecule comprises a second UMI sequence different from the first UMI sequence. In some embodiments, the common barcode sequence is common to a bead, and wherein the bead comprises a plurality of nucleic acid barcode molecules, wherein at least a subset of the plurality of nucleic acid barcode molecules comprise the common barcode sequence. In some embodiments, the first nucleic acid barcode molecule comprises a first capture sequence configured to capture the first nucleic acid molecule, and wherein the second nucleic acid barcode molecule comprises a second capture sequence configured to capture the second nucleic acid molecule. In some embodiments, the first capture sequence or the second capture sequence comprises a polyT sequence. In some embodiments, the first capture sequence or the second capture sequence comprises a random Nmer sequence. In some embodiments, the first capture sequence or the second capture sequence comprises a target-specific sequence. In some embodiments, the first capture sequence or the second capture sequence comprises a sequence configured to couple to a reporter oligonucleotide of a feature-binding group. In some embodiments, (b) comprises capturing the first nucleic acid molecule using the first capture sequence and extending the first nucleic acid barcode molecule to generate the first barcoded molecule. In some embodiments, (b) further comprises capturing the second nucleic acid molecule using the second capture sequence and extending the second nucleic acid barcode molecule to generate the second barcoded molecule. In some embodiments, (c) is performed using a circularization enzyme. In some embodiments, the circularization enzyme is CircLigase. In some embodiments, (c) is performed using a ligase. In some embodiments, the ligase is a T4 DNA ligase, T7 DNA Ligase, SplintR, T4RNA Ligase, KOD RNA Ligase, a double stranded DNA ligase, or an Acanthocystis turfacea chlorella virus 1-isolated ligase. In some embodiments, (c) is performed using a splint molecule, wherein the splint molecule comprises (i) a first splint sequence complementary to a 5' end of the first barcoded molecule and (ii) a second splint sequence complementary to a 3' end of the first barcoded molecule. In some embodiments, (c) is performed using a splint molecule, wherein the splint molecule comprises (i) a first splint sequence complementary to a 5' end of the second barcoded molecule and (ii) a second splint sequence complementary to a 3' end of the second barcoded molecule. In some embodiments, the first nucleic acid barcode molecule comprises, in the 5' to 3' direction, the first barcode sequence, the first primer sequence, the second primer sequence, and a capture sequence. In some embodiments, (d) comprises using a primer molecule to, at the second primer sequence, perform an extension reaction to generate the first extension molecule. In some embodiments, the first nucleic acid barcode molecule further comprises a unique molecular identifier (UMI) sequence adjacent to the first nucleic acid barcode sequence. In some embodiments, the second nucleic acid barcode molecule comprises, in the 5' to 3' direction, the second primer sequence, the first primer sequence, the second barcode sequence, and a capture sequence. In some embodiments, (d) comprises using a primer molecule to, at the first primer sequence, perform an extension reaction to generate the second extension molecule. In some embodiments, the second nucleic acid barcode molecule further comprises a unique molecular identifier (UMI) sequence adjacent to the second nucleic acid barcode sequence. In some embodiments, (b) is performed in a partition. In some embodiments, prior to (b), the method further comprises partitioning the first nucleic acid barcode molecule, the second nucleic acid barcode molecule, the first nucleic acid molecule, and the second nucleic acid molecule in the partition. In some embodiments, the partition is a droplet or a well. In some embodiments, (c) is performed outside of the partition. In some embodiments, (c) is performed in the partition. In some embodiments, the method comprises partitioning cell or components of a cell comprising the first nucleic acid molecule and the second nucleic acid molecule and the first and second nucleic acid barcode molecules into the partition. In some embodiments, the generating step (b) occurs in the partition or in bulk. In some embodiments, the generating in bulk comprises hybridizing the first nucleic acid molecule to the first nucleic acid barcode molecule in the partition. In some embodiments, the generating in bulk comprises hybridizing the second nucleic acid molecule to the second nucleic acid barcode molecule in the partition. In some embodiments, the generating in bulk comprises generating extensions molecules (*e.g*., the first extension molecule and the second extension molecule in (d)) in bulk. In some embodiments, the generating in the partition comprises hybridizing in the partition and generating extension molecules (*e.g*., the first extension molecule and the second extension molecule in (d)) in the partition.

Provided herein is a method, comprising: (a) generating a barcoded molecule using a nucleic acid molecule and a nucleic acid barcode molecule, wherein the barcoded molecule comprises, in a 5' to 3' direction, a barcode sequence and a sequence complementary to at least a portion of the nucleic acid molecule; (b) circularizing the barcoded molecule to generate a circularized molecule; and (c) generating an extension product from the circularized molecule, wherein the extension product comprises, in a 5' to 3' direction, a first sequence corresponding to the nucleic acid molecule and a second sequence corresponding to the barcode sequence. In some embodiments, (a) is performed in bulk. In some embodiments, (a) is performed in a partition. In some embodiments, the partition is a droplet or a well. In some embodiments, (b) is performed outside of the partition. In some embodiments, (b) is performed in the partition. In some embodiments, the partition comprises a plurality of nucleic acid barcode molecules comprising the nucleic acid barcode molecule. In some embodiments, the plurality of nucleic acid barcode molecules comprises an additional nucleic acid barcode molecule comprising an additional barcode sequence. In some embodiments, the nucleic acid barcode molecule comprises, in a 5' to 3' direction, the barcode sequence, a first primer sequence, a second primer sequence, and the sequence complementary to at least the portion of the nucleic acid molecule. In some embodiments, the additional nucleic acid barcode molecule comprises, in the 5' to 3' direction, the second primer sequence, the first primer sequence, the barcode sequence, and the sequence complementary to at least the portion of the nucleic acid molecule. In some embodiments, the barcode sequence and the additional barcode sequence are identical. In some embodiments, the nucleic acid barcode molecule, or the additional nucleic acid barcode molecule comprises a unique molecular identifier (UMI). In some embodiments, both of the nucleic acid barcode molecule and the additional nucleic acid barcode molecule comprise a unique molecular identifier (UMI). In some embodiments, the UMI is disposed 5' of the barcode sequence of the nucleic acid barcode molecule or the additional barcode sequence of the additional nucleic acid barcode molecule. In some embodiments, the UMI is disposed 3' of the barcode sequence of the nucleic acid barcode molecule or the additional barcode sequence of the additional nucleic acid barcode molecule. In some embodiments, the method further comprises generating an additional barcoded molecule using an additional nucleic acid molecule and the additional nucleic acid barcode molecule, wherein the additional barcoded molecule comprises, in a 5' to 3' direction, the additional barcode sequence and a sequence complementary to at least a portion of the additional nucleic acid molecule. In some embodiments, the nucleic acid barcode molecule is provided on a bead prior to (a). In some embodiments, the nucleic acid barcode molecule and the additional nucleic acid barcode molecule are provided on a bead prior to (a). In some embodiments, the nucleic acid barcode molecule comprises, at a 3' end, a capture sequence configured to hybridize to at least a portion of the nucleic acid molecule. In some embodiments, the capture sequence comprises a polyT sequence. In some embodiments, the capture sequence comprises a random Nmer sequence. In some embodiments, the capture sequence comprises a target-specific sequence. In some embodiments, the capture sequence comprises a sequence configured to couple to a reporter oligonucleotide of a feature-binding group. In some embodiments, (a) comprises capturing the nucleic acid molecule using the capture sequence, and extending the nucleic acid barcode molecule to generate the barcoded molecule. In some embodiments, the barcoded molecule is single stranded. In some embodiments, (b) is performed using a single stranded circularization enzyme. In some embodiments, the circularization enzyme is CircLigase. In some embodiments, (b) is performed using a ligase. In some embodiments, the ligase is a T4 DNA ligase, T7 DNA Ligase, SplintR, Acanthocystis turfacea chlorella virus-isolated ligase, T4RNA Ligase, KOD RNA Ligase, or a double stranded DNA ligase. In some embodiments, (b) is performed using a splint molecule, wherein the splint molecule comprises (i) a first splint sequence complementary to a 5' end of the barcoded molecule and (ii) a second splint sequence complementary to a 3' end of the barcoded molecule.

In another aspect, provided herein is a method comprising: (a) generating a barcoded molecule using a nucleic acid molecule and a nucleic acid barcode molecule, wherein the barcoded molecule comprises a barcode sequence, and, in a 5' to 3' direction:, (i) a first primer sequence, (ii) a second primer sequence, and (iii) a sequence complementary to at least a portion of the nucleic acid molecule; circularizing the barcoded molecule to generate a circularized molecule; and (c) generating an extension product from the circularized molecule, wherein the extension product comprises, the barcode sequence or complement thereof, and, in a 5' to 3' direction, a first sequence corresponding to the second primer sequence, a second sequence corresponding to the nucleic acid molecule, and a third sequence corresponding to the first primer sequence. In some embodiments, the nucleic acid barcode molecule is provided on a bead prior to (a). In some embodiments, the bead comprises a plurality of nucleic acid barcode molecules coupled thereto, including the nucleic acid barcode molecule, wherein at least a subset of the plurality of nucleic acid barcode molecules comprises a common barcode sequence. In some embodiments, at least the subset of the plurality of nucleic acid barcode molecules further comprises a unique molecular identifier (UMI) sequence that differs among the plurality of nucleic acid barcode molecules coupled to the bead. In some embodiments, wherein the nucleic acid barcode molecule comprises, at a 3' end, a capture sequence configured to hybridize to at least a portion of the nucleic acid molecule. In some embodiments, the capture sequence comprises a polyT sequence. In some embodiments, the capture sequence comprises a random Nmer sequence. In some embodiments, the capture sequence comprises a target-specific sequence. In some embodiments, capture sequence comprises a sequence configured to couple to a reporter oligonucleotide of a feature-binding group. In some embodiments, (a) comprises capturing the nucleic acid molecule using the capture sequence, and extending the nucleic acid barcode molecule to generate the barcoded molecule. In some embodiments, the barcoded molecule is single stranded. In some embodiments, (b) is performed using a single stranded circularization enzyme. In some embodiments, the circularization enzyme is CircLigase. In some embodiments, (b) is performed using a ligase. In some embodiments, the ligase is a T4 DNA ligase, T7 DNA Ligase, SplintR, Acanthocystis turfacea chlorella virus-isolated ligase, T4RNA Ligase, KOD RNA Ligase, or a double stranded DNA ligase. In some embodiments, (b) is performed using a splint molecule, wherein the splint molecule comprises (i) a first splint sequence complementary to a 5' end of the barcoded molecule and (ii) a second splint sequence complementary to a 3' end of the barcoded molecule. In some embodiments, the nucleic acid barcode molecule comprises, in the 5' to 3' direction, the barcode sequence, a unique molecular identifier (UMI) sequence, the first primer sequence, the second primer sequence, and a capture sequence. In some embodiments, (c) comprises using a primer molecule to, at the second primer sequence, perform an extension reaction to generate the extension product. In some embodiments, the nucleic acid barcode molecule comprises, in the 5' to 3' direction, the first primer sequence, the second primer sequence, the barcode sequence, a unique molecular identifier (UMI) sequence, and a capture sequence. In some embodiments, (c) comprises using a primer molecule to, at the second primer sequence, perform an extension reaction to generate the extension product. In some embodiments, (a) is performed in bulk. In some embodiments, (a) is performed in a partition. In some embodiments, the partition is a droplet or a well. In some embodiments, (b) is performed outside of the partition. In some embodiments, (b) is performed in the partition.

In another aspect, provided herein is a method comprising: (a) generating a single-stranded barcoded nucleic acid molecule using a nucleic acid molecule and a single-stranded nucleic acid barcode molecule, wherein the single-stranded barcoded nucleic acid molecule comprises a nucleic acid barcode sequence; (b) subsequent to (a), circularizing the single-stranded barcoded nucleic acid molecule by ligating a first end and a second end of the single-stranded barcoded nucleic acid molecule to generate a circularized molecule; and (c) generating a non-concatemeric extension product from the circularized molecule, wherein the non-concatemeric extension product comprises a first sequence corresponding to the nucleic acid molecule and a second sequence corresponding to the nucleic acid barcode sequence. In some embodiments, prior to (a), the nucleic acid barcode molecule is provided on a bead. In some embodiments, the bead comprises a plurality of nucleic acid barcode molecules coupled thereto, including the nucleic acid barcode molecule, wherein at least a subset of the plurality of nucleic acid barcode molecules comprises the nucleic acid barcode sequence. In some embodiments, at least a subset of the plurality of nucleic acid barcode molecules further comprises a unique molecular identifier (UMI) sequence that differs among the plurality of nucleic acid barcode molecules coupled to the bead. In some embodiments, the nucleic acid barcode molecule comprises, at a 3' end, a capture sequence configured to hybridize to at least a portion of the nucleic acid molecule. In some embodiments, the capture sequence comprises a polyT sequence. In some embodiments, the capture sequence comprises a random Nmer sequence. In some embodiments, the capture sequence comprises a target-specific sequence. In some embodiments, the capture sequence comprises a sequence configured to couple to a reporter oligonucleotide of a feature-binding group. In some embodiments, (a) comprises capturing the nucleic acid molecule using the capture sequence, and extending the nucleic acid barcode molecule to generate the single-stranded barcoded nucleic acid molecule. In some embodiments, (b) is performed using a single stranded circularization enzyme. In some embodiments, the circularization enzyme is CircLigase. In some embodiments, (b) is performed using a ligase. In some embodiments, the ligase is a T4 DNA ligase, T7 DNA Ligase, SplintR, Acanthocystis turfacea chlorella virus-isolated ligase, T4RNA Ligase, KOD RNA Ligase, or a double stranded DNA ligase. In some embodiments, (b) is performed using a splint molecule, wherein the splint molecule comprises (i) a first splint sequence complementary to a 5' end of the single-stranded barcoded molecule and (ii) a second splint sequence complementary to a 3' end of the single-stranded barcoded molecule. In some embodiments, the nucleic acid barcode molecule comprises, in the 5' to 3' direction, the nucleic acid barcode sequence, a first primer sequence, a second primer sequence, and a capture sequence. In some embodiments, (c) comprises using a primer molecule to, at the second primer sequence, perform an extension reaction to generate the extension product. In some embodiments, the nucleic acid barcode molecule further comprises a unique molecular identifier (UMI) sequence adjacent to the nucleic acid barcode sequence. In some embodiments, the nucleic acid barcode molecule comprises, in the 5' to 3' direction, a second primer sequence, a first primer sequence, the nucleic acid barcode sequence, and a capture sequence. In some embodiments, (c) comprises using a primer molecule to, at the first primer sequence, perform an extension reaction to generate the extension product. In some embodiments, the nucleic acid barcode molecule further comprises a unique molecular identifier (UMI) sequence adjacent to the nucleic acid barcode sequence. In some embodiments, (a) is performed in bulk. In some embodiments, (a) is performed in a partition. In some embodiments, the partition is a droplet or a well. In some embodiments, (b) is performed outside of the partition. In some embodiments, (b) is performed in the partition. In some embodiments, the single-stranded nucleic acid barcode molecule comprises a capture sequence complementary to a portion of the nucleic acid molecule. In some embodiments, (a) comprises (i) hybridizing the nucleic acid molecule to the capture sequence and performing a nucleic acid reaction to generate a double-stranded barcoded nucleic acid molecule, and (ii) generating the single-stranded barcoded nucleic acid molecule from the double-stranded barcoded nucleic acid molecule. In some embodiments, the nucleic acid reaction comprises reverse transcription. In some embodiments, the reverse transcription is performed using a reverse transcriptase selected from the group consisting of thermostable group II intron reverse transcriptase (TGIRT), and Marathon reverse transcriptase. In some embodiments, the nucleic acid barcode molecule comprises, in the 5' to 3' direction, the barcode sequence, a first primer sequence, a second primer sequence, and a capture sequence. In some embodiments, the nucleic acid barcode molecule comprises, adjacent to the barcode sequence, a unique molecular identifier (UMI) sequence. In some embodiments, (c) comprises using a primer molecule to, at the second primer sequence, perform an extension reaction to generate the extension product. In some embodiments, the nucleic acid barcode molecule comprises, in the 5' to 3' direction, a first primer sequence, a second primer sequence, the barcode sequence, and a capture sequence. In some embodiments, the nucleic acid barcode molecule comprises, adjacent to the barcode sequence, a unique molecular identifier (UMI) sequence.

In yet another aspect, provided herein is a kit comprising: a first nucleic acid barcode molecule comprising, in a 5' to 3' direction, a first barcode sequence, a first primer sequence, and a second primer sequence; a second nucleic acid barcode molecule comprising, in a 5' to 3' direction, the second primer sequence, the first primer sequence, and a second barcode sequence; a first primer molecule comprising a first sequence complementary to the first primer sequence; and a second primer molecule comprising a second sequence complementary to the second primer sequence. In some embodiments, the kit further comprises a bead, wherein the bead comprises the first nucleic acid barcode molecule and the second nucleic acid barcode molecule coupled thereto. In some embodiments, the bead comprises a plurality of first nucleic acid barcode molecules and a plurality of second nucleic acid barcode molecules, wherein the plurality of first nucleic acid barcode molecules comprises the first nucleic acid barcode molecule and comprises the first barcode sequence, wherein the plurality of second nucleic acid barcode molecules comprises the second nucleic acid barcode molecule and comprises the second barcode sequence. In some embodiments, the kit further comprises a plurality of beads, wherein the plurality of beads comprises the bead, wherein the plurality of beads comprises a plurality of first nucleic acid barcode molecules and a plurality of second nucleic acid barcode molecules coupled thereto. In some embodiments, the first nucleic acid barcode molecule is releasably coupled to the bead. In some embodiments, the first barcode sequence and the second barcode sequence comprises a common sequence that is common to the bead. In some embodiments, the kit further comprises a first bead coupled to the first nucleic acid barcode molecule and a second bead coupled to the second nucleic acid barcode molecule. In some embodiments, the kit further comprises a plurality of first beads and a plurality of second beads, wherein the plurality of first beads comprises (i) the first bead and (ii) a plurality of first nucleic acid barcode molecules coupled thereto, wherein the plurality of second beads comprises (i) the second bead and (ii) a plurality of second nucleic acid barcode molecules coupled thereto. In some embodiments, the kit further comprises a plurality of first nucleic acid barcode molecules and a plurality of second nucleic acid barcode molecules, wherein the plurality of first nucleic acid barcode molecules comprises the first nucleic acid barcode molecule and comprises the first barcode sequence, wherein the plurality of second nucleic acid barcode molecules comprises the second nucleic acid barcode molecule and comprises the second barcode sequence. In some embodiments, the kit further comprises a plurality of first primer molecules and a plurality of second primer molecules, wherein the plurality of first primer molecules comprises the first primer molecule, wherein the plurality of second primer molecules comprises the second primer molecule. In some embodiments, the first barcode sequence and the second barcode sequence comprises a common sequence. In some embodiments, the first nucleic acid barcode molecule further comprises a first unique molecular identifier (UMI) sequence and the second nucleic acid barcode molecule further comprises a second UMI sequence different from the first UMI sequence. In some embodiments, the first nucleic acid barcode molecule and the second nucleic acid barcode molecule comprise, at a 3' end, a capture sequence. In some embodiments, the capture sequence comprises a polyT sequence. In some embodiments, the capture sequence comprises a random Nmer sequence. In some embodiments, the capture sequence comprises a target-specific sequence. In some embodiments, the kit further comprises a feature-binding group comprising a reporter oligonucleotide. In some embodiments, the capture sequence comprises a sequence configured to couple to the reporter oligonucleotide. In some embodiments, the kit further comprises an enzyme configured to circularize a nucleic acid molecule. In some embodiments, the enzyme is a single stranded circularization enzyme. In some embodiments, the enzyme is CircLigase. In some embodiments, the kit further comprises a ligase. In some embodiments, the ligase is a T4 DNA ligase, T7 DNA Ligase, SplintR, Acanthocystis turfacea chlorella virus-isolated ligase, T4RNA Ligase, KOD RNA Ligase, or a double stranded DNA ligase. In some embodiments, the kit further comprises a splint molecule comprising a splint sequence complementary to a sequence of the first nucleic acid barcode molecule or the second nucleic acid barcode molecule. In some embodiments, the kit further comprises reagents for performing any one or more of: a nucleic acid extension, ligation, and amplification reaction. In some embodiments, the kit further comprises a reverse transcriptase. In some embodiments, the reverse transcriptase is a thermostable group II intron reverse transcriptase (TGIRT), and Marathon reverse transcriptase. In some embodiments, the first nucleic acid barcode molecule comprises, in the 5' to 3' direction, (i) a common barcode sequence and (ii) a first unique molecular identifier (UMI) sequence, the first primer sequence, the second primer sequence, and a capture sequence. In some embodiments, the second nucleic acid barcode molecule comprises, in the 5' to 3' direction, the second primer sequence, the first primer sequence, the common barcode sequence and a second UMI sequence different form the first UMI sequence, and the capture sequence. In some embodiments, the kit further comprises instructions for performing any of the methods provided herein.

Provided herein is a composition, comprising: a particle comprising (i) a first nucleic acid barcode molecule comprising, in a 5' to 3' direction, a first barcode sequence, a first primer sequence, and a second primer sequence; and (ii) a second nucleic acid barcode molecule comprising, in a 5' to 3' direction, the second primer sequence, the first primer sequence, and a second barcode sequence. In some embodiments, the particle is a bead. In some embodiments, the composition further comprises a partition, wherein the partition comprises the particle. In some embodiments, the partition further comprises a reverse transcriptase. In some embodiments, the reverse transcriptase is a thermostable group II intron reverse transcriptase (TGIRT), and Marathon reverse transcriptase. In some embodiments, the partition further comprises a ligase. In some embodiments, the ligase is a CircLigase, T4 DNA ligase, T7 DNA Ligase, SplintR, Acanthocystis turfacea chlorella virus-isolated ligase, T4RNA Ligase, KOD RNA Ligase, or a double stranded DNA ligase. In some embodiments, the partition further comprises a splint molecule comprising a splint sequence complementary to a sequence of the first nucleic acid barcode molecule. In some embodiments, the partition further comprises a splint molecule comprising a splint sequence complementary to a sequence of the second nucleic acid barcode molecule. In some embodiments, the partition further comprises a first primer molecule comprising a first sequence complementary to the first primer sequence and a second primer molecule comprising a second sequence complementary to the second primer sequence. In some embodiments, the partition is a droplet or a well.

An aspect of the present disclosure provides a non-transitory computer readable medium comprising machine executable code that, upon execution by one or more computer processors, implements any of the methods above or elsewhere herein.

Another aspect of the present disclosure provides a system comprising one or more computer processors and computer memory coupled thereto. The computer memory comprises machine executable code that, upon execution by the one or more computer processors, implements any of the methods above or elsewhere herein.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

### BRIEF DESCRIPTION OF THE DRAWINGS

The novel features of the present disclosure are set forth with particularity in the appended claims. A better understanding of the features and advantages of the present disclosure will be obtained by reference to the following detailed description that sets forth illustrative embodiments, in which the principles of the present disclosure are utilized, and the accompanying drawings (also "Figure" and "FIG." herein), of which:
**FIG. 1** schematically illustrates a non-limiting example of a microfluidic channel structure for partitioning individual biological particles or analyte carriers.
**FIG. 2** schematically illustrates a non-limiting example of a microfluidic channel structure for the controlled partitioning of beads into discrete droplets.
**FIG. 3** schematically illustrates a non-limiting example of a barcode carrying bead.
**FIG. 4** schematically illustrates a non-limiting example of a barcode carrying bead coupled to an analyte via a capture sequence.
**FIG. 5** schematically illustrates a non-limiting example microwell array.
**FIG. 6** schematically illustrates a non-limiting example workflow for processing nucleic acid molecules.
**FIG. 7A** schematically illustrates a non-limiting example workflow for barcoding nucleic acid molecules via circularization.
**FIG. 7B** schematically illustrates a non-limiting example workflow for barcoding nucleic acid molecules via circularization.
**FIG. 8** schematically illustrates a non-limiting example workflow for barcoding nucleic acid molecules via circularization using a splint molecule.
**FIG. 9** schematically illustrates a non-limiting example workflow for barcoding nucleic acid molecules.
**FIG. 10** schematically illustrates a non-limiting example workflow for barcoding nucleic acid molecules.
**FIG. 11** schematically illustrates exemplary labelling agents with nucleic acid molecules attached thereto.
**FIG. 12A** schematically illustrates a non-limiting example of labelling agents.
**FIG. 12B** schematically illustrates a non-limiting example workflow for processing nucleic acid molecules.
**FIG. 12C** schematically illustrates a non-limiting example workflow for processing nucleic acid molecules.
**FIG. 13** schematically illustrates a non-limiting example of a barcode-carrying bead.
**FIG. 14** schematically illustrates a computer system that is programmed or otherwise configured to implement methods provided herein.
**FIG. 15** is a gel electrophoresis data of the circularized molecules that were ligated using an 80 nM concentration of the splint molecule. Lanes 1-5 of the gel (excluding the DNA ladder) indicate an input cDNA concentration of 12 nM, 24 nM, 48 nM, 96 nM, and 12 nM with no splint (negative control), respectively.
**FIG. 16** schematically illustrates a non-limiting example of a barcode carrying bead.
**FIG. 17** schematically illustrates a non-limiting example of a microfluidic channel structure for delivering barcode carrying beads to droplets.

### DETAILED DESCRIPTION

While various embodiments of the present disclosure have been shown and described herein, it will be obvious to those skilled in the art that such embodiments are provided by way of example only. Numerous variations, changes, and substitutions can occur to those skilled in the art without departing from the present disclosure. It should be understood that various alternatives to the embodiments of the present disclosure described herein can be employed.

Where values are described as ranges, it will be understood that such disclosure includes the disclosure of all possible sub-ranges within such ranges, as well as specific numerical values that fall within such ranges irrespective of whether a specific numerical value or specific sub-range is expressly stated.

### Definition

All terms are intended to be understood as they would be understood by a person skilled in the art. Unless defined otherwise, all technical and scientific terms used herein have the same meaning as commonly understood by one of ordinary skill in the art to which the disclosure pertains.

The following definitions supplement those in the art and are directed to the current application and are not to be imputed to any related or unrelated cases, e.g., to any commonly owned patent or application. Although any methods and materials similar or equivalent to those described herein can be used in the practice for testing of the present disclosure, the preferred materials and methods are described herein. Accordingly the terminology used herein is for the purpose of describing particular embodiments only, and is not intended to be limiting.

The terms "a," "an," and "the," as used herein, generally refers to singular and plural references unless the context clearly dictates otherwise.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, the use of these terms in the specification does not by itself connote any required priority, precedence, or order.

The term "barcode," as used herein, generally refers to a label, or identifier, that conveys or is capable of conveying information about an analyte. A barcode can be part of an analyte. A barcode can be independent of an analyte. A barcode can be a tag attached to an analyte (*e.g*., nucleic acid molecule) or a combination of the tag in addition to an endogenous characteristic of the analyte (*e.g*., size of the analyte or end sequence(s)). A barcode can be unique. Barcodes can have a variety of different formats. For example, barcodes can include: polynucleotide barcodes; random nucleic acid and/or amino acid sequences; and synthetic nucleic acid and/or amino acid sequences. A barcode can be attached to an analyte in a reversible or irreversible manner. A barcode can be added to, for example, a fragment of a deoxyribonucleic acid (DNA) or ribonucleic acid (RNA) sample before, during, and/or after sequencing of the sample. Barcodes can allow for identification and/or quantification of individual sequencing-reads.

The term "corresponding to," as used herein, generally refers to a relation of a molecule to another molecule. For example, a nucleic acid molecule can comprise a sequence that corresponds to an additional sequence of an additional nucleic acid molecule; such sequences can be the same sequence or they can be different. In some instances, a nucleic acid sequence that corresponds to an additional sequence of an additional nucleic acid molecule can comprise the same sequence or a complementary sequence to the additional sequence. In some instances, a nucleic acid sequence that corresponds to an additional sequence of another nucleic acid molecule can comprise a portion of the additional sequence. In some instances, a nucleic acid sequence that corresponds to an additional sequence of another nucleic acid molecule can comprise the additional sequence (or portion thereof), along with other sequences that are not present in the additional sequence. The nucleic acid molecule can be derived from the additional nucleic acid molecule; for example, the nucleic acid molecule can be a complement or reverse complement of the additional nucleic acid molecule or the nucleic acid molecule can be generated from an extension reaction or amplification of the additional nucleic acid molecule.

The term "real time," as used herein, can refer to a response time of less than about 1 second, for example, a tenth of a second, a hundredth of a second, a millisecond, or less. The response time can be greater than 1 second. In some instances, real time can refer to simultaneous or substantially simultaneous processing, detection or identification.

The term "subject," as used herein, generally refers to an animal, such as a mammal (*e.g*., human) or avian (*e.g*., bird), or other organism, such as a plant. For example, the subject can be a vertebrate, a mammal, a rodent *(e.g.,* a mouse), a primate, a simian or a human. Animals can include, but are not limited to, farm animals, sport animals, and pets. A subject can be a healthy or asymptomatic individual, an individual that has or is suspected of having a disease (*e.g*., cancer) or a pre-disposition to the disease, and/or an individual that is in need of therapy or suspected of needing therapy. A subject can be a patient. A subject can be a microorganism or microbe (e.g., bacteria, fungi, archaea, viruses).

The term "genome," as used herein, generally refers to genomic information from a subject, which can be, for example, at least a portion or an entirety of a subject's hereditary information. A genome can be encoded either in DNA or in RNA. A genome can comprise coding regions (*e.g*., that code for proteins) as well as non-coding regions. A genome can include the sequence of all chromosomes together in an organism. For example, the human genome ordinarily has a total of 46 chromosomes. The sequence of all of these together can constitute a human genome.

The terms "adaptor(s)", "adapter(s)" and "tag(s)" can be used synonymously. An adaptor or tag can be coupled to a polynucleotide sequence to be "tagged" by any approach, including ligation, hybridization, or other approaches.

The term "sequencing," as used herein, generally refers to methods and technologies for determining the sequence of nucleotide bases in one or more polynucleotides. The polynucleotides can be, for example, nucleic acid molecules such as deoxyribonucleic acid (DNA) or ribonucleic acid (RNA), including variants or derivatives thereof (e.g., single stranded DNA). Sequencing can be performed by various systems currently available, such as, without limitation, a sequencing system by Illumina^{®}, Pacific Biosciences (PacBio^{®}), Oxford Nanopore^{®}, or Life Technologies (Ion Torrent^{®}). Alternatively or in addition, sequencing can be performed using nucleic acid amplification, polymerase chain reaction (PCR) (*e.g.,* digital PCR, quantitative PCR, or real time PCR), or isothermal amplification. Such systems can provide a plurality of raw genetic data corresponding to the genetic information of a subject (*e.g*., human), as generated by the systems from a sample provided by the subject. In some examples, such systems provide sequencing reads (also "reads" herein). A read can include a string of nucleic acid bases corresponding to a sequence of a nucleic acid molecule that has been sequenced. In some situations, systems and methods provided herein can be used with proteomic information.

The term "bead," as used herein, generally refers to a particle. The bead can be a solid or semi-solid particle. The bead can be a gel bead. The gel bead can include a polymer matrix (*e.g*., matrix formed by polymerization or cross-linking). The polymer matrix can include one or more polymers (*e.g*., polymers having different functional groups or repeat units). Polymers in the polymer matrix can be randomly arranged, such as in random copolymers, and/or have ordered structures, such as in block copolymers. Cross-linking can be via covalent, ionic, or inductive, interactions, or physical entanglement. The bead can be a macromolecule. The bead can be formed of nucleic acid molecules bound together. The bead can be formed via covalent or non-covalent assembly of molecules (*e.g*., macromolecules), such as monomers or polymers. Such polymers or monomers can be natural or synthetic. Such polymers or monomers can be or include, for example, nucleic acid molecules (*e.g*., DNA or RNA). The bead can be formed of a polymeric material. The bead can be magnetic or non-magnetic. The bead can be rigid. The bead can be flexible and/or compressible. The bead can be disruptable or dissolvable. The bead can be a solid particle (*e.g*., a metal-based particle including but not limited to iron oxide, gold or silver) covered with a coating comprising one or more polymers. Such coating can be disruptable or dissolvable.

As used herein, the term "barcoded nucleic acid molecule", interchangeably used herein with "barcoded molecule", generally refers to a nucleic acid molecule that results from, for example, the processing of a nucleic acid barcode molecule with a nucleic acid sequence (*e.g*., nucleic acid sequence complementary to a nucleic acid primer sequence encompassed by the nucleic acid barcode molecule). The nucleic acid sequence can be a targeted sequence or a non-targeted sequence. For example, in the methods and systems described herein, hybridization and reverse transcription of a nucleic acid molecule (*e.g*., a messenger RNA (mRNA) molecule) of a cell with a nucleic acid barcode molecule (*e.g*., a nucleic acid barcode molecule containing a barcode sequence and a nucleic acid primer sequence complementary to a nucleic acid sequence of the mRNA molecule) results in a barcoded nucleic acid molecule that has a sequence corresponding to the nucleic acid sequence of the mRNA and the barcode sequence (or a reverse complement thereof). A barcoded nucleic acid molecule can serve as a template, such as a template polynucleotide, that can be further processed (*e.g*., amplified) and sequenced to obtain the target nucleic acid sequence. For example, in the methods and systems described herein, a barcoded nucleic acid molecule can be further processed (*e.g*., amplified) and sequenced to obtain the nucleic acid sequence of the mRNA.

The term "sample," as used herein, generally refers to a biological sample of a subject. The biological sample can comprise any number of macromolecules, for example, cellular macromolecules. The sample can be a cell sample. The sample can be a cell line or cell culture sample. The sample can include one or more cells. The sample can include one or more microbes. The biological sample can be a nucleic acid sample or protein sample. The biological sample can also be a carbohydrate sample or a lipid sample. The biological sample can be derived from another sample. The sample can be a tissue sample, such as a biopsy, core biopsy, needle aspirate, or fine needle aspirate. The sample can be a fluid sample, such as a blood sample, urine sample, or saliva sample. The sample can be a skin sample. The sample can be a cheek swab. The sample can be a plasma or serum sample. The sample can be a cell-free or cell free sample. A cell-free sample can include extracellular polynucleotides. Extracellular polynucleotides can be isolated from a bodily sample that can be selected from the group consisting of blood, plasma, serum, urine, saliva, mucosal excretions, sputum, stool and tears.

The term "biological particle" or "analyte carrier" as used herein, generally refers to a discrete biological system derived from a biological sample. The biological particle can be a macromolecule. The biological particle can be a small molecule. The biological particle can be a virus. The biological particle can be a cell or derivative of a cell. The biological particle can be an organelle. The biological particle can be a rare cell from a population of cells. The biological particle can be any type of cell, including without limitation prokaryotic cells, eukaryotic cells, bacterial, fungal, plant, mammalian, or other animal cell type, mycoplasmas, normal tissue cells, tumor cells, or any other cell type, whether derived from single cell or multicellular organisms. The biological particle can be a constituent of a cell. The biological particle can be or can include DNA, RNA, organelles, proteins, or any combination thereof. The biological particle can be or can include a matrix (*e.g*., a gel or polymer matrix) comprising a cell or one or more constituents from a cell (*e.g*., cell bead), such as DNA, RNA, organelles, proteins, or any combination thereof, from the cell. The biological particle can be obtained from a tissue of a subject. The biological particle can be a hardened cell. Such hardened cell may or may not include a cell wall or cell membrane. The biological particle can include one or more constituents of a cell, but may not include other constituents of the cell. An example of such constituents is a nucleus or an organelle. A cell can be a live cell. The live cell can be capable of being cultured, for example, being cultured when enclosed in a gel or polymer matrix, or cultured when comprising a gel or polymer matrix.

The term "macromolecular constituent," as used herein, generally refers to a macromolecule contained within or from a biological particle. The macromolecular constituent can comprise a nucleic acid. In some cases, the biological particle can be a macromolecule. The macromolecular constituent can comprise DNA. The macromolecular constituent can comprise RNA. The RNA can be coding or non-coding. The RNA can be messenger RNA (mRNA), ribosomal RNA (rRNA) or transfer RNA (tRNA), for example. The RNA can be a transcript. The RNA can be small RNA that are less than 200 nucleic acid bases in length, or large RNA that are greater than 200 nucleic acid bases in length. Small RNAs can include 5.8S ribosomal RNA (rRNA), 5S rRNA, transfer RNA (tRNA), microRNA (miRNA), small interfering RNA (siRNA), small nucleolar RNA (snoRNAs), Piwi-interacting RNA (piRNA), tRNA-derived small RNA (tsRNA) and small rDNA-derived RNA (srRNA). The RNA can be double-stranded RNA or single-stranded RNA. The RNA can be circular RNA. The macromolecular constituent can comprise a protein. The macromolecular constituent can comprise a peptide. The macromolecular constituent can comprise a polypeptide.

The term "molecular tag," as used herein, generally refers to a molecule capable of binding to a macromolecular constituent. The molecular tag can bind to the macromolecular constituent with high affinity. The molecular tag can bind to the macromolecular constituent with high specificity. The molecular tag can comprise a nucleotide sequence. The molecular tag can comprise a nucleic acid sequence. The nucleic acid sequence can be at least a portion or an entirety of the molecular tag. The molecular tag can be a nucleic acid molecule or can be part of a nucleic acid molecule. The molecular tag can be an oligonucleotide or a polypeptide. The molecular tag can comprise a DNA aptamer. The molecular tag can be or comprise a primer. The molecular tag can be, or comprise, a protein. The molecular tag can comprise a polypeptide. The molecular tag can be a barcode.

The term "partition," as used herein, generally, refers to a space or volume that can be suitable to contain one or more species or conduct one or more reactions. A partition can be a physical compartment, such as a droplet or well. The partition can isolate space or volume from another space or volume. The droplet can be a first phase (*e.g*., aqueous phase) in a second phase (*e.g*., oil) immiscible with the first phase. The droplet can be a first phase in a second phase that does not phase separate from the first phase, such as, for example, a capsule or liposome in an aqueous phase. A partition can comprise one or more other (inner) partitions. In some cases, a partition can be a virtual compartment that can be defined and identified by an index (*e.g*., indexed libraries) across multiple and/or remote physical compartments. For example, a physical compartment can comprise a plurality of virtual compartments.

Provided herein are methods, compositions, and kits for processing nucleic acid molecules (*e.g*., template nucleic acid molecules, sample nucleic acid molecules, nucleic acid analytes, etc.) using nucleic acid barcode molecules comprising a barcode sequence and one or more primer sequences. The nucleic acid molecules can be barcoded using the nucleic acid barcode molecules, circularized, and subjected to one or more nucleic acid reactions (*e.g*., nucleic acid extension, amplification) to generate one or more nucleic acid extension products. The nucleic acid extension products (interchangeably used herein with "extension molecules") can comprise a sequence corresponding to the barcode sequence and the one or more primer sequences. In some instances, the order of the barcode sequence and the one or more primer sequences (*e.g.*, in a 5' to 3' direction) changes following circularization and the one or more nucleic acid reactions. Such methods, compositions, and kits can be useful in barcoding nucleic acid molecules without the need for template switching and can improve target sampling (*e.g*., by increasing sample nucleic acid coverage, range, and increasing sampling of incompletely transcribed RNA molecules etc.). For example, the methods, compositions, and kits disclosed herein are useful for providing 5' sequence information for RNA molecules or their derivatives, without the need for template switch reactions which can be inefficient or introduce biases into the barcoding reaction (see, *e.g*., Wulf, Madalee G et al. "Non-templated addition and template switching by Moloney murine leukemia virus (MMLV)-based reverse transcriptases co-occur and compete with each other." The Journal of Biological Chemistry vol. 294,48 (2019): 18220-18231. doi:10.1074/jbc.RA119.010676). Furthermore, the methods, compositions, and kits disclosed herein are useful for providing both 5' and 3' sequence information for RNA molecules or their derivatives from one or more biological particles (*e.g*., one or more single cells, single cell beads, or single nuclei). In addition, the circularization approaches disclosed herein can increase the stability of the barcoded products generated by one or more barcoding reactions disclosed herein.

### Nucleic Acid Barcoding and Circularization

A method of the present disclosure can comprise, *inter alia,* providing nucleic acid barcode molecules and nucleic acid molecules (*e.g*., sample nucleic acids, nucleic acid analytes, target nucleic acids, etc.), and using the nucleic acid barcode molecules and the nucleic acid molecules to generate barcoded molecules (e.g., barcoded nucleic acid molecules), *e.g*., via coupling or attaching of the nucleic acid barcode molecules to the nucleic acid molecules. The resultant barcoded nucleic acid molecules can be subjected to conditions sufficient to circularize the barcoded molecules (e.g., barcoded nucleic acid molecules), thereby generating circularized molecules. The circularized molecules can be subjected to one or more nucleic acid extension molecules to generate extension molecules. The extension molecules can optionally be further amplified, *e.g*., using polymerase chain reaction (PCR) or linear amplification, and characterized, *e.g*., via sequencing. The methods described herein can facilitate gene expression profiling with single cell resolution, for example, via barcoding of nucleic acid molecules using two types of nucleic acid barcode molecules, circularization of the barcoded molecules (e.g., barcoded nucleic acid molecules), and extending the circularized molecules. One or more methods, compositions, and kits provided herein can be useful in barcoding nucleic acid molecules without the use of template switching reactions or template switch oligonucleotides. In some instances, the methods described herein can result in extension molecules that comprise, from a 5' to 3' direction, a plurality of sequences in a particular arrangement, which can differ from the arrangement of the plurality of sequences of the barcoded molecule (*e.g*., prior to the circularization or extension). For example, the arrangement of a subset of the sequences (*e.g*., in a 5' to 3' direction) of the barcoded molecule (e.g., barcoded nucleic acid molecule) can change after and performing the nucleic acid extension reaction.

A method of the present disclosure can comprise providing (i) a first nucleic acid barcode molecule and a second nucleic acid barcode molecule and a (ii) a first nucleic acid molecule (*e.g*., a nucleic acid analyte, a target nucleic acid, etc.) and a second nucleic acid molecule (*e.g*., a nucleic acid analyte, a target nucleic acid, etc.). The first nucleic acid barcode molecule can comprise, in a 5' to 3' direction, a first barcode sequence, a first primer sequence, and a second primer sequence, and the second nucleic acid barcode molecule can comprise, in a 5' to 3' direction, the second primer sequence, the first primer sequence, and a second barcode sequence. The method can additionally comprise generating a first barcoded molecule (e.g., first barcoded nucleic acid molecule) using the first nucleic acid molecule and the first nucleic acid barcode molecule and generating a second barcoded molecule (e.g., second barcoded nucleic acid molecule) using the second nucleic acid molecule and the second nucleic acid barcode molecule. Such generation of barcoded molecules (e.g., barcoded nucleic acid molecules) can be performed, for example, by coupling (*e.g*., via hybridization and/or ligation) at least a portion of the nucleic acid molecules to a sequence of the nucleic acid barcode molecules, as is described elsewhere herein. Optionally, a nucleic acid extension reaction can be performed to generate the barcoded molecules (e.g., barcoded nucleic acid molecules). The first barcoded molecule and the second barcoded molecule can be circularized to generate a first circularized molecule and a second circularized molecule, respectively. The first circularized molecule and the second circularized molecule can be subjected to a nucleic acid extension reaction using primers (*e.g*., a first primer molecule comprising a sequence complementary to the first primer sequence and/or a second primer molecule comprising the same sequence or complementary sequence to the second primer sequence) to generate a first extension molecule and a second extension molecule. The first extension molecule can comprise a sequence corresponding to the first barcode sequence that is 5' to the sequence corresponding to the first nucleic acid molecule. The second extension molecule can comprise a sequence corresponding to the second barcode sequence that is 3' to a sequence corresponding to the second nucleic acid molecule. In some embodiments, one or more operations can be performed in a partition (*e.g*., droplet, well).

The nucleic acid molecules analyzed by the methods described herein can comprise a single-stranded or a double-stranded nucleic acid molecule. A double-stranded nucleic acid molecule can be completely or partially denatured to provide access to a target region (*e.g*., a target sequence) of a strand of the nucleic acid molecule. Denaturation can be achieved by, for example, adjusting the temperature or pH of a solution comprising the nucleic acid molecule; using a chemical agent such as formamide, guanidine, sodium salicylate, dimethyl sulfoxide, propylene glycol, urea, or an alkaline agent (*e.g.,* NaOH); or using mechanical agitation (*e.g*., centrifuging or vortexing a solution including the nucleic acid molecule).

The nucleic acid molecules can comprise a target nucleic acid molecule or a nucleic acid analyte. For example, the nucleic acid molecules (*e.g*., the first nucleic acid molecule or the second nucleic acid molecule) can comprise an RNA molecule. The RNA molecule can be, for example, a transfer RNA (tRNA) molecule, ribosomal RNA (rRNA) molecule, mitochondrial RNA (mtRNA) molecule, messenger RNA (mRNA) molecule, non-coding RNA molecule, synthetic RNA molecule, or another type of RNA molecule. For example, the RNA molecule can be an mRNA molecule. In some cases, the nucleic acid molecule can be a viral or pathogenic RNA. In some cases, the nucleic acid molecule can be a synthetic nucleic acid molecule previously introduced into or onto a cell. For example, the nucleic acid molecule can comprise a plurality of barcode sequences, and two or more barcode sequences can be target regions of the nucleic acid molecule.

The nucleic acid molecule (*e.g.,* RNA molecule) can comprise one or more features selected from the group consisting of a 5' cap structure, an untranslated region (UTR), a 5' triphosphate moiety, a 5' hydroxyl moiety, a Kozak sequence, a Shine-Dalgarno sequence, a coding sequence, a codon, an intron, an exon, an open reading frame, a regulatory sequence, an enhancer sequence, a silencer sequence, a promoter sequence, and a poly(A) sequence (*e.g.,* a poly(A) tail). For example, the nucleic acid molecule can comprise one or more features selected from the group consisting of a 5' cap structure, an untranslated region (UTR), a Kozak sequence, a Shine-Dalgarno sequence, a coding sequence, and a poly(A) sequence (*e.g.,* a poly(A) tail).

Features of the nucleic acid molecules can have any useful characteristics. A 5' cap structure can comprise one or more nucleoside moieties joined by a linker such as a triphosphate (ppp) linker. A 5' cap structure can comprise naturally occurring nucleoside and/or non-naturally occurring (*e.g*., modified) nucleosides. For example, a 5' cap structure can comprise a guanine moiety or a modified (*e.g*., alkylated, reduced, or oxidized) guanine moiety such as a 7-methylguanylate (m⁷G) cap. Examples of 5' cap structures include, but are not limited to, m⁷GpppG, m⁷Gpppm⁷G, m⁷GpppA, m⁷GpppC, GpppG, m^{2,7}GpppG, m^{2,2,7}GpppG, and anti-reverse cap analogs such as m^{7,2'Ome}GpppG, m^{7,2'd}GpppG, m^{7,3'Ome}GpppG, and m^{7,3'd}GpppG. An untranslated region (UTR) can be a 5' UTR or a 3' UTR. A UTR can include any number of nucleotides. For example, a UTR can comprise at least 3, 5, 7, 10, 20, 30, 40, 50, 60, 70, 80, 90, 100, or more nucleotides. In some cases, a UTR can comprise fewer than 20 nucleotides. In other cases, a UTR can comprise at least 100 nucleotides, such as more than 200, 300, 400, 500, 600, 700, 800, 900, or 1000 nucleotides. Similarly, a coding sequence can include any number of nucleotides, such as at least 3, 5, 10, 20, 30, 40, 50, 60, 70, 80, 90, 100, or more nucleotides. A UTR, coding sequence, or other sequence of a nucleic acid molecule can have any nucleotide or base content or arrangement. For example, a sequence of a nucleic acid molecule can comprise any number or concentration of guanine, cytosine, uracil, and adenine bases. A nucleic acid molecule can also include non-naturally occurring (*e.g*., modified) nucleosides. A modified nucleoside can comprise one or more modifications (*e.g*., alkylations, hydroxylation, oxidation, or other modification) in its nucleobase and/or sugar moieties.

When more than one nucleic acid molecule is provided, (*e.g*., a first nucleic acid molecule and a second nucleic acid molecule), the nucleic acid molecules can be the same or different types and can comprise the same or different sequences. For example, the first nucleic acid molecule can be a RNA molecule and the second nucleic acid molecule can be a DNA molecule. In another example, the first nucleic acid molecule and the second nucleic acid molecule can both be RNA molecules that are identical, or the first nucleic acid molecule and the second nucleic acid molecule can both be RNA molecules comprising different sequences.

The nucleic acid molecules (*e.g*., RNA molecules, such as mRNA molecules) of a sample can be included within a biological particle (*e.g.,* cell, cell nucleus, or cell bead). For example, the sample can comprise a cell or multiple cells comprising the nucleic acid molecules. The cell can comprise additional nucleic acid molecules that can be the same as or different from a nucleic acid molecule of interest. In some cases, the sample can comprise a plurality of cells, and each cell can contain one or more nucleic acid molecules. The cell can be, for example, a human cell, an animal cell, or a plant cell. In some cases, the cell can be derived from a tissue or fluid, as described herein. The cell can be a prokaryotic cell or a eukaryotic cell. The cell can be a lymphocyte such as a B cell or T cell.

Access to a nucleic acid molecule included in a cell can be provided by lysing or permeabilizing the cell. Lysing the cell can release the nucleic acid molecule contained therein from the cell. A cell can be lysed using a lysis agent such as a bioactive agent. A bioactive agent useful for lysing a cell can be, for example, an enzyme (*e.g*., as described herein). An enzyme used to lyse a cell may or may not be capable of carrying out additional functions such as degrading, extending, reverse transcribing, or otherwise altering a nucleic acid molecule. Alternatively, an ionic or nonionic surfactant such as TritonX-100, Tween 20, sarcosyl, or sodium dodecyl sulfate can be used to lyse a cell. Cell lysis can also be achieved using a cellular disruption method such as an electroporation or a thermal, acoustic, or mechanical disruption method. Alternatively, a cell can be permeabilized to provide access to a nucleic acid molecule included therein. Permeabilization can involve partially or completely dissolving or disrupting a cell membrane or a portion thereof. Permeabilization can be achieved by, for example, contacting a cell membrane with an organic solvent (*e.g*., methanol) or a detergent such as Triton X-100 or NP-40.

One or more operations described herein can be performed in bulk or in a partition. For example, the nucleic acid molecules and the nucleic acid barcode molecules can be provided in individual partitions (*e.g*., droplets in emulsion, wells, as is described elsewhere herein). One or more reagents can be co-partitioned with the nucleic acid molecules. For example, a nucleic acid molecule or a derivative thereof or a cell comprising the nucleic acid molecule or a derivative thereof can be co-partitioned with one or more reagents selected from the group consisting of lysis agents or buffers, permeabilizing agents, enzymes (*e.g*., enzymes capable of digesting one or more RNA molecules, extending one or more nucleic acid molecules, reverse transcribing an RNA molecule, permeabilizing or lysing a cell, or carrying out other actions), fluorophores, oligonucleotides, primers, probes, barcodes, nucleic acid barcode molecules (*e.g*., nucleic acid barcode molecules comprising one or more barcode sequences), buffers, deoxynucleotide triphosphates, detergents, reducing agents, chelating agents, oxidizing agents, nanoparticles, beads, and antibodies. In some cases, a nucleic acid molecule or a derivative thereof, or a cell comprising the nucleic acid molecule or a derivative thereof (*e.g*., a cell bead), can be co-partitioned with one or more reagents selected from the group consisting of temperature-sensitive enzymes, pH-sensitive enzymes, light-sensitive enzymes, reverse transcriptases, proteases, ligase, polymerases, restriction enzymes, nucleases, protease inhibitors, exonucleases, and nuclease inhibitors. For example, a nucleic acid molecule or a derivative thereof or a cell comprising the nucleic acid molecule or a derivative thereof can be co-partitioned with a polymerase and nucleotide molecules. Partitioning a nucleic acid molecule or a derivative thereof or a cell comprising the nucleic acid molecule or a derivative thereof and one or more reagents can comprise flowing a first phase comprising an aqueous fluid, the cell, and the one or more reagents and a second phase comprising a fluid that is immiscible with the aqueous fluid toward a junction. Upon interaction of the first and second phases, a discrete droplet of the first phase comprising the nucleic acid molecule or a derivative thereof or a cell comprising the nucleic acid molecule or a derivative thereof (*e.g*., a cell bead) and the one or more reagents can be formed. In some cases, the partition can comprise a single cell. The cell can be lysed or permeabilized within the partition (*e.g*., droplet) to provide access to the nucleic acid molecule of the cell.

In some instances, multiple operations are performed within the partition. For example, the nucleic acid molecules (*e.g*., from or within a cell) and the nucleic acid barcode molecules (*e.g.,* a plurality of the first nucleic acid barcode molecules and a plurality of the second nucleic acid barcode molecules) can be provided in a partition. Alternatively, the nucleic acid molecules and the nucleic acid barcode molecules can be provided in bulk, allowed to hybridize, and then partitioned. Barcoding can be performed within the partition, to generate a first barcoded molecule, e.g., first barcoded nucleic acid molecule (or plurality of first barcoded molecules, e.g., first barcoded nucleic acid molecules) and a second barcoded molecule, e.g., second barcoded nucleic acid molecule (or plurality of second barcoded molecules, e.g., second barcoded nucleic acid molecules). Subsequent to barcoding, circularization of the barcoded molecules can be performed within the partition. Alternatively, the barcoded molecules can be collected or released from the partitions and subjected to circularization in bulk (outside of the partitions). Similarly, the extension of the circularized molecules to generate extension molecules can occur in partitions or in bulk.

The first nucleic acid barcode molecule can comprise a different sequence than the second nucleic acid barcode molecule, or the first nucleic acid barcode molecule can comprise one or more same sequences as the second nucleic acid barcode molecules, but in different regions. For example, the first nucleic acid barcode molecule can comprise, in a 5' to 3' direction, a first barcode sequence, a first primer sequence, and a second primer sequence, and the second nucleic acid barcode molecule can comprise, in a 5' to 3' direction, the second primer sequence, the first primer sequence, and a second barcode sequence, which can be the same as or different from the first barcode sequence.

Barcoding of the nucleic acid molecules to generate the barcoded molecules, e.g., barcoded nucleic acid molecules, can include the use of hybridization, ligation, or other nucleic acid reactions. For example, the first nucleic molecule can comprise a sequence that is complementary to a sequence of the first nucleic acid barcode molecule. Hybridization and extension of the complementary sequences can be sufficient to generate the first barcoded molecule, e.g., first barcoded nucleic acid molecule. Alternatively or in addition to, a ligation reaction can be performed on the hybridized molecules to generate the first barcoded molecule. In some instances, a nucleic acid extension reaction can be performed to generate the first barcoded molecule. The extension reaction can comprise the use of an enzyme (*e.g*., polymerase, reverse transcriptase) to add one or more nucleotides to an end of the first nucleic acid barcode molecule. For example, the extension reaction can be performed using the nucleic acid molecule (*e.g.,* RNA molecule, target nucleic acid) as a template strand; in such instances, the first barcoded molecule can comprise a first sequence complementary to at least a portion of the first nucleic acid molecule. Similarly, the second nucleic acid molecule can comprise a sequence that is complementary to a sequence of the second nucleic acid barcode molecule. Hybridization of the complementary sequences can be sufficient to generate the second barcoded molecule, e.g., second barcoded nucleic acid molecule. Alternatively or in addition to, a ligation reaction can be performed on the hybridized molecules to generate the second barcoded molecule. In some instances, a nucleic acid extension reaction can be performed to generate the second barcoded molecule, as described above. In such instances, the second barcoded molecule can comprise a second sequence complementary to at least a portion of the second nucleic acid molecule.

The first nucleic acid barcode molecule, the second nucleic acid barcode molecule, or both the first and second nucleic acid barcode molecules can comprise a capture sequence. In some embodiments, the capture sequence is configured to hybridize to a sequence present in a nucleic acid molecule, *e.g*., by complementary base pairing. The capture sequence of the first nucleic acid barcode molecule can be the same or different as the capture sequence of the second nucleic acid barcode molecule. The capture sequence can be or comprise a primer sequence, such as an mRNA specific priming sequence (*e.g*., poly-T sequence), a targeted (or target-specific) priming sequence, and/or a random priming sequence *(e.g.,* a random Nmer), for example, to facilitate an assay for gene expression, as is described elsewhere herein. In some instances, the capture sequence can comprise a sequence that is complementary to a binding sequence of a reporter oligonucleotide of a feature binding group (*e.g.,* an antibody, affirmer, aptamer, lipophilic moiety). The feature binding group can be capable of binding to an analyte present in or on a surface of a biological particle (*e.g*., a protein, a carbohydrate, a lipid, a nucleic acid molecule, or a combination thereof) and can be useful in combination with the methods described herein, *e.g.,* for analysis of proteomic or metabolomic features of samples or cells. Examples of systems and methods for analyzing multiple analyte types using feature binding groups comprising reporter oligonucleotides are disclosed in U.S. Pat. No. 10,011,872, issued July 3, 2018, U.S. Pat. No. 10,323,278, issued June 18, 2019, U.S. Pat. No. 10,480,029, issued November 19, 2019, U.S. Pat. No. 10,858,702, issued December 8, 2020, U.S. Pat. No. 10,793,905, issued October 6, 2020, U.S. Pat. No. 10,954,562, issued March 23, 2021, U.S. Pat. No. 10,725,027, issued July 28, 2020, U.S. Pat. No. 10,816,543, issued October 27, 2020, U.S. Pat. No. 11,002,731, issued May 11, 2021, and U.S. Pat. No. 10,928,386, issued February 23, 2021, each of which are incorporated by reference herein in its entirety.

Alternatively or in addition to the capture sequence, the first nucleic acid barcode molecule and the second nucleic acid barcode molecule can comprise one or more additional adapter sequences. The adapter sequences can include, in non-limiting examples, primers, primer-binding sites, sequencing primers or partial sequencing primers, unique molecular identifiers (UMIs), additional barcode sequences, capture sequences, binding sequences, restriction recognition sites, PCR primer regions, spacers, etc. The adapter sequences can include one or more functional sequences as described herein. The adapter sequences can be located in any useful location along the length of the nucleic acid barcode molecule. In some embodiments, a capture sequence (*e.g.,* a poly-T sequence for capturing mRNA, a random Nmer sequence, a target-specific sequence, etc.) is positioned at a 3' end of the nucleic acid barcode molecule to facilitate capture of the nucleic acid molecule (*e.g.,* a nucleic acid analyte such as an RNA molecule) and, in some instances, an extension reaction (*e.g*., reverse transcription) to generate the barcoded molecules, e.g., first barcoded nucleic acid molecules. In some embodiments, the nucleic acid barcode molecules comprise one or more capture sequences that are complementary to at least a portion of the nucleic acid molecules (*e.g*., nucleic acid analytes).

The barcoded molecules, e.g., barcoded nucleic acid molecules described herein can be generated using reagents provided with the nucleic acid molecules and the nucleic acid barcode molecules (*e.g*., in bulk or in partitions). As described above, in some instances, a nucleic acid extension reaction is performed to generate the first barcoded molecule (e.g., first barcoded nucleic acid molecule) and the second barcoded molecule (e.g., second barcoded nucleic acid molecule). In such instances, an enzyme configured for performing the extension reaction can be provided. Such an enzyme can include, for example, a polymerase (*e.g*., DNA polymerase, RNA polymerase, etc.) or a reverse transcriptase, which can be naturally occurring or an engineered variant. For example, the nucleic acid molecules can comprise RNA molecules, and the barcoded molecules can be generated by hybridizing the nucleic acid barcode molecules to at least a portion of the RNA molecules and performing a reverse transcription reaction. Such a reverse transcription reaction can be performed using a reverse transcriptase, such as those that are naturally occurring or engineered variants, such as a thermostable group II intron reverse transcriptase (TGIRT) or a Marathon reverse transcriptase.

The barcoded molecules can be single-stranded, double-stranded, or partially double-stranded (*e.g*., comprising "sticky ends" or overhang sequences) and can comprise DNA, RNA, or both. For example, following annealing of the nucleic acid molecules (*e.g*., RNA molecules) to the nucleic acid barcode molecules (*e.g*., DNA barcode molecules) and extension (*e.g*., reverse transcription), the barcoded molecule can be a double-stranded DNA-RNA molecule. In some instances, the barcoded molecules can be treated to generate single-stranded barcoded molecules, *e.g.,* via denaturation (via enzymes, heat, chemicals, etc.), using a degrading enzyme (*e.g*., RNase), etc. The single-stranded barcoded molecule (*e.g*., barcoded DNA molecule) can then be circularized.

The barcoded molecules can be subjected to conditions sufficient to circularize the barcoded molecules, thereby generating circularized molecules. In some instances, the circularization is performed by hybridization of "sticky ends" or overhang sequences of the ends of partially double-stranded barcoded molecules, which can be generated from one or more nucleic acid extension reactions or by providing an enzyme capable of cleaving the barcoded molecules (*e.g*., restriction endonucleases). For example, while or subsequent to generating the barcoded molecules, the barcoded molecules can be contacted with a restriction endonuclease, which can generate overhang sequences on either or both the 5' and 3' end of the barcoded molecules. The sticky ends can subsequently be annealed to one another to generate the circularized molecules. Alternatively or in addition to, the circularization can be performed using an enzyme, such as a circularization enzyme and/or a ligase. Non-limiting examples of circularization enzymes or ligases that can circularize nucleic acid molecules include CircLigase, T4 DNA ligase, T7 DNA ligase, SplintR^{®}, T4RNA Ligase, KOD RNA Ligase, double-stranded DNA ligase, isolated ligases from other organisms or viruses, or engineered variants thereof. For example, a ligase used for circularization can be isolated from a virus, *e.g*., Acanthocystis turfacea chlorella virus 1 (ATCV1).

Non-limiting examples of virus-isolated ligases include those described in U.S. Provisional Patent Application No. 63/171,031, filed April 5, 2021, which is incorporated by reference herein in its entirety. Examples of other circularization enzymes or ligases are described in U.S. Patent Nos. 10,597,650 and 10,597,710. In such examples, the barcoded molecules can be single-stranded, and circularization (*e.g*., using CircLigase or a DNA ligase) is performed on the single-stranded barcoded molecules and results in single-stranded circularized molecules.

In some instances, circularization of the barcoded molecules can be mediated by intramolecular ligation using a splint nucleic acid molecule (also referred to herein as a "splint molecule" or "splint oligonucleotide. For example, circularization of the barcoded molecules that are longer in length (*e.g.,* greater than 500 base pairs, greater than 1000 base pairs, greater than 2000 base pairs, greater than 5000 base pairs, or more) can be more efficient using a splint molecule under certain conditions. The splint molecule can comprise a first splint sequence and a second splint sequence; the first splint sequence can be complementary to a portion of the 5' end of a barcoded molecule, and the second splint sequence can be complementary to a portion of the 3' end of the barcoded molecule. Accordingly, the splint molecule can hybridize to the 5' and 3' ends of the barcoded molecule, thereby circularizing the barcoded molecule. In some instances, ligation of the 5' and 3' ends of the circularized barcoded molecule can be performed to generate the circularized molecule. In some instances, the circularized molecules can be directly sequenced (*e.g*., using PacBio HiFi sequencing), or the circularized molecules can be further processed (*e.g.*, linearized, amplified, etc.), as described below."). Ligases suitable for intramolecular ligation using a splint oligonucleotide include CircLigase, T4 DNA ligase, T7 DNA ligase, SplintR^{®}, T4RNA Ligase, KOD RNA Ligase, double-stranded DNA ligase, ATCV1 ligase, and the like. In some instances, the ligase used for intramolecular ligation using a splint oligonucleotide is a T4 DNA ligase, *e.g.,* an ATCV1 ligase disclosed herein.

Subsequent to circularization, the circularized molecules (*e.g*., the first circularized molecule and the second circularized molecule) can be subjected to conditions sufficient to perform a nucleic acid reaction (*e.g*., nucleic acid extension). Primer molecules can be provided which comprise sequences that are complementary to at least a portion of the nucleic acid barcode molecules (*e.g*., the first nucleic acid barcode molecule and the second nucleic acid barcode molecule). For example, the first nucleic acid barcode molecule and the second nucleic acid molecule can each comprise a first primer sequence and a second primer sequence. Accordingly, provision of (i) a first primer molecule comprising a sequence complementary to the first primer sequence and (ii) a second primer molecule comprising the same sequence or a complementary sequence to the second primer sequence can allow for extension, *e.g*., using a polymerase and nucleotides, of the barcoded molecules to generate extension molecules (*e.g.,* a first extension molecule and a second extension molecule). In some embodiments, the extension reaction is an amplification reaction, *e.g.,* a linear amplification reaction such as, e.g., RCA, or an exponential amplification reaction such as, e.g., PCR. In some instances, the resultant extension product (e.g., nucleic acid extension product) can comprise the same sequences as the barcoded molecule, and/or a reverse complement thereof. Beneficially, the use of primer molecules (*e.g.,* a first primer molecule and a second primer molecule) for performing the extension reaction can generate non-concatemeric extension products from the circularized molecule (*e.g.,* a single linear copy of the sequences comprised within the barcoded molecule) without requiring any cleavage operations. Such non-concatemeric extension products can comprise a first sequence corresponding to the nucleic acid molecule (*e.g*., target mRNA) and a second sequence corresponding to the barcode sequence. In alternative embodiments, the extension reaction is an RCA reaction that generates a concatemeric product.

Subsequent to barcoding, circularization, and extension, the resultant first extension molecule (derived from the first barcoded molecule) and the second extension molecule (derived from the second barcoded molecule) can comprise a different order of sequences. For example, the first barcoded molecule can comprise, in the 5' to 3' direction, a first barcode sequence, a first primer sequence and the second primer sequence. The first barcoded molecule can comprise a first target sequence complementary to the first nucleic acid molecule (*e.g*., target nucleic acid molecule or nucleic acid analyte), and the first target sequence can be located 3' of the first barcode sequence, the first primer sequence, and the second primer sequence. Subsequent to circularization and extension, the first extension molecule, derived from the first barcoded molecule, can comprise, in the 5' to 3' direction, a sequence corresponding to the second primer sequence, a sequence corresponding to the first barcode sequence, and a sequence corresponding to the first primer sequence. In some instances, subsequent to circularization and extension, the first target sequence (or a sequence corresponding thereto) is located 5' of the first barcode sequence. Accordingly, the methods described herein can enable a shift in the position of a barcode sequence relative to a target sequence of the nucleic acid molecule (*e.g*., target nucleic acid molecule or nucleic acid analyte).

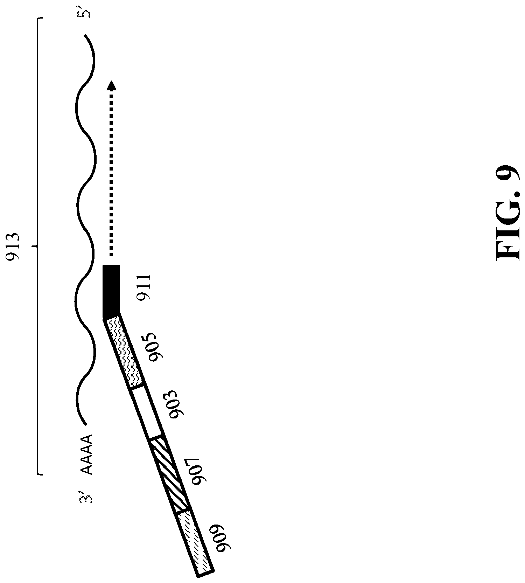

Similarly, the second barcoded molecule can comprise, in a 5' to 3' direction, the second primer sequence, the first primer sequence, and the second barcode sequence. In some instances, the second barcoded molecule comprises a second target sequence complementary to the second nucleic acid molecule (*e.g*., target nucleic acid molecule or nucleic acid analyte), and the second target sequence can be located 3' of the second barcode sequence. Subsequent to circularization and extension, the second extension molecule can comprise, in a 5' to 3' direction, a sequence corresponding to the first primer sequence, a sequence corresponding to the second barcode sequence, and a sequence corresponding to the second primer sequence. In some instances, subsequent to circularization and extension, the second target sequence remains 3' of the second barcode sequence. As such, provision of the first nucleic acid barcode molecule and the second nucleic acid barcode molecule, can allow for barcoding of nucleic acid molecules in which, in the extended product, the barcode sequence is positioned 5' relative to the target nucleic acid molecule (*e.g.,* the first extension product) or 3' relative to the target nucleic acid molecule (*e.g.,* the second extension product). Beneficially, the use of both the first nucleic acid barcode molecule and the second nucleic acid barcode molecule within a workflow can enable increased sample characterization depth, *e.g*., by designing capture sequences with different target regions that can anneal to multiple regions within the nucleic acid molecule, *e.g*., mRNA, rather than at just an end, as described in **FIG. 9** below.

**FIGS. 7A-7B** schematically illustrate example methods for barcoding nucleic acid molecules, as described herein. **FIG. 7A** shows an example of generating a first barcoded molecule and **FIG. 7B** shows an example of generating a second barcoded molecule. **FIG. 7A** Panel A shows a first nucleic acid barcode molecule **701,** comprising, in a 5' to 3' direction, a first barcode sequence **703,** optionally a unique molecular identifier (UMI) sequence **705,** a first primer sequence **707,** a second primer sequence **709,** and a capture sequence (*e.g*., poly-T sequence) **711.** Panel B of **FIG. 7A** shows contacting of a nucleic acid molecule (*e.g.,* a target nucleic acid molecule, a nucleic acid analyte such as an mRNA molecule, etc.) **713** with the first nucleic acid barcode molecule **701.** The capture sequence **711** can be complementary to a sequence (*e.g*., poly-A) sequence of the nucleic acid molecule **713** and can hybridize thereto. In some embodiments the capture sequence **711** does not hybridize at a 5' end of the nucleic acid molecule. In some embodiments the capture sequence **711** hybridizes at or near a 3' end of the nucleic acid molecule **713.** In some embodiments the capture sequence **711** hybridizes at a non-end region of a nucleic acid molecule **713.** Following hybridization, a nucleic acid extension can be performed, *e.g*., using a polymerase or a reverse transcriptase, thereby yielding a first barcoded molecule **715,** as shown in Panel C of **FIG. 7A.** In some instances, the first barcoded molecule **715** is single-stranded and can be generated by degrading the nucleic acid molecule **713** (*e.g.,* using RNase) or by denaturing (*e.g.,* using chemical or thermal approaches) the nucleic acid molecule **713** from the extension product of Panel B. The first barcoded molecule can comprise the original nucleic acid barcode molecule sequences (**703**, **705, 707, 709, 711**) or complements thereof, as well as a first target sequence **717,** which can comprise a same sequence or a complement of the nucleic acid molecule **713.** Panel D of **FIG. 7A** illustrates a first circularized molecule **719** generated from the first barcoded molecule **715,** which can be generated using a circularizing enzyme (*e.g*., CircLigase) (not shown), or by intramolecular ligation using a splint nucleic acid molecule (not shown). The first circularized molecule **719** can comprise the same sequences as the first barcoded molecule **715** (*e.g.,* **703, 705, 707, 709, 711,** and **717**). Subsequent to circularization, the first circularized molecule **719** can be subjected to a nucleic acid extension reaction to generate a first extension molecule **721.** The first extension molecule **721** can be generated, for example, by providing a first primer molecule comprising a sequence complementary to the first primer sequence **707** and a second primer molecule comprising a sequence that is identical to or complementary to the second primer sequence **709,** and providing conditions sufficient to perform the nucleic acid extension reaction. In some embodiments the extension reaction is an amplification reaction, *e.g.,* a RCA reaction or PCR reaction. The first extension molecule **721** can comprise, from a 5' to 3' direction, the second primer sequence **709,** the capture sequence **711,** the first target sequence **717** (*e.g.,* a sequence corresponding to the nucleic acid molecule **713**), the barcode sequence **703,** the UMI sequence **705,** and the first primer sequence **707.** In some embodiments, the method as described in **FIG. 7A** generates a barcoded product wherein the capture sequence **711** or reverse complement thereof is distal to the barcode sequence **703** and UMI sequence **705** (or reverse complements thereof). Analysis of the barcoded product (or derivative thereof) by sequencing, *e.g*., by using a sequencing by synthesis approach can provide 5' sequence information of the nucleic acid analyte **713.**

**FIG. 7B** shows another example of a barcoding scheme, which can be alternatively or in addition to the workflow shown in **FIG. 7A.** **FIG. 7B** Panel A shows a second nucleic acid barcode molecule **702,** comprising, in a 5' to 3' direction, a second primer sequence **709** (which can be the same sequence as the second primer sequence of **FIG. 7A** ), a first primer sequence **707** (which can be the same sequence as the first primer sequence of **FIG. 7A** ), a second barcode sequence **703** (which can be the same sequence as the first barcode sequence of **FIG. 7A** ), optionally a UMI sequence **705,** and a capture sequence (*e.g*., poly-T sequence) **711.**

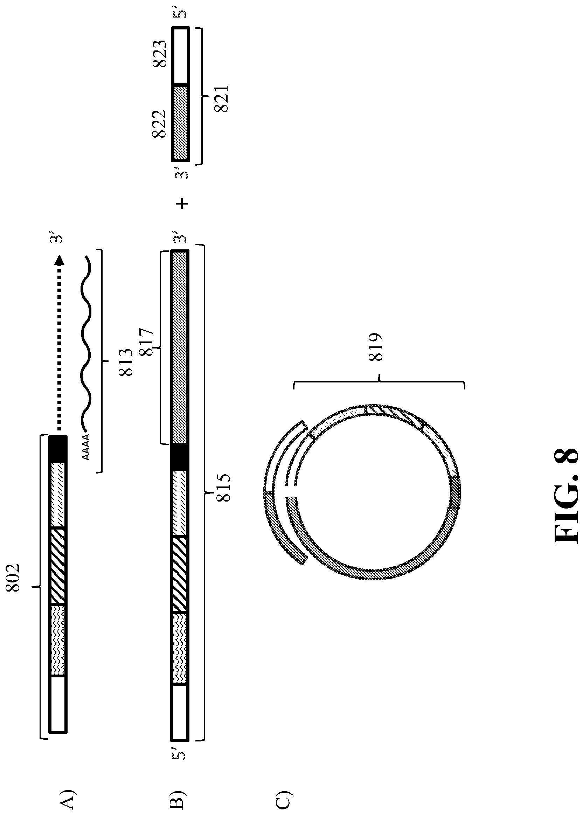

Panel B of **FIG. 7B** shows contacting of a nucleic acid molecule (*e.g.,* a target nucleic acid molecule, a nucleic acid analyte such as an mRNA molecule, etc.) **714** with the second nucleic acid barcode molecule **702.** The nucleic acid molecule **714** can comprise the same or different sequences than the nucleic acid molecule shown in **FIG. 7A .**The capture sequence **711** can be complementary to a sequence (*e.g*., poly-A) sequence of the nucleic acid molecule **714** and can hybridize thereto. In some embodiments the capture sequence **711** does not hybridize at a 5' end of the nucleic acid molecule. In some embodiments the capture sequence **711** hybridizes at or near a 3' end of the nucleic acid molecule **714.** In some embodiments the capture sequence **711** hybridizes at a non-end region of the nucleic acid molecule **714.** Following hybridization, a nucleic acid extension can be performed, *e.g*., using a polymerase or a reverse transcriptase, thereby yielding a second barcoded molecule **716,** as shown in Panel C of **FIG. 7B.** In some instances, the second barcoded molecule **716** is single-stranded and can be generated by degrading the nucleic acid molecule **714** (*e.g.,* using RNase) or by denaturing (*e.g*., using chemical or thermal approaches) the nucleic acid molecule **714** from the extension product of Panel B. The second barcoded molecule **716** can comprise the original nucleic acid barcode molecule sequences **(709,707, 703, 705, 711)** or complements thereof, as well as a second target sequence **718,** which can comprise a same sequence or a complement of the nucleic acid molecule **714.** Panel D of **FIG. 7B** illustrates a second circularized molecule **720** generated from the second barcoded molecule **716,** which can be generated using a circularizing enzyme (*e.g*., CircLigase) (not shown), or by intramolecular ligation using a splint nucleic acid molecule (not shown). The second circularized molecule **720** can comprise the same sequences as the first barcoded molecule **716** (*e.g*., **709,707, 703, 705, 711,** and **718**). Subsequent to circularization, the second circularized molecule **720** can be subjected to a nucleic acid extension reaction to generate a second extension molecule **722.** The second extension molecule **722** can be generated, for example, by providing a first primer molecule comprising a sequence complementary to the first primer sequence **707** and a second primer molecule comprising a sequence that is identical to or complementary to the second primer sequence **709,** and providing conditions sufficient to perform the nucleic acid extension reaction. In some embodiments the extension reaction is an amplification reaction, *e.g*., a RCA reaction or PCR reaction. The second extension molecule **722** can comprise, from a 5' to 3' direction, the first primer sequence **707,** the second barcode sequence **703,** the capture sequence **711,** the second target sequence **718** (*e.g*., a sequence corresponding to the nucleic acid molecule **713**), the UMI sequence **705,** and the first primer sequence **707.** In some embodiments, the method as described in **FIG. 7B** generates a barcoded product wherein the capture sequence **711** or reverse complement thereof is proximal to the barcode sequence **703** and UMI sequence **705** (or reverse complements thereof). Analysis of the barcoded product (or derivative thereof) by sequencing, *e.g*., using a sequencing by synthesis approach can provide 3' sequence information of the nucleic acid analyte **713.** As described herein, the circularization can be mediated by a splint molecule. **FIG. 8** schematically shows an example circularization process using a splint oligonucleotide. In such an example, a nucleic acid barcode molecule **802** can be provided and contacted with a nucleic acid molecule **813** (*e.g.,* a target analyte, mRNA molecule, etc.), as shown in **FIG. 8** Panel A. An extension reaction can be performed to generate a barcoded molecule **815,** as shown in **FIG. 8** Panel B. The barcoded molecule **815** can comprise a target sequence **817,** which can comprise a same sequence or a complement of the nucleic acid molecule **813.** Additionally, a splint molecule **821** can be provided. The splint molecule can comprise a first sequence **822** complementary to a portion of the 5' end of the barcoded molecule **815** and a second sequence **823** complementary to a portion of the 3' end of the barcoded molecule **815** (*e.g.,* all or a portion of the target sequence **817**). In Panel C, the first sequence of the splint molecule hybridizes to the 5' end of the barcoded molecule **815** and the second sequence of the splint molecule hybridizes to the 3' end of the barcoded molecule **815,** thereby generating a circularized molecule **819.** The ends of the circularized molecule **819** can subsequently be ligated (*e.g*., when the ends are adjacent to one another) or gap-filled (*e.g.,* using a polymerase, when the ends are not adjacent to one another or are separated by a number of nucleotides).

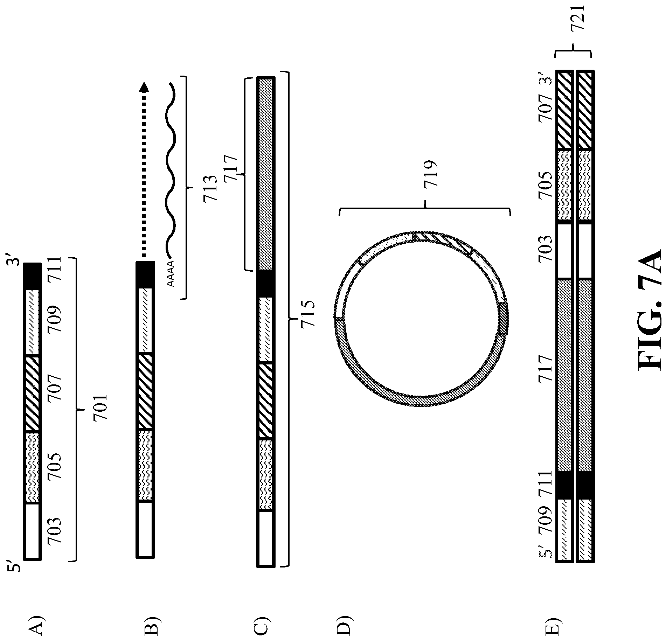

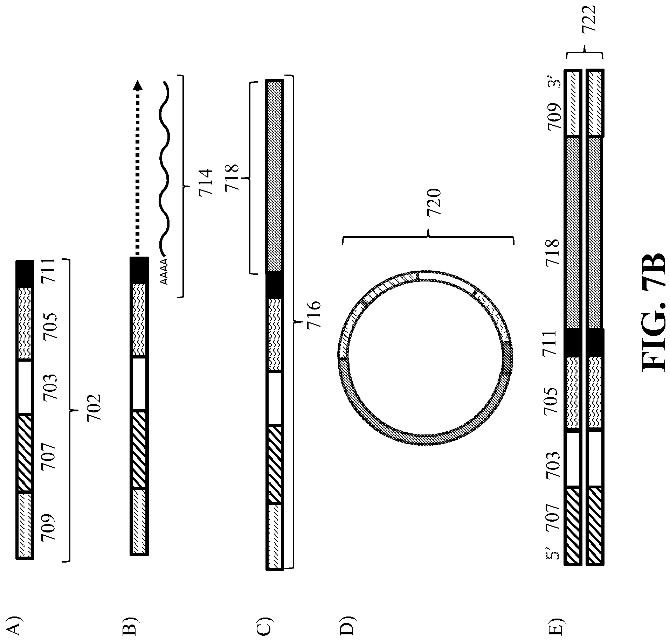

Although **FIGS. 7-8** illustrate nucleic acid barcode molecules comprising capture sequences that anneal to an end of a nucleic acid molecule (*e.g*., **713, 714, 813**), it will be appreciated that the capture sequence can hybridize to other regions of the nucleic acid molecule. For example, **FIG. 9** shows an example of a nucleic acid barcode molecule (*e.g*., a second nucleic acid barcode molecule, such as that shown in **FIG. 7B** ) that anneals to a non-end region of a nucleic acid molecule **913** (*e.g*., a target analyte, a mRNA molecule, etc.). The nucleic acid barcode molecule can comprise, from a 5' to 3' end, a second primer sequence **909** (which can be the same sequence as the second primer sequence of **FIG. 7A** ), a first primer sequence **907** (which can be the same sequence as the first primer sequence of **FIG. 7A** ), a barcode sequence **903** (which can be the same sequence as the first or second barcode sequence of **FIGS. 7A-7B**), optionally a UMI sequence **905,** and a capture sequence **911.** The capture sequence **911** can be or comprise a target-specific priming sequence that can anneal to a portion of the nucleic acid molecule **913.** Subsequent extension, *e.g*., via reverse transcription, can be performed to generate a barcoded molecule comprising a portion of the nucleic acid molecule **913.** The barcoded molecule (not shown) can be further processed, e.g., subjected to circularization and extension to generate an extension molecule.

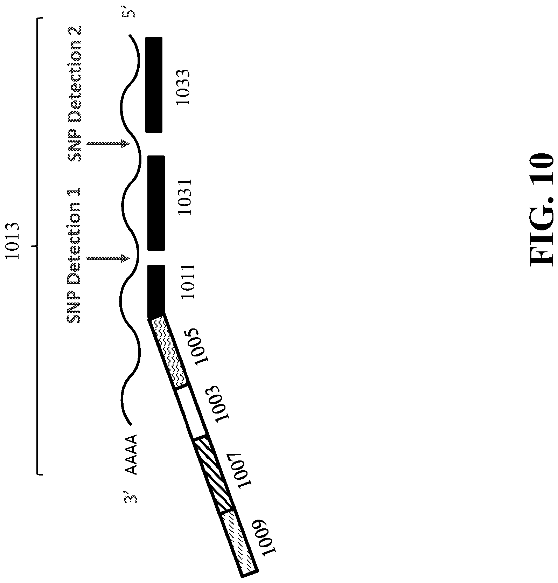

In some examples, the nucleic acid barcode molecules can be used for detection of single nucleotide polymorphisms (SNPs) or indels of nucleic acid molecules (*e.g.*, RNA molecules). **FIG. 10** shows an example of a nucleic acid barcode molecule (*e.g*., a second nucleic acid barcode molecule, such as that shown in **FIG. 7B** ) that anneals to a region of a nucleic acid molecule **1013** (*e.g*., a target analyte, a mRNA molecule, etc.) comprising two SNP sites. The nucleic acid barcode molecule can comprise, from a 5' to 3' end, a second primer sequence **1009** (which can be the same sequence as the second primer sequence of **FIG. 7A** ), a first primer sequence **1007** (which can be the same sequence as the first primer sequence of **FIG. 7A** ), a barcode sequence **1003** (which can be the same sequence as the first or second barcode sequence of **FIGS. 7A-7B**), a UMI sequence **1005,** and a capture sequence **1011.** The capture sequence **1011** can be or comprise a target-specific priming sequence that can anneal to a portion of the nucleic acid molecule **1013.** In some instances, capture sequence 1011 hybridizes to a sequence of the nucleic acid molecule **1013** that is upstream of a first SNP. Extension of the nucleic acid barcode molecule can generate a barcoded product that includes the first SNP and second SNP sites. The barcoded molecule (not shown) can be further processed, *e.g*., subjected to circularization and extension to generate an extension molecule.

Subsequent to circularization and extension, the extension molecules can be further processed. Referring again to **FIGS. 7A-7B**and **FIGS. 8-10****,** the extension molecules (*e.g*., the first extension molecule **721** and the second extension molecule **722**) can be processed using approaches that allow for further characterization. For example, subsequent to circularization and extension, the extension molecules can be subjected to amplification, and cleanup. In some instances, adapter sequences can be added to the extension molecules. For example, sequencing primer sequences or partial sequencing primer sequences, read sequences, sequences for attachment to a flow cell, etc. can be added to the extension molecules. Further characterization, *e.g*., via sequencing, can be performed.

It will be appreciated that any number of operations can be performed in a partition. For example, referring to **FIGS. 7A-7B**and **FIGS. 8-10****,** the nucleic acid molecules (*e.g*., **713, 714, 813**), which optionally can be comprised within a cell or cell bead, and the nucleic acid barcode molecules (*e.g*., **701, 702, 802**) can be provided within a partition. For example, performing one or more of the operations described in **FIG. 7A** and **FIG. 7B** in one or more partitions can advantageously provide both 5' and 3' sequence information for RNA analytes from one or more biological particles (*e.g*., single cells, single cell beads, or single nuclei). Alternatively, the provision of the nucleic acid molecules and the nucleic acid barcode molecules can occur in bulk, and then the nucleic acid molecules and the nucleic acid barcode molecules (or complexes thereof) can be subsequently partitioned. Similarly, the generation of the barcoded molecule (*e.g*., via annealing of the nucleic acid barcode molecule to the nucleic acid molecule and extension reaction), the circularization of the barcoded molecule, and the extension reaction to generate extension molecules can be performed in a partition or outside of a partition (*e.g*., in bulk). The products of any of the operations described herein can be removed from the partitions at any useful or convenient step; for example, the barcoded molecules can be removed from the partitions prior to circularization and extension, which can occur in bulk.

In one non-limiting example, a set of nucleic acid barcode molecules comprising the first nucleic acid barcode molecule (*e.g*., as shown in **FIG. 7A** ) and the second nucleic acid barcode molecule (*e.g.,* as shown in **FIG. 7B** ) are provided in a partition (*e.g*., droplet or well) with a biological particle (*e.g.,* a cell, cell nucleus) comprising a first nucleic acid molecule (*e.g.,* a first mRNA molecule) and a second nucleic acid molecule (*e.g*., a second mRNA molecule). The cell can be permeabilized and/or fixed, either prior to or within the partition, or the cell can be lysed in the partition, thus releasing the first nucleic acid molecule and the second nucleic acid molecule into the partition. The first nucleic acid molecule can anneal to the first nucleic acid barcode molecule (*e.g*., via a first capture sequence), and the second nucleic acid barcode molecule can anneal to the second nucleic acid barcode molecule (*e.g*., via a second capture sequence). In some instances, the first nucleic acid molecule can be capable of annealing to either the first nucleic acid molecule or the second nucleic acid barcode molecule. Subsequently, a nucleic acid extension reaction (*e.g*., using a reverse transcriptase) can be performed within the partition to generate a first barcoded molecule and a second barcoded molecule. In some instances, following the extension reaction, the double-stranded barcoded molecules can be subjected to conditions sufficient to generate single-stranded barcoded molecules (*e.g*., denaturation using chemicals or heat, treatment with a degrading enzyme, such as RNase). The first barcoded molecule and the second barcoded molecule can be removed or collected from the partition and circularized in bulk. Subsequent processing (*e.g*., extension reactions to generate the extension products) can also occur in bulk. In other instances, the circularization, the extension reaction, or both processes can occur in the partition. Subsequent characterization (*e.g*., amplification, sequencing) can occur in bulk.

The nucleic acid barcode molecules (*e.g*., the first nucleic acid barcode molecule, the second nucleic acid barcode molecule, or both the first and the second nucleic acid barcode molecules) can be attached to a support, such as a particle or bead, *e.g.,* as described elsewhere herein. The bead can be provided (*e.g*., co-partitioned) with the nucleic acid molecules or the cell comprising the nucleic acid molecules. The bead can comprise a plurality of nucleic acid barcode molecules that can be the same or different. For example, the bead can comprise a plurality of the first nucleic acid barcode molecules. Alternatively or in addition to, the bead can comprise a plurality of the second nucleic acid barcode molecules. The bead can comprise at least 10,000 nucleic acid barcode molecules attached thereto. For example, the bead can comprise at least 100,000, 1,000,000, 10,000,000, 100 million, or 1 billion nucleic acid barcode molecules attached thereto. In instances when a bead comprises both the first nucleic acid barcode molecule and the second nucleic acid barcode molecule, any useful ratio of the first nucleic acid barcode molecule to the second nucleic acid barcode molecule can be used, *e.g.,* about 10000: 1, about 1000: 1, about 100: 1, about 30:, about 10:1, about 3:1, about 1:1, about 1:3, about 1:10, about 1:30, about 1:100, about 1:1000, or about 1:10000.

In some cases, each nucleic acid barcode molecule of the plurality of nucleic acid barcode molecules coupled to a support can comprise a common barcode sequence. The common barcode sequence can be a sequence that is the same or common to all or a portion of the nucleic acid barcode molecules of a given support. In some instances, a plurality of the first nucleic acid barcode molecules and a plurality of the second nucleic acid barcode molecules can be attached to a given support, and both pluralities (of the first and second nucleic acid barcode molecules) can comprise the common barcode sequence. Such examples of all or a portion of the nucleic acid barcode molecules comprising the common barcode sequence can be useful in attributing a given nucleic acid molecule (*e.g*., target nucleic acid molecule) back to a given partition or biological particle (*e.g*., a cell or cell nucleus). For example, the supports comprising the nucleic acid barcode molecules can be partitioned, into a plurality of partitions, with cells comprising the nucleic acid molecules (*e.g*., target nucleic acid molecules). Partitioning can occur such that a subset of the partitions comprise a single support and a single cell. Accordingly, the common barcode sequence can be attributable to a particular partition or cell, and any barcoded products derived therefrom can similarly be attributable to the particular partition or cell. In other instances, the plurality of the first nucleic acid barcode molecules can comprise a first common barcode sequence, and the plurality of the second nucleic acid barcode molecules can comprise a second common barcode sequence that is different from the first common barcode sequence. The nucleic acid barcode molecules can further comprise an additional barcode sequence that can be different for each nucleic acid barcode molecule attached to the bead (*e.g*., a unique molecular identifier sequence).

The plurality of nucleic acid barcode molecules can be releasably attached to the bead. The plurality of nucleic acid barcode molecules can be releasable from the bead upon application of a stimulus. Such a stimulus can be selected from the group consisting of a thermal stimulus, a photo stimulus, and a chemical stimulus. For example, the stimulus can be a reducing agent such as dithiothreitol. Application of a stimulus can result in one or more of (i) cleavage of a linkage between nucleic acid barcode molecules of the plurality of nucleic acid barcode molecules and the bead, and (ii) degradation or dissolution of the bead to release nucleic acid barcode molecules of the plurality of nucleic acid barcode molecules from the bead. In some cases, one or more nucleic acid barcode molecules can be released from the bead prior to hybridization of a binding sequence of a nucleic acid barcode molecule to a target sequence of a nucleic acid molecule (*e.g*., nucleic acid analyte). The one or more nucleic acid barcode molecules can be released from the bead within a partition comprising the bead and the nucleic acid molecule (or a cell comprising the nucleic acid molecule).

Beads and methods of preparation and use are described further herein.

In another aspect, provided herein is a method for barcoding nucleic acid molecules. The method can comprise generating a barcoded molecule using a nucleic acid molecule (*e.g.,* a target nucleic acid molecule, nucleic acid analyte) and a nucleic acid barcode molecule. The barcoded molecule can comprise, in a 5' to 3' direction, a barcode sequence and a sequence (*e.g*., target sequence) complementary to at least a portion of the nucleic acid molecule. The barcoded molecule can be subjected to conditions to circularize the barcoded molecule, thereby generating a circularized molecule, and the circularized molecule can be subjected to a nucleic acid reaction, *e.g*., nucleic acid extension, to generate an extension product. The extension product can comprise, in a 5' to 3' direction, a first sequence corresponding to the nucleic acid molecule (*e.g.,* a target sequence) and a second sequence corresponding to the barcode sequence.

In other aspects of the present disclosure, provided herein is a method, comprising generating a barcoded molecule using a nucleic acid molecule (*e.g.,* a target nucleic acid molecule, nucleic acid analyte) and a nucleic acid barcode molecule. The barcoded molecule can comprise, in a 5' to 3' direction, a first primer sequence, a second primer sequence, and a sequence complementary to at least a portion of the nucleic acid molecule (*e.g.,* a target sequence). The barcoded molecule can be subjected to conditions to circularize the barcoded molecule, thereby generating a circularized molecule, and the circularized molecule can be subjected to a nucleic acid reaction, e.g., nucleic acid extension, to generate an extension product. The extension product can comprise, in a 5' to 3' direction, a first sequence corresponding to the second primer sequence, a second sequence corresponding to the nucleic acid molecule and a third sequence corresponding to the first primer sequence.

In another aspect, provided herein is a method for barcoding nucleic acid molecules, comprising generating a single-stranded barcoded nucleic acid molecule using a nucleic acid molecule and a single-stranded nucleic acid barcode molecule. The single-stranded barcoded nucleic acid molecule can comprise a barcode sequence. Subsequently, the single-stranded barcoded nucleic acid molecule can be circularized by ligating a first end and a second end of the single-stranded barcoded nucleic acid molecule, thereby generating a circularized molecule. A non-concatemeric extension product can be generated from the circularized molecule, in which the non-concatemeric extension product comprises a first sequence corresponding to the first nucleic acid molecule and a second sequence corresponding to the nucleic acid barcode sequence.

Also provided herein are kits for performing the methods described herein. Such kits can comprise reagents for performing one or all operations of the methods described herein. In an example, a kit can comprise (i) a first nucleic acid barcode molecule, comprising in a 5' to 3' direction, a first barcode sequence, a first primer sequence, and a second primer sequence, (ii) a second nucleic acid barcode molecule comprising, in a 5' to 3' direction, the second primer sequence, the first primer sequence, and a second barcode sequence. The kit can additionally comprise primer molecules, such as a first primer molecule comprising a first sequence complementary to the first primer sequence, and a second primer molecule comprising a second sequence that is the same or complementary to the second primer sequence.

In some instances, the kit can further comprise a bead, and the first nucleic acid barcode molecule and the second nucleic acid barcode molecule can be coupled to the bead. The bead can comprise a plurality of first nucleic acid molecules and a plurality of second nucleic acid molecules, in which the plurality of first nucleic acid barcode molecules comprise the first nucleic acid barcode molecule comprising the first barcode sequence, and the plurality of second nucleic acid barcode molecules comprises the second nucleic acid barcode molecule comprising the second barcode sequence. In some instances, the kit further comprises a plurality of such beads, in which the plurality of beads comprise a plurality of first nucleic acid barcode molecules and a plurality of second nucleic acid barcode molecules coupled (*e.g*., releasably coupled) thereto. In some instances, the first barcode sequence and the second barcode sequence is the same or comprises a common sequence for a given bead, which can differ across the beads.

In other instances, and as described elsewhere herein, the kit can comprise a first bead coupled to the first nucleic acid barcode molecule and a second bead coupled to the second nucleic acid barcode molecule. The first bead can be among a plurality of first beads comprising a plurality of first nucleic acid barcode molecules coupled thereto. Similarly, the second bead can be among a plurality of second beads comprising a plurality of second nucleic acid barcode molecules coupled thereto.

In some instances, the kit can comprise a plurality of first nucleic acid barcode molecules (*e.g*., comprising the abovementioned first nucleic acid barcode molecule) and a plurality of second nucleic acid barcode molecules (*e.g*., comprising the abovementioned second nucleic acid barcode molecule). All or a subset of the first nucleic acid barcode molecules can each comprise the first barcode sequence, and all or a subset of the second nucleic acid barcode molecules can each comprise the second barcode sequence. In some instances, the kit can comprise a plurality of first primer molecules and second primer molecules.

As described elsewhere herein, the first barcode sequence can be the same as the second barcode sequence, or the first barcode sequence and the second barcode sequence can comprise a common sequence. In some instances, the first nucleic acid barcode molecule further comprises a first UMI sequence and the second nucleic acid barcode molecule comprises a second UMI sequence that is different from the first UMI sequence.

The first nucleic acid barcode molecule and/or the second nucleic acid barcode molecule can comprise a capture sequence (*e.g*., at a 3' end). In some instances, the first nucleic acid barcode molecule comprises the same capture sequence as the second nucleic acid barcode molecule. In other instances, the capture sequences of the first nucleic acid barcode molecule and the second nucleic acid barcode molecule are different. The capture sequence, as described herein, can be a poly-T sequence, a random N-mer, a target-specific sequence, a reporter oligonucleotide-binding sequence, etc.

In one non-limiting example, the first nucleic acid barcode molecule comprises, in a 5' to 3' direction, a common barcode sequence, a first UMI sequence, the first primer sequence, the second primer sequence and a first capture sequence. The second nucleic acid barcode molecule can comprise, in a 5' to 3' direction, the second primer sequence, the first primer sequence, the common barcode sequence, and a second UMI sequence different from the first UMI sequence, and a second capture sequence (which can be the same or different as the first capture sequence).

The kits of the present disclosure can further comprise reagents for performing the operations of the method (*e.g*., barcoding, circularization, extension). For example, a kit can comprise an enzyme configured to circularize a nucleic acid molecule, such as a single-stranded circularization enzyme, a CircLigase, or other ligase, such as T4 DNA ligase, T7 DNA ligase, SplintR, etc., as is described elsewhere herein. The kit can further comprise reagents for performing nucleic acid reactions, such as a nucleic acid extension reaction, ligation, amplification, restriction digest, reverse transcription, etc. For example, the kit can include a reverse transcriptase (*e.g*., TGIRT, Marathon, or other conventional reverse transcriptase), which can be useful in generating the barcoded molecules described herein.

In addition, the kits of the present disclosure can comprise instructions for performing any of the methods described herein.

In other aspects of the present disclosure, provided herein are compositions which can be useful in performing the methods described herein. Such a composition can comprise, for example, a particle or support comprising (i) a first nucleic acid barcode molecule comprising, in a 5' to 3' direction, a first barcode sequence, a first primer sequence, and a second primer sequence; and (ii) a second nucleic acid barcode molecule comprising, in a 5' to 3' direction, the second primer sequence, the first primer sequence, and a second barcode sequence. In some instances, the particle is a bead.

In some instances, the compositions can also comprise a partition (*e.g*., a well or droplet), and the partition can comprise the particle. In such instances, the partition can further comprise reagents for performing the nucleic acid reactions described herein; *e.g.,* reverse transcriptase (*e.g.,* a thermostable group II intron reverse transcriptase (TGIRT), or Marathon reverse transcriptase) for generating barcoded molecules, a ligase, *e.g*., CircLigase, T4 DNA ligase, T7 DNA Ligase, SplintR, T4RNA Ligase, KOD RNA Ligase, or a double stranded DNA ligase for performing circularization, etc. In some instances, the partition further comprises a first primer molecule comprising a first sequence complementary to the first primer sequence and a second primer molecule comprising a second sequence complementary to the second primer sequence.

### Systems and Methods for Sample Compartmentalization

In an aspect, the systems and methods described herein provide for the compartmentalization, depositing, or partitioning of one or more particles (*e.g*., biological particles, macromolecular constituents of biological particles, beads, reagents, etc.) into discrete compartments or partitions (referred to interchangeably herein as partitions), where each partition maintains separation of its own contents from the contents of other partitions. A partition can be a volume, wherein diffusion of contents beyond the volume is inhibited. For example, the partitions can include a porous matrix that is capable of entraining and/or retaining materials within its matrix. The partition can be a droplet in an emulsion or a well. A partition can comprise one or more other partitions.

A partition can include one or more particles. A partition can include one or more types of particles. For example, a partition of the present disclosure can comprise one or more biological particles and/or macromolecular constituents thereof. A partition can comprise one or more beads. A partition can comprise one or more gel beads. A partition can comprise one or more cell beads. A partition can include a single gel bead, a single cell bead, or both a single cell bead and single gel bead. A partition can include one or more reagents. Alternatively, a partition can be unoccupied. For example, a partition may not comprise a bead. A cell bead can be a biological particle and/or one or more of its macromolecular constituents encased inside of a gel or polymer matrix, such as via polymerization of a droplet containing the biological particle and precursors capable of being polymerized or gelled. Unique identifiers, such as barcodes, can be injected into the droplets previous to, subsequent to, or concurrently with droplet generation, such as via a microcapsule (*e.g*., bead), as described elsewhere herein.

The methods and systems of the present disclosure can comprise methods and systems for generating one or more partitions such as droplets. The droplets can comprise a plurality of droplets in an emulsion. In some examples, the droplets can comprise droplets in a colloid. In some cases, the emulsion can comprise a microemulsion or a nanoemulsion. In some examples, the droplets can be generated with aid of a microfluidic device and/or by subjecting a mixture of immiscible phases to agitation (*e.g*., in a container). In some cases, a combination of the mentioned methods can be used for droplet and/or emulsion formation.

The partitions described herein can comprise small volumes, for example, less than about 10 microliters (µL), 5 µL, 1 µL, 500 nanoliters (nL), 100 nL, 50 nL, 10 nL, 5 nL, 1 nL, 900 picoliters (pL), 800 pL, 700 pL, 600 pL, 500 pL, 400pL, 300 pL, 200 pL, 100pL, 50 pL, 20 pL, 10 pL, 1 pL, or less.

For example, in the case of droplet based partitions, the droplets can have overall volumes that are less than about 1000 pL, 900 pL, 800 pL, 700 pL, 600 pL, 500 pL, 400pL, 300 pL, 200 pL, 100pL, 50 pL, 20 pL, 10 pL, 1 pL, or less. Where co-partitioned with beads, it will be appreciated that the sample fluid volume, *e.g*., including co-partitioned biological particles and/or beads, within the partitions can be less than about 90% of the above described volumes, less than about 80%, less than about 70%, less than about 60%, less than about 50%, less than about 40%, less than about 30%, less than about 20%, or less than about 10% of the above described volumes.

As is described elsewhere herein, partitioning species can generate a population or plurality of partitions. In such cases, any suitable number of partitions can be generated or otherwise provided. For example, at least about 1,000 partitions, at least about 5,000 partitions, at least about 10,000 partitions, at least about 50,000 partitions, at least about 100,000 partitions, at least about 500,000 partitions, at least about 1,000,000 partitions, at least about 5,000,000 partitions at least about 10,000,000 partitions, at least about 50,000,000 partitions, at least about 100,000,000 partitions, at least about 500,000,000 partitions, at least about 1,000,000,000 partitions, or more partitions can be generated or otherwise provided. Moreover, the plurality of partitions can comprise both unoccupied partitions (*e.g*., empty partitions) and occupied partitions.

Droplets can be formed by creating an emulsion by mixing and/or agitating immiscible phases. Mixing or agitation can comprise various agitation techniques, such as vortexing, pipetting, tube flicking, or other agitation techniques. In some cases, mixing or agitation can be performed without using a microfluidic device. In some examples, the droplets can be formed by exposing a mixture to ultrasound or sonication. Systems and methods for droplet and/or emulsion generation by agitation are described in International Application No. PCT/US20/17785, which is entirely incorporated herein by reference for all purposes.

### Microfluidic Systems

Microfluidic devices or platforms comprising microfluidic channel networks (*e.g*., on a chip) can be utilized to generate partitions such as droplets and/or emulsions as described herein. Methods and systems for generating partitions such as droplets, methods of encapsulating biological particles in partitions, methods of increasing the throughput of droplet generation, and various geometries, architectures, and configurations of microfluidic devices and channels are described in U.S. Patent Publication Nos. 2019/0367997 and 2019/0064173, each of which is entirely incorporated herein by reference for all purposes.

In some examples, individual particles can be partitioned to discrete partitions by introducing a flowing stream of particles in an aqueous fluid into a flowing stream or reservoir of a non-aqueous fluid, such that droplets can be generated at the junction of the two streams/reservoir, such as at the junction of a microfluidic device provided elsewhere herein.

The methods of the present disclosure can comprise generating partitions and/or encapsulating particles, such as biological particles or analyte carriers, in some cases, individual biological particles or analyte carriers such as single cells. In some examples, reagents can be encapsulated and/or partitioned (*e.g*., co-partitioned with biological particles or analyte carriers) in the partitions. Various mechanisms can be employed in the partitioning of individual particles. An example can comprise porous membranes through which aqueous mixtures of cells can be extruded into fluids (*e.g*., non-aqueous fluids).

The partitions can be flowable within fluid streams. The partitions can comprise, for example, micro-vesicles that have an outer barrier surrounding an inner fluid center or core. In some cases, the partitions can comprise a porous matrix that is capable of entraining and/or retaining materials within its matrix. The partitions can be droplets of a first phase within a second phase, wherein the first and second phases are immiscible. For example, the partitions can be droplets of aqueous fluid within a non-aqueous continuous phase (*e.g.,* oil phase). In another example, the partitions can be droplets of a non-aqueous fluid within an aqueous phase. In some examples, the partitions can be provided in a water-in-oil emulsion or oil-in-water emulsion. A variety of different vessels are described in, for example, U.S. Patent Application Publication No. 2014/0155295, which is entirely incorporated herein by reference for all purposes. Emulsion systems for creating stable droplets in non-aqueous or oil continuous phases are described in, for example, U.S. Patent Application Publication No. 2010/0105112, which is entirely incorporated herein by reference for all purposes.

Fluid properties (*e.g.,* fluid flow rates, fluid viscosities, etc.), particle properties (*e.g.,* volume fraction, particle size, particle concentration, etc.), microfluidic architectures (*e.g*., channel geometry, etc.), and other parameters can be adjusted to control the occupancy of the resulting partitions (*e.g*., number of biological particles per partition, number of beads per partition, etc.). For example, partition occupancy can be controlled by providing the aqueous stream at a certain concentration and/or flow rate of particles. To generate single biological particle partitions, the relative flow rates of the immiscible fluids can be selected such that, on average, the partitions can contain less than one biological particle per partition in order to ensure that those partitions that are occupied are primarily singly occupied. In some cases, partitions among a plurality of partitions can contain at most one biological particle (*e.g*., bead, DNA, cell or cellular material). In some embodiments, the various parameters (*e.g*., fluid properties, particle properties, microfluidic architectures, etc.) can be selected or adjusted such that a majority of partitions are occupied, for example, allowing for only a small percentage of unoccupied partitions. The flows and channel architectures can be controlled as to ensure a given number of singly occupied partitions, less than a certain level of unoccupied partitions and/or less than a certain level of multiply occupied partitions.

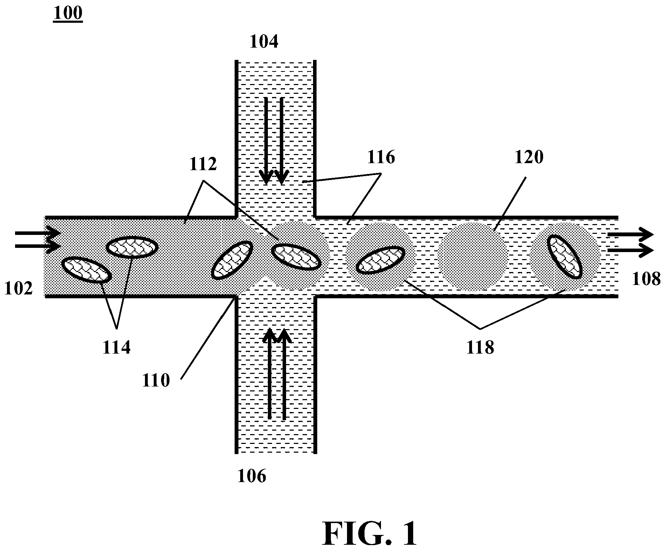

**FIG. 1** shows a non-limiting example of a microfluidic channel structure **100** for partitioning individual biological particles. The channel structure **100** can include channel segments **102, 104, 106** and **108** communicating at a channel junction **110.** In operation, a first aqueous fluid **112** that includes suspended biological particles (or cells) **114** can be transported along channel segment **102** into junction **110,** while a second fluid **116** that is immiscible with the aqueous fluid **112** is delivered to the junction **110** from each of channel segments **104** and **106** to create discrete droplets **118, 120** of the first aqueous fluid **112** flowing into channel segment **108,** and flowing away from junction **110.** The channel segment **108** can be fluidically coupled to an outlet reservoir where the discrete droplets can be stored and/or harvested. A discrete droplet generated can include an individual biological particle **114** (such as droplets **118**). A discrete droplet generated can include more than one individual biological particle **114** (not shown in **FIG**. **1**). A discrete droplet can contain no biological particle **114** (such as droplet **120**). Each discrete partition can maintain separation of its own contents (*e.g*., individual biological particle **114**) from the contents of other partitions.

The second fluid **116** can comprise an oil, such as a fluorinated oil, that includes a fluorosurfactant for stabilizing the resulting droplets, for example, inhibiting subsequent coalescence of the resulting droplets **118, 120.** Examples of particularly useful partitioning fluids and fluorosurfactants are described, for example, in U.S. Patent Application Publication No. 2010/0105112, which is entirely incorporated herein by reference for all purposes.

As will be appreciated, the channel segments described herein can be coupled to any of a variety of different fluid sources or receiving components, including reservoirs, tubing, manifolds, or fluidic components of other systems. As will be appreciated, the microfluidic channel structure **100** can have other geometries. For example, a microfluidic channel structure can have more than one channel junction. For example, a microfluidic channel structure can have 2, 3, 4, or 5 channel segments each carrying particles (*e.g*., biological particles, cell beads, and/or gel beads) that meet at a channel junction. Fluid can be directed to flow along one or more channels or reservoirs via one or more fluid flow units. A fluid flow unit can comprise compressors (*e.g*., providing positive pressure), pumps (*e.g*., providing negative pressure), actuators, and the like to control flow of the fluid. Fluid can also or otherwise be controlled via applied pressure differentials, centrifugal force, electrokinetic pumping, vacuum, capillary or gravity flow, or the like.

The generated droplets can comprise two subsets of droplets: (1) occupied droplets **118,** containing one or more biological particles **114,** and (2) unoccupied droplets **120,** not containing any biological particles **114.** Occupied droplets **118** can comprise singly occupied droplets (having one biological particle) and multiply occupied droplets (having more than one biological particle). As described elsewhere herein, in some cases, the majority of occupied partitions can include no more than one biological particle per occupied partition and some of the generated partitions can be unoccupied (of any biological particle). In some cases, though, some of the occupied partitions can include more than one biological particle. In some cases, the partitioning process can be controlled such that fewer than about 25% of the occupied partitions contain more than one biological particle, and in many cases, fewer than about 20% of the occupied partitions have more than one biological particle, while in some cases, fewer than about 10% or even fewer than about 5% of the occupied partitions include more than one biological particle per partition.

In some cases, it can be desirable to minimize the creation of excessive numbers of empty partitions, such as to reduce costs and/or increase efficiency. While this minimization can be achieved by providing a sufficient number of biological particles (*e.g*., biological particles **114**) at the partitioning junction **110,** such as to ensure that at least one biological particle is encapsulated in a partition, the Poissonian distribution can expectedly increase the number of partitions that include multiple biological particles. As such, where singly occupied partitions are to be obtained, at most about 95%, 90%, 85%, 80%, 75%, 70%, 65%, 60%, 55%, 50%, 45%, 40%, 35%, 30%, 25%, 20%, 15%, 10%, 5% or less of the generated partitions can be unoccupied.

In some cases, the flow of one or more of the biological particles (*e.g*., in channel segment 102), or other fluids directed into the partitioning junction (*e.g*., in channel segments **104, 106**) can be controlled such that, in many cases, no more than about 50% of the generated partitions, no more than about 25% of the generated partitions, or no more than about 10% of the generated partitions are unoccupied. These flows can be controlled so as to present a non-Poissonian distribution of single-occupied partitions while providing lower levels of unoccupied partitions (*e.g*., no more than about 50%, about 25%, or about 10% unoccupied). The above noted ranges of unoccupied partitions can be achieved while still providing any of the single occupancy rates described above. For example, in many cases, the use of the systems and methods described herein can create resulting partitions that have multiple occupancy rates of less than about 25%, less than about 20%, less than about 15%, less than about 10%, and in many cases, less than about 5%, while having unoccupied partitions of less than about 50%, less than about 40%, less than about 30%, less than about 20%, less than about 10%, less than about 5%, or less.

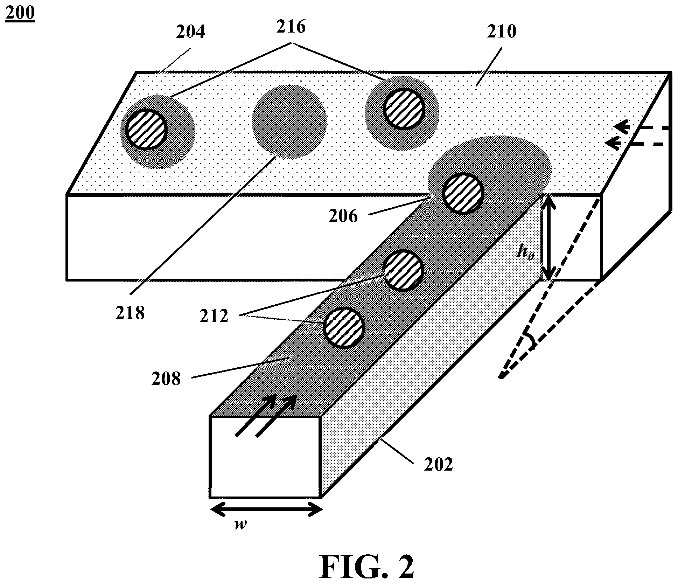

As will be appreciated, the above-described occupancy rates are also applicable to partitions that include both biological particles and additional reagents, including, but not limited to, microcapsules or beads (*e.g.,* gel beads) carrying barcoded nucleic acid molecules (*e.g*., oligonucleotides, nucleic acid barcode molecules) (described in relation to **FIG. 2**). The occupied partitions (*e.g*., at least about 10%, 20%, 30%, 40%, 50%, 60%, 70%, 80%, 90%, 95%, or 99% of the occupied partitions) can include both a microcapsule (*e.g*., bead) comprising barcoded nucleic acid molecules and a biological particle.

In some examples, a partition of the plurality of partitions can comprise a single biological particle (*e.g*., a single cell or a single nucleus of a cell). In some examples, a partition of the plurality of partitions can comprise multiple biological particles. Such partitions can be referred to as multiply occupied partitions, and can comprise, for example, two, three, four or more cells and/or beads (*e.g*., beads) comprising nucleic acid barcode molecules within a single partition. Accordingly, as noted above, the flow characteristics of the biological particle and/or bead containing fluids and partitioning fluids can be controlled to provide for such multiply occupied partitions. In particular, the flow parameters can be controlled to provide a given occupancy rate at greater than about 50% of the partitions, greater than about 75%, and in some cases greater than about 80%, 90%, 95%, or higher.

Microfluidic systems for partitioning are further described in U.S. Patent Application Pub. No. US 2015/0376609, which is hereby incorporated by reference in its entirety.

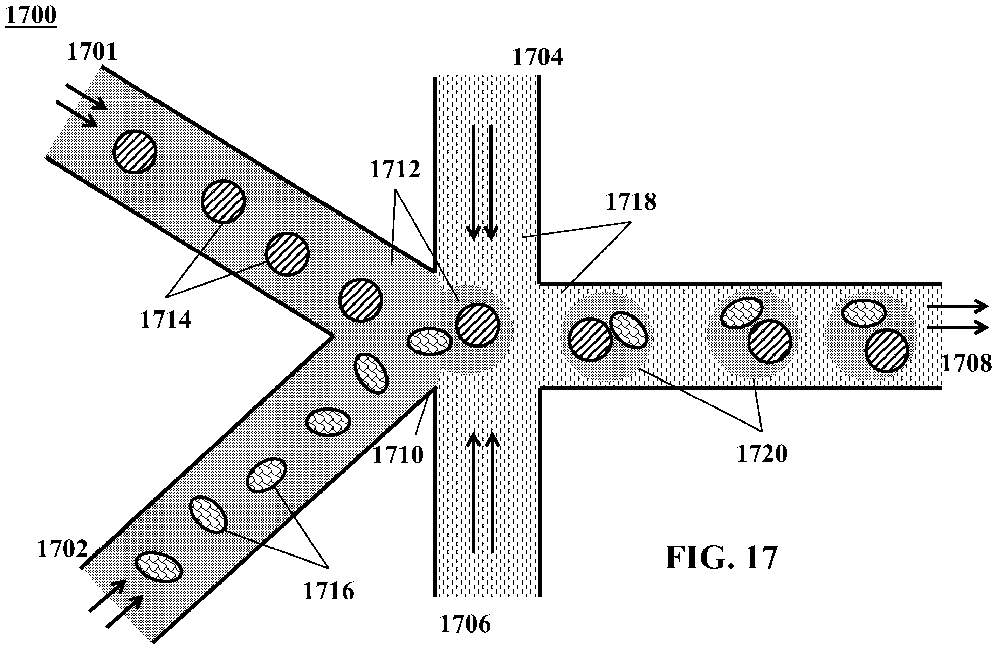

**FIG. 17** shows an example of a microfluidic channel structure **1700** for delivering barcode carrying beads to droplets. The channel structure **1700** can include channel segments **1701, 1702, 1704, 1706** and **1708** communicating at a channel junction **1710.** In operation, the channel segment **1701** can transport an aqueous fluid **1712** that includes a plurality of beads **1714** (*e.g*., with nucleic acid molecules, *e.g*., nucleic acid barcode molecules or barcoded oligonucleotides, molecular tags) along the channel segment **1701** into junction **1710.** The plurality of beads **1714** can be sourced from a suspension of beads. For example, the channel segment **1701** can be connected to a reservoir comprising an aqueous suspension of beads **1714.** The channel segment **1702** can transport the aqueous fluid **1712** that includes a plurality of biological particles **1716** along the channel segment **1702** into junction **1710.** The plurality of biological particles **1716** can be sourced from a suspension of biological particles. For example, the channel segment **1702** can be connected to a reservoir comprising an aqueous suspension of biological particles **1716.** In some instances, the aqueous fluid **1712** in either the first channel segment **1701** or the second channel segment **1702,** or in both segments, can include one or more reagents, as further described below. A second fluid **1718** that is immiscible with the aqueous fluid **1712** *(e.g.,* oil) can be delivered to the junction **1710** from each of channel segments **1704** and **1706.** Upon meeting of the aqueous fluid **1712** from each of channel segments **1701** and **1702** and the second fluid **1718** from each of channel segments **1704** and **1706** at the channel junction **1710,** the aqueous fluid **1712** can be partitioned as discrete droplets **1720** in the second fluid **1718** and flow away from the junction **1710** along channel segment **1708.** The channel segment **1708** can deliver the discrete droplets to an outlet reservoir fluidly coupled to the channel segment **1708,** where they can be harvested. As an alternative, the channel segments **1701** and **1702** can meet at another junction upstream of the junction **1710.** At such junction, beads and biological particles can form a mixture that is directed along another channel to the junction **1710** to yield droplets **1720.** The mixture can provide the beads and biological particles in an alternating fashion, such that, for example, a droplet comprises a single bead and a single biological particle.

### Controlled Partitioning

In some aspects, provided are systems and methods for controlled partitioning. Droplet size can be controlled by adjusting certain geometric features in channel architecture (e.g., microfluidics channel architecture). For example, an expansion angle, width, and/or length of a channel can be adjusted to control droplet size.

**FIG. 2** shows an example of a microfluidic channel structure for the controlled partitioning of beads into discrete droplets. A channel structure **200** can include a channel segment **202** communicating at a channel junction **206** (or intersection) with a reservoir **204.** The reservoir **204** can be a chamber. Any reference to "reservoir," as used herein, can also refer to a "chamber." In operation, an aqueous fluid **208** that includes suspended beads **212** can be transported along the channel segment **202** into the junction **206** to meet a second fluid **210** that is immiscible with the aqueous fluid **208** in the reservoir **204** to create droplets **216, 218** of the aqueous fluid **208** flowing into the reservoir **204.** At the junction **206** where the aqueous fluid **208** and the second fluid **210** meet, droplets can form based on factors such as the hydrodynamic forces at the junction **206,** flow rates of the two fluids **208, 210,** fluid properties, and certain geometric parameters *(e.g., w, h₀, α,* etc.) of the channel structure **200.** A plurality of droplets can be collected in the reservoir **204** by continuously injecting the aqueous fluid **208** from the channel segment **202** through the junction **206.**

In some instances, the aqueous fluid **208** can have a substantially uniform concentration or frequency of beads **212.** The beads **212** can be introduced into the channel segment **202** from a separate channel (not shown in **FIG. 2****).** The frequency of beads **212** in the channel segment **202** can be controlled by controlling the frequency in which the beads **212** are introduced into the channel segment **202** and/or the relative flow rates of the fluids in the channel segment **202** and the separate channel. In some instances, the beads can be introduced into the channel segment **202** from a plurality of different channels, and the frequency controlled accordingly.

In some instances, the aqueous fluid **208** in the channel segment **202** can comprise biological particles. In some instances, the aqueous fluid **208** can have a substantially uniform concentration or frequency of biological particles. As with the beads, the biological particles can be introduced into the channel segment **202** from a separate channel. The frequency or concentration of the biological particles in the aqueous fluid **208** in the channel segment **202** can be controlled by controlling the frequency in which the biological particles are introduced into the channel segment **202** and/or the relative flow rates of the fluids in the channel segment **202** and the separate channel. In some instances, the biological particles can be introduced into the channel segment **202** from a plurality of different channels, and the frequency controlled accordingly. In some instances, a first separate channel can introduce beads and a second separate channel can introduce biological particles into the channel segment **202.** The first separate channel introducing the beads can be upstream or downstream of the second separate channel introducing the biological particles.

The second fluid **210** can comprise an oil, such as a fluorinated oil, that includes a fluorosurfactant for stabilizing the resulting droplets, for example, inhibiting subsequent coalescence of the resulting droplets.

In some instances, the second fluid **210** may not be subjected to and/or directed to any flow in or out of the reservoir **204.** For example, the second fluid **210** can be substantially stationary in the reservoir **204.** In some instances, the second fluid **210** can be subjected to flow within the reservoir **204,** but not in or out of the reservoir **204,** such as via application of pressure to the reservoir **204** and/or as affected by the incoming flow of the aqueous fluid **208** at the junction **206.** Alternatively, the second fluid **210** can be subjected and/or directed to flow in or out of the reservoir **204.** For example, the reservoir **204** can be a channel directing the second fluid **210** from upstream to downstream, transporting the generated droplets.

Systems and methods for controlled partitioning are described further in PCT/US2018/047551, which is hereby incorporated by reference in its entirety.

### Cell beads

In another aspect, in addition to or as an alternative to droplet based partitioning, biological particles can be comprised within (*e.g.*, encapsulated within) a particulate material to form a "cell bead".

A cell bead can contain a biological particle *(e.g.,* a cell) or macromolecular constituents *(e.g.,* RNA, DNA, proteins, etc.) of a biological particle. A cell bead can include a single cell or multiple cells, or a derivative of the single cell or multiple cells. For example after lysing and washing the cells, inhibitory components from cell lysates can be washed away and the macromolecular constituents can be bound as cell beads. Systems and methods disclosed herein can be applicable to both cell beads (and/or droplets or other partitions) containing biological particles and cell beads (and/or droplets or other partitions) containing macromolecular constituents of biological particles. Cell beads can be or include a cell, cell derivative, cellular material and/or material derived from the cell in, within, or encased in a matrix, such as a polymeric matrix. In some cases, a cell bead can comprise a live cell. In some instances, the live cell can be capable of being cultured when enclosed in a gel or polymer matrix, or of being cultured when comprising a gel or polymer matrix. In some instances, the polymer or gel can be diffusively permeable to certain components and diffusively impermeable to other components (*e.g*., macromolecular constituents).

Cell beads can provide certain potential advantages of being more storable and more portable than droplet-based partitioned biological particles. Furthermore, in some cases, it can be desirable to allow biological particles to incubate for a select period of time before analysis, such as in order to characterize changes in such biological particles over time, either in the presence or absence of different stimuli (or reagents).

Suitable polymers or gels can include one or more of disulfide cross-linked polyacrylamide, agarose, alginate, polyvinyl alcohol, polyethylene glycol (PEG)-diacrylate, PEG-acrylate, PEG-thiol, PEG-azide, PEG-alkyne, other acrylates, chitosan, hyaluronic acid, collagen, fibrin, gelatin, or elastin. The polymer or gel can comprise any other polymer or gel.

Encapsulation of biological particles can be performed by a variety of processes. Such processes can combine an aqueous fluid containing the biological particles with a polymeric precursor material that can be capable of being formed into a gel or other solid or semi-solid matrix upon application of a particular stimulus to the polymer precursor. The conditions sufficient to polymerize or gel the precursors can comprise any conditions sufficient to polymerize or gel the precursors. Such stimuli can include, for example, thermal stimuli (*e.g.*, either heating or cooling), photo-stimuli (*e.g.*, through photo-curing), chemical stimuli (*e.g.*, through crosslinking, polymerization initiation of the precursor (*e.g.*, through added initiators)), electromagnetic radiation, mechanical stimuli, or any combination thereof.

In some cases, air knife droplet or aerosol generators can be used to dispense droplets of precursor fluids into gelling solutions in order to form cell beads that include individual biological particles or small groups of biological particles. Likewise, membrane-based encapsulation systems can be used to generate cell beads comprising encapsulated biological particles as described herein. Microfluidic systems of the present disclosure, such as that shown in **FIG. 1****,** can be readily used in encapsulating biological particles (*e.g*., cells) as described herein. Exemplary methods for encapsulating biological particles (*e.g*., cells) are also further described in U.S. Patent Application Pub. No. US 2015/0376609 and PCT/US2018/016019, which are hereby incorporated by reference in their entirety. In particular, and with reference to **FIG. 1****,** the aqueous fluid **112** comprising (i) the biological particles **114** and (ii) the polymer precursor material (not shown) is flowed into channel junction **110,** where it is partitioned into droplets **118, 120** through the flow of non-aqueous fluid **116.** In the case of encapsulation methods, non-aqueous fluid **116** can also include an initiator (not shown) to cause polymerization and/or crosslinking of the polymer precursor to form the bead that includes the entrained biological particles. Examples of polymer precursor/initiator pairs include those described in U.S. Patent Application Publication No. 2014/0378345, which is entirely incorporated herein by reference for all purposes.

In some cases, encapsulated biological particles can be selectively releasable from the cell bead, such as through passage of time or upon application of a particular stimulus, that degrades the bead sufficiently to allow the biological particles (*e.g.,* cell), or its other contents to be released from the bead, such as into a partition (*e.g.*, droplet). Exemplary stimuli suitable for degradation of the bead are described in U.S. Patent Application Publication No. 2014/0378345, which is entirely incorporated herein by reference for all purposes.

The polymer or gel can be diffusively permeable to chemical or biochemical reagents. The polymer or gel can be diffusively impermeable to macromolecular constituents of the biological particle. In this manner, the polymer or gel can act to allow the biological particle to be subjected to chemical or biochemical operations while spatially confining the macromolecular constituents to a region of the droplet defined by the polymer or gel.

The polymer or gel can be functionalized to bind to targeted analytes, such as nucleic acids, proteins, carbohydrates, lipids or other analytes. The polymer or gel can be functionalized to bind to targeted analytes, such as nucleic acids, proteins, carbohydrates, lipids or other analytes. The polymer or gel, *e.g.,* polymer gel matrix, hydrogel or hydrogel matrix, can be functionalized to couple or link to a plurality of capture agents. The plurality of capture agents can, *e.g.*, covalently or non-covalently, couple or link to the backbone of the polymer. See, *e.g.,* U.S. Pat. 10,590,244, which is incorporated by reference in its entirety, for exemplary cell bead functionalization strategies. In an embodiment, a first capture agent of a plurality of capture agents can be a polypeptide or aptamer that (i) couples or links to the backbone of the polymer, and (ii) binds a specific analyte *(e.g.,* antibody or antigen-binding fragment thereof) secreted by the cell, *e.g.,* B cell. By way of example, a first capture agent of a plurality of capture agents can be a polypeptide, *e.g*., antibody, or aptamer that couples/links to the backbone of the polymer and binds to a secreted antibody, *e.g.*, at its Fc region. It will be understood that, in some embodiments, the first capture agent of the plurality of capture agents can, rather than couple/link to the backbone of the polymer of the gel matrix, embed in/couple to the cell membrane. In these embodiments, the first capture agent, *e.g*., polypeptide or aptamer, can (i) embed in the membrane of the cell and/or bind to a cell surface protein and (ii) bind the specific analyte, *e.g*., antibody or antigen-binding fragment thereof, thereby tethering the secreted analyte, *e.g.*, antibody, to the cell.

The polymer or gel can be polymerized or gelled via a passive mechanism. The polymer or gel can be stable in alkaline conditions or at elevated temperature. The polymer or gel can have mechanical properties similar to the mechanical properties of the bead. For instance, the polymer or gel can be of a similar size to the bead. The polymer or gel can have a mechanical strength (*e.g.*, tensile strength) similar to that of the bead. The polymer or gel can be of a lower density than an oil. The polymer or gel can be of a density that is roughly similar to that of a buffer. The polymer or gel can have a tunable pore size. The pore size can be chosen to, for instance, retain denatured nucleic acids. The pore size can be chosen to maintain diffusive permeability to exogenous chemicals such as sodium hydroxide (NaOH) and/or endogenous chemicals such as inhibitors. The polymer or gel can be biocompatible. The polymer or gel can maintain or enhance cell viability. The polymer or gel can be biochemically compatible. The polymer or gel can be polymerized and/or depolymerized thermally, chemically, enzymatically, and/or optically.

The encapsulation of biological particles can constitute the partitioning of the biological particles into which other reagents are co-partitioned. Alternatively or in addition, encapsulated biological particles can be readily deposited into other partitions (e.g., droplets) as described above.

### Beads

Nucleic acid barcode molecules can be delivered to a partition (*e.g.*, a droplet or well) via a solid support or carrier (*e.g.*, a bead). In some cases, nucleic acid barcode molecules are initially associated with the solid support and then released from the solid support upon application of a stimulus, which allows the nucleic acid barcode molecules to dissociate or to be released from the solid support. In specific examples, nucleic acid barcode molecules are initially associated with the solid support (*e.g.*, bead) and then released from the solid support upon application of a biological stimulus, a chemical stimulus, a thermal stimulus, an electrical stimulus, a magnetic stimulus, and/or a photo stimulus.

The solid support can be a bead. A solid support, *e.g*., a bead, can be porous, non-porous, hollow, solid, semi-solid, and/or a combination thereof. Beads can be solid, semi-solid, semi-fluidic, fluidic, and/or a combination thereof. In some instances, a solid support, *e.g.*, a bead, can be at least partially dissolvable, disruptable, and/or degradable. In some cases, a solid support, *e.g.*, a bead, may not be degradable. In some cases, the solid support, *e.g*., a bead, can be a gel bead. A gel bead can be a hydrogel bead. A gel bead can be formed from molecular precursors, such as a polymeric or monomeric species. A semi-solid support, *e.g.*, a bead, can be a liposomal bead. Solid supports, *e.g.*, beads, can comprise metals including iron oxide, gold, and silver. In some cases, the solid support, *e.g.,* the bead, can be a silica bead. In some cases, the solid support, *e.g.,* a bead, can be rigid. In other cases, the solid support, *e.g.*, a bead, can be flexible and/or compressible.

A partition can comprise one or more unique identifiers, such as barcodes. Barcodes can be previously, subsequently or concurrently delivered to the partitions that hold the compartmentalized or partitioned biological particle. For example, barcodes can be injected into droplets or deposited in microwells previous to, subsequent to, or concurrently with droplet generation or providing of reagents in the microwells, respectively. The delivery of the barcodes to a particular partition allows for the later attribution of the characteristics of the individual biological particle to the particular partition. Barcodes can be delivered, for example on a nucleic acid molecule (*e.g.*, an oligonucleotide), to a partition via any suitable mechanism. Nucleic acid barcode molecules can be delivered to a partition via a bead. Beads are described in further detail below.

In some cases, nucleic acid barcode molecules can be initially associated with the bead and then released from the bead. Release of the nucleic acid barcode molecules can be passive (*e.g.*, by diffusion out of the bead). In addition or alternatively, release from the bead can be upon application of a stimulus which allows the nucleic acid barcode molecules to dissociate or to be released from the bead. Such stimulus can disrupt the bead, an interaction that couples the nucleic acid barcode molecules to or within the bead, or both. Such stimulus can include, for example, a thermal stimulus (*e.g.*, where elevation of the temperature of the beads environment will result in cleavage of a linkage or other release of the nucleic acid barcode molecules from the beads), photo-stimulus (*e.g*., through cleavage of a photo-labile linkage that releases the nucleic acid barcode molecules), chemical stimulus (*e.g.*, change in pH or use of a reducing agent(s)), a mechanical stimulus, a radiation stimulus; a biological stimulus (*e.g.*, enzyme), or any combination thereof.

Methods and systems for partitioning barcode carrying beads into droplets are provided herein, and in US. Patent Publication Nos. 2019/0367997 and 2019/0064173, and International Application No. PCT/US20/17785, each of which is herein entirely incorporated by reference for all purposes.

A bead can be porous, non-porous, solid, semi-solid, semi-fluidic, fluidic, and/or a combination thereof. In some instances, a bead can be dissolvable, disruptable, and/or degradable. Degradable beads, as well as methods for degrading beads, are described in PCT/US2014/044398, which is hereby incorporated by reference in its entirety. In some cases, any combination of stimuli, *e.g.*, stimuli described in PCT/US2014/044398 and US Patent Application Pub. No. 2015/0376609, hereby incorporated by reference in its entirety, can trigger degradation of a bead. For example, a change in pH can enable a chemical agent (*e.g.*, DTT) to become an effective reducing agent.

In some cases, a bead may not be degradable. In some cases, the bead can be a gel bead. A gel bead can be a hydrogel bead. A gel bead can be formed from molecular precursors, such as a polymeric or monomeric species. A semi-solid bead can be a liposomal bead. Solid beads can comprise metals including iron oxide, gold, and silver. In some cases, the bead can be a silica bead. In some cases, the bead can be rigid. In other cases, the bead can be flexible and/or compressible.

A bead can be of any suitable shape. Examples of bead shapes include, but are not limited to, spherical, non-spherical, oval, oblong, amorphous, circular, cylindrical, and variations thereof.

Beads can be of uniform size or heterogeneous size. In some cases, the diameter of a bead can be at least about 10 nanometers (nm), 100 nm, 500 nm, 1 micrometer (µm), 5µm, 10µm, 20µm, 30µm, 40µm, 50µm, 60µm, 70µm, 80µm, 90µm, 100µm, 250µm, 500µm, 1mm, or greater. In some cases, a bead can have a diameter of less than about 10 nm, 100 nm, 500 nm, 1µm, 5µm, 10µm, 20µm, 30µm, 40µm, 50µm, 60µm, 70µm, 80µm, 90µm, 100µm, 250µm, 500µm, 1mm, or less. In some cases, a bead can have a diameter in the range of about 40-75 µm, 30-75µm, 20-75µm, 40-85µm, 40-95µm, 20-100µm, 10-100µm, 1-100µm, 20-250µm, or 20-500µm.

In certain aspects, beads can be provided as a population or plurality of beads having a relatively monodisperse size distribution. Where it can be desirable to provide relatively consistent amounts of reagents within partitions, maintaining relatively consistent bead characteristics, such as size, can contribute to the overall consistency. In particular, the beads described herein can have size distributions that have a coefficient of variation in their cross-sectional dimensions of less than 50%, less than 40%, less than 30%, less than 20%, and in some cases less than 15%, less than 10%, less than 5%, or less.

A bead can comprise natural and/or synthetic materials. For example, a bead can comprise a natural polymer, a synthetic polymer or both natural and synthetic polymers. See, e.g., PCT/US2014/044398, which is hereby incorporated by reference in its entirety. Beads can also be formed from materials other than polymers, including lipids, micelles, ceramics, glass-ceramics, material composites, metals, other inorganic materials, and others.

In some cases, the bead can comprise covalent or ionic bonds between polymeric precursors *(e.g.,* monomers, oligomers, linear polymers), nucleic acid barcode molecules (*e.g*., oligonucleotides), primers, and other entities. In some cases, the covalent bonds can be carbon-carbon bonds, thioether bonds, or carbon-heteroatom bonds.

In some cases, a plurality of nucleic acid barcode molecules can be attached to a bead. The nucleic acid barcode molecules can be attached directly or indirectly to the bead. In some cases, the nucleic acid barcode molecules can be covalently linked to the bead. In some cases, the nucleic acid barcode molecules are covalently linked to the bead via a linker. In some cases, the linker is a degradable linker. In some cases, the linker comprises a labile bond configured to release the nucleic acid barcode molecule of the plurality of nucleic acid barcode molecules. In some cases, the labile bond comprises a disulfide linkage.

Activation of disulfide linkages within a bead can be controlled such that only a small number of disulfide linkages are activated. Methods of controlling activation of disulfide linkages within a bead are described in PCT/US2014/044398, which is hereby incorporated by reference in its entirety.

In some cases, a bead can comprise an acrydite moiety, which in certain aspects can be used to attach one or more nucleic acid barcode molecules (*e.g.*, barcode sequence, nucleic acid barcode molecule, barcoded oligonucleotide, primer, or other oligonucleotide) to the bead. Acrydite moieties, as well as their uses in attaching nucleic acid molecules to beads, are described in PCT/US2014/044398, which is hereby incorporated by reference in its entirety.

For example, precursors (*e.g.*, monomers, cross-linkers) that are polymerized to form a bead can comprise acrydite moieties, such that when a bead is generated, the bead also comprises acrydite moieties. The acrydite moieties can be attached to a nucleic acid molecule, *e.g.,* a nucleic acid barcode molecule described herein.

In some cases, precursors comprising a functional group that is reactive or capable of being activated such that it becomes reactive can be polymerized with other precursors to generate gel beads comprising the activated or activatable functional group. The functional group can then be used to attach additional species (*e.g*., disulfide linkers, primers, other oligonucleotides, etc.) to the gel beads. Exemplary precursors comprising functional groups are described in PCT/US2014/044398, which is hereby incorporated by reference in its entirety.

Other non- limiting examples of labile bonds that can be coupled to a precursor or bead are described in PCT/US2014/044398, which is hereby incorporated by reference in its entirety. A bond can be cleavable via other nucleic acid molecule targeting enzymes, such as restriction enzymes (*e.g.*, restriction endonucleases), as described further below.

Species can be encapsulated in beads during bead generation (*e.g.*, during polymerization of precursors). Such species may or may not participate in polymerization. See, *e.g.*, PCT/US2014/044398, which is hereby incorporated by reference in its entirety. Such species can include, for example, nucleic acid molecules (*e.g*., oligonucleotides), reagents for a nucleic acid amplification reaction *(e.g.,* primers, polymerases, dNTPs, co-factors (*e.g.*, ionic co-factors), buffers) including those described herein, reagents for enzymatic reactions (*e.g.*, enzymes, co-factors, substrates, buffers), reagents for nucleic acid modification reactions such as polymerization, ligation, or digestion, and/or reagents for template preparation (*e.g.*, tagmentation) for one or more sequencing platforms (*e.g.*, Nextera^{®} for Illumina^{®}). Such species can include one or more enzymes described herein, including without limitation, polymerase, reverse transcriptase, restriction enzymes (*e.g.*, endonuclease), transposase, ligase, proteinase K, DNase, etc. Such species can include one or more reagents described elsewhere herein (*e.g*., lysis agents, inhibitors, inactivating agents, chelating agents, stimulus). Alternatively or in addition, species can be partitioned in a partition (*e.g.*, droplet) during or subsequent to partition formation. Such species can include, without limitation, the abovementioned species that can also be encapsulated in a bead.

In some cases, beads can be non-covalently loaded with one or more reagents. The beads can be non-covalently loaded by, for instance, subjecting the beads to conditions sufficient to swell the beads, allowing sufficient time for the reagents to diffuse into the interiors of the beads, and subjecting the beads to conditions sufficient to de-swell the beads. The swelling of the beads can be accomplished, for instance, by placing the beads in a thermodynamically favorable solvent, subjecting the beads to a higher or lower temperature, subjecting the beads to a higher or lower ion concentration, and/or subjecting the beads to an electric field. The swelling of the beads can be accomplished by various swelling methods. The de-swelling of the beads can be accomplished, for instance, by transferring the beads in a thermodynamically unfavorable solvent, subjecting the beads to lower or high temperatures, subjecting the beads to a lower or higher ion concentration, and/or removing an electric field. The de-swelling of the beads can be accomplished by various de-swelling methods. Transferring the beads can cause pores in the bead to shrink. The shrinking can then hinder reagents within the beads from diffusing out of the interiors of the beads. The hindrance can be due to steric interactions between the reagents and the interiors of the beads. The transfer can be accomplished microfluidically. For instance, the transfer can be achieved by moving the beads from one co-flowing solvent stream to a different co-flowing solvent stream. The swellability and/or pore size of the beads can be adjusted by changing the polymer composition of the bead.

Any suitable number of molecular tag molecules (*e.g.*, primer, barcoded oligonucleotide) can be associated with a bead such that, upon release from the bead, the molecular tag molecules *(e.g.,* primer, *e.g.,* barcoded oligonucleotide) are present in the partition at a pre-defined concentration. Such pre-defined concentration can be selected to facilitate certain reactions for generating a sequencing library, *e.g*., amplification, within the partition. In some cases, the pre-defined concentration of the primer can be limited by the process of producing oligonucleotide bearing beads.

### Nucleic Acid Barcode Molecules

Nucleic acid barcode molecules, *e.g*., first and second nucleic acid barcode molecules used in exemplary barcoding and circularization methods are described herein.

A nucleic acid barcode molecule can contain one or more barcode sequences. A plurality of nucleic acid barcode molecules can be coupled to a bead. The one or more barcode sequences can include sequences that are the same for all nucleic acid molecules coupled to a given bead and/or sequences that are different across all nucleic acid molecules coupled to the given bead. The nucleic acid molecule can be incorporated into the bead.

The nucleic acid barcode sequences can include from about 6 to about 20 or more nucleotides within the sequence of the nucleic acid molecules (*e.g.*, oligonucleotides). The nucleic acid barcode sequences can include from about 6 to about 20, 30, 40, 50, 60, 70, 80, 90, 100 or more nucleotides. In some cases, the length of a barcode sequence can be about 6, 7, 8, 9, 10, 11, 12, 13, 14, 15, 16, 17, 18, 19, 20 nucleotides or longer. In some cases, the length of a barcode sequence can be at least about 6, 7, 8, 9, 10, 11, 12, 13, 14, 15, 16, 17, 18, 19, 20 nucleotides or longer. In some cases, the length of a barcode sequence can be at most about 6, 7, 8, 9, 10, 11, 12, 13, 14, 15, 16, 17, 18, 19, 20 nucleotides or shorter. These nucleotides can be completely contiguous, *i.e.*, in a single stretch of adjacent nucleotides, or they can be separated into two or more separate subsequences that are separated by 1 or more nucleotides. In some cases, separated barcode subsequences can be from about 4 to about 16 nucleotides in length. In some cases, the barcode subsequence can be about 4, 5, 6, 7, 8, 9, 10, 11, 12, 13, 14, 15, 16 nucleotides or longer. In some cases, the barcode subsequence can be at least about 4, 5, 6, 7, 8, 9, 10, 11, 12, 13, 14, 15, 16 nucleotides or longer. In some cases, the barcode subsequence can be at most about 4, 5, 6, 7, 8, 9, 10, 11, 12, 13, 14, 15, 16 nucleotides or shorter.

Nucleic acid barcode molecules can comprise one or more functional sequences for coupling to an analyte or analyte tag such as a reporter oligonucleotide. Such functional sequences can include, *e.g.,* a template switch oligonucleotide (TSO) sequence, a primer sequence *(e.g.,* a poly T sequence, or a nucleic acid primer sequence complementary to a target nucleic acid sequence and/or for amplifying a target nucleic acid sequence, a random primer, and a primer sequence for messenger RNA).

As described herein, the nucleic acid barcode molecules can comprise a unique molecular identifier (UMI) sequence. A UMI sequence can be about 5-8 nucleotides in length. A UMI sequence can be less than about 5 nucleotides in length. A UMI sequence can be more than 8 nucleotides in length. A UMI sequence can be about 8-20 nucleotides in length. A UMI sequence can be about 12-16 nucleotides in length. A UMI sequence can be a unique sequence that varies across individual first and second nucleic acid barcode molecules **701** and **702** (see, *e.g.,* **FIGS. 7A** and **7B****)** coupled to a single bead. A UMI sequence can be a unique sequence that varies across individual first nucleic acid barcode molecules **701** (see, *e.g.,* **FIG. 7A****)** coupled to a single bead. The UMI sequence can be a unique sequence that varies across individual first nucleic acid barcode molecules **702** (see, *e.g.,* **FIG. 7B** )coupled to a single bead. In some cases, a UMI sequence can be a random sequence (*e.g*., such as a random N-mer sequence). For example, the UMI can provide a unique identifier of the starting analyte (*e.g*., mRNA) molecule that was captured, in order to allow quantitation of the number of original expressed RNA molecules.

In some cases, the nucleic acid barcode molecule can comprise one or more functional sequences, for example, for attachment to a sequencing flow cell, such as, for example, a P5 sequence (or a portion thereof) for Illumina^{®} sequencing. In some cases, the nucleic acid barcode molecule or derivative thereof (e.g., oligonucleotide or polynucleotide generated from the nucleic acid molecule) can comprise another functional sequence, such as, for example, a P7 sequence (or a portion thereof) for attachment to a sequencing flow cell for Illumina sequencing. In some cases, the nucleic acid molecule can comprise an R1 primer sequence for Illumina sequencing. In some cases, the nucleic acid molecule can comprise an R2 primer sequence for Illumina sequencing. In some cases, a functional sequence can comprise a partial sequence, such as a partial barcode sequence, partial anchoring sequence, partial sequencing primer sequence (e.g., partial R1 sequence, partial R2 sequence, etc.), a partial sequence configured to attach to the flow cell of a sequencer (e.g., partial P5 sequence, partial P7 sequence, etc.), or a partial sequence of any other type of sequence described elsewhere herein. A partial sequence can contain a contiguous or continuous portion or segment, but not all, of a full sequence, for example. In some cases, a downstream procedure can extend the partial sequence, or derivative thereof, to achieve a full sequence of the partial sequence, or derivative thereof.

Examples of such nucleic acid molecules (*e.g.*, oligonucleotides, polynucleotides, etc.) and uses thereof, as can be used with compositions, devices, methods and systems of the present disclosure, are provided in U.S. Patent Pub. Nos. 2014/0378345 and 2015/0376609, each of which is entirely incorporated herein by reference.

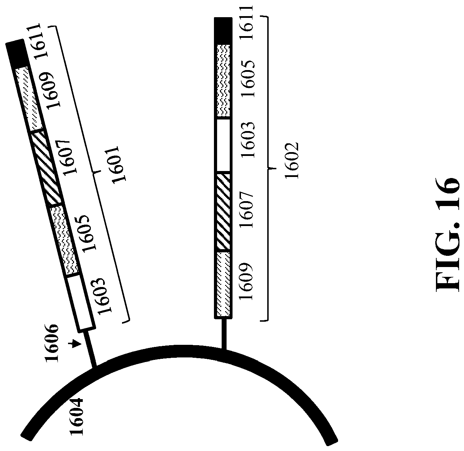

**FIG. 16** illustrates a non-limiting example of a barcode carrying bead. One or more first nucleic acid barcode molecules **1601** *(e.g.,* described in **FIG. 7A****),** can be coupled to a bead **1604** by a releasable linkage **1606,** such as, for example, a disulfide linker. The same bead **1604** can be coupled (*e.g.*, via releasable linkage) to one or more second nucleic acid barcode molecules 1602 (*e.g.*, described in **FIG. 7B** ). In some cases, the first primer sequence **1607** can be or can comprise a functional sequence that can be used in subsequent processing. In some cases, the second primer sequence **1609** can be or can comprise a functional sequence that can be used in subsequent processing. Functional sequences are described herein.

In operation, a biological particle (*e.g*., cell, cell bead, cell nucleus) can be co-partitioned along with a barcode bearing bead **1604.** The first and second nucleic acid barcode molecules **1601** and **1602** can be released from the bead **1604** in the partition. By way of example, in the context of analyzing sample RNA, the capture sequence (*e.g*., **1611)** of a released nucleic acid barcode molecules can hybridize to a mRNA molecule *(e.g.,* to a poly-A sequence of a mRNA molecule). Reverse transcription can result in a cDNA transcript of the mRNA, but which transcript includes each of the sequence segments **1603, 1605, 1607,** and **1609** of the nucleic acid barcode molecule . Within any given partition, the cDNA transcripts of the individual mRNA molecules can include a common barcode sequence segment **1603.** However, the transcripts made from the different mRNA molecules within a given partition can vary at the unique molecular identifier sequence (UMI) segment **1605.**

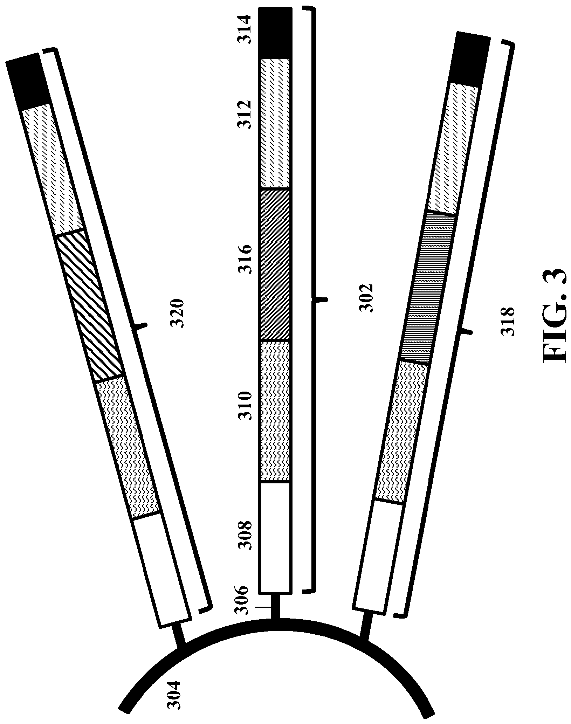

**FIG. 3** illustrates another non-limiting example of a barcode carrying bead. A nucleic acid barcode molecule **302** can be coupled to a bead **304** by a releasable linkage **306,** such as, for example, a disulfide linker. The same bead **304** can be coupled *(e.g.,* via releasable linkage) to one or more other nucleic acid barcode molecules **318, 320.** The nucleic acid barcode molecule **302** can be or comprise a barcode. As noted elsewhere herein, the structure of the barcode can comprise a number of sequence elements. The nucleic acid barcode molecule **302** can comprise a functional sequence **308** that can be used in subsequent processing. For example, the functional sequence **308** can include one or more of a sequencer specific flow cell attachment sequence *(e.g.,* a P5 sequence for Illumina^{®} sequencing systems) and a sequencing primer sequence *(e.g.,* a R1 primer for Illumina^{®} sequencing systems), or partial sequence(s) thereof. The nucleic acid barcode molecule **302** can comprise a barcode sequence **310** for use in barcoding the sample (*e.g*., DNA, RNA, protein, etc.). In some cases, the barcode sequence **310** can be bead-specific such that the barcode sequence **310** is common to all nucleic acid barcode molecules (*e.g.*, including nucleic acid barcode molecule 302) coupled to the same bead **304.** Alternatively or in addition, the barcode sequence **310** can be partition-specific such that the barcode sequence **310** is common to all nucleic acid barcode molecules coupled to one or more beads that are partitioned into the same partition. The nucleic acid barcode molecule **302** can comprise sequence **312** complementary to an analyte of interest, *e.g.,* a priming sequence. Sequence **312** can be a poly-T sequence complementary to a poly-A tail of an mRNA analyte, a targeted priming sequence, and/or a random priming sequence. The nucleic acid barcode molecule **302** can comprise an anchoring sequence **314** to ensure that the specific priming sequence **312** hybridizes at the sequence end *(e.g.,* of the mRNA). For example, the anchoring sequence **314** can include a random short sequence of nucleotides, such as a 1-mer, 2-mer, 3-mer or longer sequence, which can ensure that a poly-T segment is more likely to hybridize at the sequence end of the poly-A tail of the mRNA.

The nucleic acid barcode molecule **302** can comprise a unique molecular identifying sequence **316** *(e.g.,* unique molecular identifier (UMI)). In some cases, the unique molecular identifying sequence **316** can comprise from about 5 to about 8 nucleotides. Alternatively, the unique molecular identifying sequence **316** can compress less than about 5 or more than about 8 nucleotides. The unique molecular identifying sequence **316** can be a unique sequence that varies across individual nucleic acid barcode molecules *(e.g.,* **302, 318, 320,** etc.) coupled to a single bead *(e.g.,* bead **304).** In some cases, the unique molecular identifying sequence **316** can be a random sequence (*e.g.*, such as a random N-mer sequence). For example, the UMI can provide a unique identifier of the starting analyte (e.g., mRNA) molecule that was captured, in order to allow quantitation of the number of original expressed RNA molecules. As will be appreciated, although **FIG. 3** shows three nucleic acid barcode molecules **302, 318, 320** coupled to the surface of the bead **304,** an individual bead can be coupled to any number of individual nucleic acid barcode molecules, for example, from one to tens to hundreds of thousands, millions, or even a billion of individual nucleic acid barcode molecules. The respective barcodes for the individual nucleic acid barcode molecules can comprise both common sequence segments or relatively common sequence segments *(e.g.,* **308, 310, 312,** etc.) and variable or unique sequence segments *(e.g.,* **316)** between different individual nucleic acid barcode molecules coupled to the same bead.

In operation, a biological particle *(e.g.,* cell, DNA, RNA, etc.) can be co-partitioned along with a barcode bearing bead **304.** The nucleic acid barcode molecules **302, 318, 320** can be released from the bead **304** in the partition. By way of example, in the context of analyzing sample RNA, the poly-T segment *(e.g.,* **312)** of one of the released nucleic acid barcode molecules *(e.g.,* **302)** can hybridize to the poly-A tail of a mRNA molecule. Reverse transcription can result in a cDNA transcript of the mRNA, but which transcript includes each of the sequence segments **308, 310, 316** of the nucleic acid barcode molecule **302.** Because the nucleic acid barcode molecule **302** comprises an anchoring sequence **314,** it will more likely hybridize to and prime reverse transcription at the sequence end of the poly-A tail of the mRNA. In some embodiments, cDNA transcripts of the individual mRNA molecules from any given partition can include a common barcode sequence segment **310.** However, the transcripts made from the different mRNA molecules within a given partition can vary at the unique molecular identifying sequence **312** segment (*e.g.*, UMI segment).

Beneficially, even following any subsequent amplification of the contents of a given partition, the number of different UMIs can be indicative of the quantity of mRNA originating from a given partition, and thus from the biological particle (*e.g*., cell). As noted above, the transcripts can be amplified, cleaned up and sequenced to identify the sequence of the cDNA transcript of the mRNA, as well as to sequence the barcode segment and the UMI segment. While a poly-T primer sequence is described, other targeted or random priming sequences can also be used in priming the reverse transcription reaction. Likewise, although described as releasing the barcoded oligonucleotides into the partition, in some cases, the nucleic acid barcode molecules bound to the bead (*e.g.*, gel bead) can be used to hybridize and capture the mRNA on the solid phase of the bead, for example, in order to facilitate the separation of the RNA from other cell contents. In such cases, further processing can be performed, in the partitions or outside the partitions (*e.g.*, in bulk). For instance, the RNA molecules on the beads can be subjected to reverse transcription or other nucleic acid processing, additional adapter sequences can be added to the barcoded nucleic acid molecules, or other nucleic acid reactions (*e.g*., circularization, amplification, nucleic acid extension) can be performed. The beads or products thereof (*e.g.*, barcoded nucleic acid molecules) can be collected from the partitions, and/or pooled together and subsequently subjected to clean up and further characterization (*e.g.*, sequencing).

The operations described herein can be performed at any useful or convenient step. For instance, the beads comprising nucleic acid barcode molecules can be introduced into a partition (e.g., well or droplet) prior to, during, or following introduction of a sample into the partition. The nucleic acid molecules of a sample can be subjected to barcoding, which can occur on the bead (in cases where the nucleic acid molecules remain coupled to the bead) or following release of the nucleic acid barcode molecules into the partition. In cases where analytes from the sample are captured by the nucleic acid barcode molecules in a partition (e.g., by hybridization), captured analytes from various partitions can be collected, pooled, and subjected to further processing (e.g., reverse transcription, adapter attachment, amplification, clean up, sequencing). For example, in cases wherein the nucleic acid molecules from the sample remain attached to the bead, the beads from various partitions can be collected, pooled, and subjected to further processing (e.g., reverse transcription, circularization, adapter attachment, amplification, clean up, sequencing). In other instances, one or more of the processing methods, *e.g*., reverse transcription, can occur in the partition. For example, conditions sufficient for barcoding, circularization, adapter attachment, reverse transcription, or other nucleic acid processing operations can be provided in the partition and performed prior to clean up and sequencing.

In some instances, a bead can comprise a capture sequence or binding sequence configured to bind to a corresponding capture sequence or binding sequence. In some instances, a bead can comprise a plurality of different capture sequences or binding sequences configured to bind to different respective corresponding capture sequences or binding sequences. For example, a bead can comprise a first subset of one or more capture sequences each configured to bind to a first corresponding capture sequence, a second subset of one or more capture sequences each configured to bind to a second corresponding capture sequence, a third subset of one or more capture sequences each configured to bind to a third corresponding capture sequence, and etc. A bead can comprise any number of different capture sequences. In some instances, a bead can comprise at least 2, 3, 4, 5, 6, 7, 8, 9, 10 or more different capture sequences or binding sequences configured to bind to different respective capture sequences or binding sequences, respectively. Alternatively or in addition, a bead can comprise at most about 10, 9, 8, 7, 6, 5, 4, 3, or 2 different capture sequences or binding sequences configured to bind to different respective capture sequences or binding sequences. In some instances, the different capture sequences or binding sequences can be configured to facilitate analysis of a same type of analyte. In some instances, the different capture sequences or binding sequences can be configured to facilitate analysis of different types of analytes (with the same bead). The capture sequence can be designed to attach to a corresponding capture sequence. Beneficially, such corresponding capture sequence can be introduced to, or otherwise induced in, a biological particle *(e.g.,* cell, cell bead, etc.) for performing different assays in various formats *(e.g.,* barcoded antibodies comprising the corresponding capture sequence, barcoded MHC dextramers comprising the corresponding capture sequence, barcoded labelling agents disclosed herein, barcoded guide RNA molecules comprising the corresponding capture sequence, etc.), such that the corresponding capture sequence can later interact with the capture sequence associated with the bead. In some instances, a capture sequence coupled to a bead (or other support) can be configured to attach to a linker molecule, such as a splint molecule, wherein the linker molecule is configured to couple the bead (or other support) to other molecules through the linker molecule, such as to one or more analytes or one or more other linker molecules.

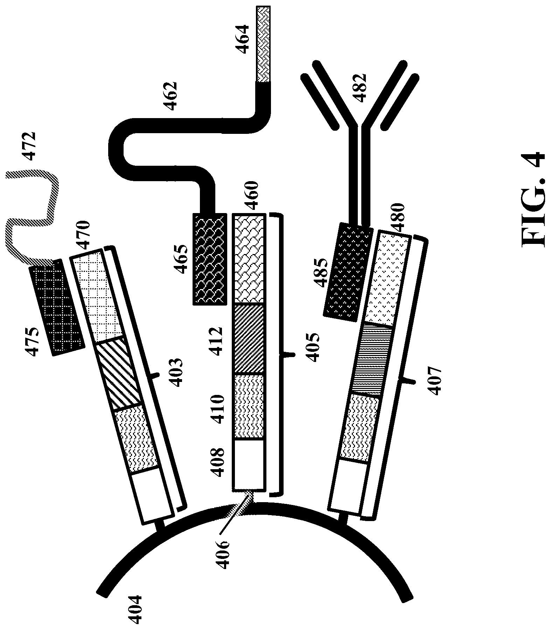

**FIG. 4** illustrates another example of a barcode carrying bead. A nucleic acid barcode molecule **405,** such as an oligonucleotide, can be coupled to a bead **404** by a releasable linkage **406,** such as, for example, a disulfide linker. The nucleic acid barcode molecule **405** can comprise a first capture sequence **460.** The same bead **404** can be coupled (*e.g.*, via releasable linkage) to one or more other nucleic acid molecules **403, 407** comprising other capture sequences. The nucleic acid barcode molecule **405** can be or comprise a barcode. As noted elsewhere herein, the structure of the barcode can comprise a number of sequence elements, such as a functional sequence **408** *(e.g.,* flow cell attachment sequence, sequencing primer sequence, etc.), a barcode sequence **410** (*e.g.*, bead-specific sequence common to bead, partition-specific sequence common to partition, etc.), and a unique molecular identifier **412** (*e.g*., unique sequence within different molecules attached to the bead), or partial sequences thereof. The capture sequence **460** can be configured to attach to a corresponding capture sequence **465.** In some instances, the corresponding capture sequence **465** can be coupled to another molecule that can be an analyte or an intermediary carrier. For example, as illustrated in **FIG. 4****,** the corresponding capture sequence **465** is coupled to a guide RNA molecule **462** comprising a target sequence **464,** wherein the target sequence **464** is configured to attach to the analyte. Another oligonucleotide molecule **407** attached to the bead **404** comprises a second capture sequence **480** which is configured to attach to a second corresponding capture sequence **485.** As illustrated in **FIG. 4****,** the second corresponding capture sequence **485** is coupled to an antibody **482.** In some cases, the antibody **482** can have binding specificity to an analyte (*e.g*., surface protein). Alternatively, the antibody **482** may not have binding specificity. Another oligonucleotide molecule **403** attached to the bead **404** comprises a third capture sequence **470** which is configured to attach to a third corresponding capture sequence **475.** As illustrated in **FIG. 4****,** the third corresponding capture sequence **475** is coupled to a molecule **472.** The molecule **472** may or may not be configured to target an analyte. The other oligonucleotide molecules **403, 407** can comprise the other sequences (*e.g*., functional sequence, barcode sequence, UMI, etc.) described with respect to oligonucleotide molecule **405.** While a single oligonucleotide molecule comprising each capture sequence is illustrated in **FIG. 4****,** it will be appreciated that, for each capture sequence, the bead can comprise a set of one or more oligonucleotide molecules each comprising the capture sequence. For example, the bead can comprise any number of sets of one or more different capture sequences. Alternatively or in addition, the bead **404** can comprise other capture sequences. Alternatively or in addition, the bead **404** can comprise fewer types of capture sequences (*e.g*., two capture sequences). Alternatively or in addition, the bead **404** can comprise oligonucleotide molecule(s) comprising a priming sequence, such as a specific priming sequence such as an mRNA specific priming sequence *(e.g.,* poly-T sequence), a targeted priming sequence, and/or a random priming sequence, for example, to facilitate an assay for gene expression. Alternatively or in addition, the bead **404** can comprise one or more first nucleic acid barcode molecules **701** *(e.g.,* **FIG. 7A****),** one or more second nucleic acid barcode molecules **702** *(e.g.,* **FIG. 7B****),** or one or more first and second nucleic acid molecules **701** and **702.**

In operation, the barcoded oligonucleotides can be released *(e.g.,* in a partition), as described elsewhere herein. Alternatively, the nucleic acid molecules bound to the bead *(e.g.,* gel bead) can be used to hybridize and capture analytes (*e.g*., one or more types of analytes) on the solid phase of the bead.

A bead injected or otherwise introduced into a partition can comprise releasably, cleavably, or reversibly attached barcodes. A bead injected or otherwise introduced into a partition can comprise activatable barcodes. A bead injected or otherwise introduced into a partition can be degradable, disruptable, or dissolvable beads.

Barcodes can be releasably, cleavably or reversibly attached to the beads such that barcodes can be released or be releasable through cleavage of a linkage between the barcode molecule and the bead, or released through degradation of the underlying bead itself, allowing the barcodes to be accessed or be accessible by other reagents, or both. In non-limiting examples, cleavage can be achieved through reduction of di-sulfide bonds, use of restriction enzymes, photo-activated cleavage, or cleavage via other types of stimuli (*e.g.*, chemical, thermal, pH, enzymatic, etc.) and/or reactions, such as described elsewhere herein. Releasable barcodes can sometimes be referred to as being activatable, in that they are available for reaction once released. Thus, for example, an activatable barcode can be activated by releasing the barcode from a bead (or other suitable type of partition described herein). Other activatable configurations are also envisioned in the context of the described methods and systems.

As will be appreciated from the above disclosure, the degradation of a bead can refer to the disassociation of a bound or entrained species from a bead, both with and without structurally degrading the physical bead itself. For example, the degradation of the bead can involve cleavage of a cleavable linkage via one or more species and/or methods described elsewhere herein. In another example, entrained species can be released from beads through osmotic pressure differences due to, for example, changing chemical environments. See, *e.g*., PCT/US2014/044398, which is hereby incorporated by reference in its entirety.

A degradable bead can be introduced into a partition, such as a droplet of an emulsion or a well, such that the bead degrades within the partition and any associated species (*e.g.*, oligonucleotides) are released within the droplet when the appropriate stimulus is applied. The free species (*e.g.*, oligonucleotides, nucleic acid molecules) can interact with other reagents contained in the partition. See, *e.g*., PCT/US2014/044398, which is hereby incorporated by reference in its entirety.

As will be appreciated, barcodes that are releasably, cleavably or reversibly attached to the beads described herein include barcodes that are released or releasable through cleavage of a linkage between the barcode molecule and the bead, or that are released through degradation of the underlying bead itself, allowing the barcodes to be accessed or accessible by other reagents, or both.

In some cases, a species (*e.g.*, oligonucleotide molecules comprising barcodes) that are attached to a solid support *(e.g.,* a bead) can comprise a U-excising element that allows the species to release from the bead. In some cases, the U-excising element can comprise a single-stranded DNA (ssDNA) sequence that contains at least one uracil. The species can be attached to a solid support via the ssDNA sequence containing the at least one uracil. The species can be released by a combination of uracil-DNA glycosylase *(e.g.,* to remove the uracil) and an endonuclease *(e.g.,* to induce an ssDNA break). If the endonuclease generates a 5' phosphate group from the cleavage, then additional enzyme treatment can be included in downstream processing to eliminate the phosphate group, *e.g.,* prior to ligation of additional sequencing handle elements, *e.g.,* Illumina full P5 sequence, partial P5 sequence, full R1 sequence, and/or partial R1 sequence.

The co-partitioned nucleic acid barcode molecules can also comprise other functional sequences useful in the processing of the nucleic acids from the co-partitioned biological particles. These sequences include, *e.g.*, targeted or random/universal amplification primer sequences for amplifying nucleic acids (e.g., mRNA, the genomic DNA) from the individual biological particles within the partitions while attaching the associated barcode sequences, sequencing primers or primer recognition sites, hybridization or probing sequences, *e.g.,* for identification of presence of the sequences or for pulling down barcoded nucleic acids, or any of a number of other potential functional sequences. Other mechanisms of co-partitioning oligonucleotides can also be employed, including, *e.g.,* coalescence of two or more droplets, where one droplet contains oligonucleotides, or microdispensing of oligonucleotides (*e.g.,* attached to a bead) into partitions, e.g., droplets within microfluidic systems.

In an example, beads are provided that each include large numbers of the above described nucleic acid barcode molecules releasably attached to the beads, where all of the nucleic acid barcode molecules attached to a particular bead will include a common nucleic acid barcode sequence, but where a large number of diverse barcode sequences are represented across the population of beads used. In some embodiments, hydrogel beads, *e.g.,* comprising polyacrylamide polymer matrices, are used as a solid support and delivery vehicle for the nucleic acid barcode molecules into the partitions, as they are capable of carrying large numbers of nucleic acid barcode molecules, and can be configured to release those nucleic acid molecules upon exposure to a particular stimulus, as described elsewhere herein. In some cases, the population of beads provides a diverse barcode sequence library that includes at least about 1,000 different barcode sequences, at least about 5,000 different barcode sequences, at least about 10,000 different barcode sequences, at least about 50,000 different barcode sequences, at least about 100,000 different barcode sequences, at least about 1,000,000 different barcode sequences, at least about 5,000,000 different barcode sequences, or at least about 10,000,000 different barcode sequences, or more. In some cases, the population of beads provides a diverse barcode sequence library that includes about 1,000 to about 10,000 different barcode sequences, about 5,000 to about 50,000 different barcode sequences, about 10,000 to about 100,000 different barcode sequences, about 50,000 to about 1,000,000 different barcode sequences, or about 100,000 to about 10,000,000 different barcode sequences.

Additionally, each bead can be provided with large numbers of nucleic acid (*e.g.,* oligonucleotide) molecules attached. In particular, the number of molecules of nucleic acid molecules including the barcode sequence on an individual bead can be at least about 1,000 nucleic acid molecules, at least about 5,000 nucleic acid molecules, at least about 10,000 nucleic acid molecules, at least about 50,000 nucleic acid molecules, at least about 100,000 nucleic acid molecules, at least about 500,000 nucleic acids, at least about 1,000,000 nucleic acid molecules, at least about 5,000,000 nucleic acid molecules, at least about 10,000,000 nucleic acid molecules, at least about 50,000,000 nucleic acid molecules, at least about 100,000,000 nucleic acid molecules, at least about 250,000,000 nucleic acid molecules and in some cases at least about 1 billion nucleic acid molecules, or more. In some embodiments, the number of nucleic acid molecules including the barcode sequence on an individual bead is between about 1,000 to about 10,000 nucleic acid molecules, about 5,000 to about 50,000 nucleic acid molecules, about 10,000 to about 100,000 nucleic acid molecules, about 50,000 to about 1,000,000 nucleic acid molecules, about 100,000 to about 10,000,000 nucleic acid molecules, about 1,000,000 to about 1 billion nucleic acid molecules.

Nucleic acid molecules of a given bead can include identical (or common) barcode sequences, different barcode sequences, or a combination of both. Nucleic acid molecules of a given bead can include multiple sets of nucleic acid molecules. Nucleic acid molecules of a given set can include identical barcode sequences. The identical barcode sequences can be different from barcode sequences of nucleic acid molecules of another set. In some embodiments, such different sequences can be associated with a given bead.

Moreover, when the population of beads is partitioned, the resulting population of partitions can also include a diverse barcode library that includes at least about 1,000 different barcode sequences, at least about 5,000 different barcode sequences, at least about 10,000 different barcode sequences, at least at least about 50,000 different barcode sequences, at least about 100,000 different barcode sequences, at least about 1,000,000 different barcode sequences, at least about 5,000,000 different barcode sequences, or at least about 10,000,000 different barcode sequences. Additionally, each partition of the population can include at least about 1,000 nucleic acid barcode molecules, at least about 5,000 nucleic acid barcode molecules, at least about 10,000 nucleic acid barcode molecules, at least about 50,000 nucleic acid barcode molecules, at least about 100,000 nucleic acid barcode molecules, at least about 500,000 nucleic acids, at least about 1,000,000 nucleic acid barcode molecules, at least about 5,000,000 nucleic acid barcode molecules, at least about 10,000,000 nucleic acid barcode molecules, at least about 50,000,000 nucleic acid barcode molecules, at least about 100,000,000 nucleic acid barcode molecules, at least about 250,000,000 nucleic acid barcode molecules and in some cases at least about 1 billion nucleic acid barcode molecules.

In some cases, the resulting population of partitions provides a diverse barcode sequence library that includes about 1,000 to about 10,000 different barcode sequences, about 5,000 to about 50,000 different barcode sequences, about 10,000 to about 100,000 different barcode sequences, about 50,000 to about 1,000,000 different barcode sequences, or about 100,000 to about 10,000,000 different barcode sequences. Additionally, each partition of the population can include between about 1,000 to about 10,000 nucleic acid barcode molecules, about 5,000 to about 50,000 nucleic acid barcode molecules, about 10,000 to about 100,000 nucleic acid barcode molecules, about 50,000 to about 1,000,000 nucleic acid barcode molecules, about 100,000 to about 10,000,000 nucleic acid barcode molecules, about 1,000,000 to about 1 billion nucleic acid barcode molecules.

In some cases, it can be desirable to incorporate multiple different barcodes within a given partition, either attached to a single or multiple beads within the partition. For example, in some cases, a mixed, but known set of barcode sequences can provide greater assurance of identification in the subsequent processing, *e.g.,* by providing a stronger address or attribution of the barcodes to a given partition, as a duplicate or independent confirmation of the output from a given partition.

The nucleic acid barcode molecules can be releasable from the beads upon the application of a particular stimulus to the beads. In some cases, the stimulus can be a photo-stimulus, *e.g.,* through cleavage of a photo-labile linkage that releases the nucleic acid barcode molecules. In other cases, a thermal stimulus can be used, where elevation of the temperature of the beads environment can result in cleavage of a linkage or other release of the nucleic acid barcode molecules from the beads. In still other cases, a chemical stimulus can be used that cleaves a linkage of the nucleic acid barcode molecules to the beads, or otherwise results in release of the nucleic barcode acid molecules from the beads. In one case, such compositions include the polyacrylamide matrices described above for encapsulation of biological particles, and can be degraded for release of the attached nucleic acid barcode molecules through exposure to a reducing agent, such as DTT.

### Reagents

In accordance with certain aspects, biological particles can be partitioned along with lysis reagents in order to release the contents of the biological particles within the partition. In such cases, the lysis agents can be contacted with the biological particle suspension concurrently with, or immediately prior to, the introduction of the biological particles into the partitioning junction/droplet generation zone *(e.g.,* junction **210**)*,* such as through an additional channel or channels upstream of the channel junction. In accordance with other aspects, additionally or alternatively, biological particles can be partitioned along with other reagents, as will be described further below.

The methods and systems of the present disclosure can comprise microfluidic devices and methods of use thereof, which can be used for co-partitioning biological particles with reagents. Such systems and methods are described in U.S. Patent Publication No. US/20190367997, which is herein incorporated by reference in its entirety for all purposes.

Beneficially, when lysis reagents and biological particles are co-partitioned, the lysis reagents can facilitate the release of the contents of the biological particles within the partition. The contents released in a partition can remain discrete from the contents of other partitions.

As will be appreciated, the channel segments of the microfluidic devices described elsewhere herein can be coupled to any of a variety of different fluid sources or receiving components, including reservoirs, tubing, manifolds, or fluidic components of other systems. As will be appreciated, the microfluidic channel structures can have various geometries and/or configurations. For example, a microfluidic channel structure can have more than two channel junctions. For example, a microfluidic channel structure can have 2, 3, 4, 5 channel segments or more each carrying the same or different types of beads, reagents, and/or biological particles that meet at a channel junction. Fluid flow in each channel segment can be controlled to control the partitioning of the different elements into droplets. Fluid can be directed flow along one or more channels or reservoirs via one or more fluid flow units. A fluid flow unit can comprise compressors (*e.g.,* providing positive pressure), pumps (*e.g.,* providing negative pressure), actuators, and the like to control flow of the fluid. Fluid can also or otherwise be controlled via applied pressure differentials, centrifugal force, electrokinetic pumping, vacuum, capillary or gravity flow, or the like.

Examples of lysis agents include bioactive reagents, such as lysis enzymes that are used for lysis of different cell types, *e.g.,* gram positive or negative bacteria, plants, yeast, mammalian, etc., such as lysozymes, achromopeptidase, lysostaphin, labiase, kitalase, lyticase, and a variety of other lysis enzymes available from, *e.g.,* Sigma-Aldrich, Inc. (St Louis, MO), as well as other commercially available lysis enzymes. Other lysis agents can additionally or alternatively be co-partitioned with the biological particles to cause the release of the biological particle's contents into the partitions. For example, in some cases, surfactant-based lysis solutions can be used to lyse cells, although these can be less desirable for emulsion based systems where the surfactants can interfere with stable emulsions. In some cases, lysis solutions can include non-ionic surfactants such as, for example, TritonX-100 and Tween 20. In some cases, lysis solutions can include ionic surfactants such as, for example, sarcosyl and sodium dodecyl sulfate (SDS). Electroporation, thermal, acoustic or mechanical cellular disruption can also be used in certain cases, *e.g.,* non-emulsion-based partitioning such as encapsulation of biological particles that can be in addition to or in place of droplet partitioning, where any pore size of the encapsulate is sufficiently small to retain nucleic acid fragments of a given size, following cellular disruption.

Alternatively or in addition to the lysis agents co-partitioned with the biological particles described above, other reagents can also be co-partitioned with the biological particles, including, for example, DNase and RNase inactivating agents or inhibitors, such as proteinase K, chelating agents, such as EDTA, and other reagents employed in removing or otherwise reducing negative activity or impact of different cell lysate components on subsequent processing of nucleic acids. In addition, in the case of encapsulated biological particles (*e.g.,* a cell or a nucleus in a polymer matrix), the biological particles can be exposed to an appropriate stimulus to release the biological particles or their contents from a co-partitioned bead. For example, in some cases, a chemical stimulus can be co-partitioned along with an encapsulated biological particle to allow for the degradation of the bead and release of the cell or its contents into the larger partition. In some cases, this stimulus can be the same as the stimulus described elsewhere herein for release of nucleic acid molecules (*e.g.,* oligonucleotides) from their respective bead. In alternative examples, this can be a different and non-overlapping stimulus, in order to allow an encapsulated biological particle to be released into a partition at a different time from the release of nucleic acid molecules into the same partition. For a description of methods, compositions, and systems for encapsulating cells (also referred to as a "cell bead"), see, *e.g.,* U.S. Pat. 10,428,326 and U.S. Pat. Pub. 20190100632, which are each incorporated by reference in their entirety.

Additional reagents can also be co-partitioned with the biological particle, such as endonucleases to fragment a biological particle's DNA, DNA polymerase enzymes and dNTPs used to amplify the biological particle's nucleic acid fragments and to attach the barcode molecular tags to the amplified fragments. Other enzymes can be co-partitioned, including without limitation, polymerase, transposase, circularization enzymes disclosed herein, splint molecules, ligase, proteinase K, DNAse, etc. Additional reagents can also include reverse transcriptase enzymes, including enzymes with terminal transferase activity, primers and oligonucleotides, and switch oligonucleotides (also referred to herein as "switch oligos" or "template switching oligonucleotides") which can be used for template switching.

In some cases, template switching can be used to increase the length of a cDNA. In some cases, template switching can be used to append a predefined nucleic acid sequence to the cDNA. Template switching is further described in PCT/US2017/068320, which is hereby incorporated by reference in its entirety. Template switching oligonucleotides can comprise a hybridization region and a template region. Template switching oligonucleotides are further described in PCT/US2017/068320, which is hereby incorporated by reference in its entirety.

Any of the reagents described in this disclosure can be encapsulated in, or otherwise coupled to, a droplet, or bead, with any chemicals, particles, and elements suitable for sample processing reactions involving biomolecules, such as, but not limited to, nucleic acid molecules and proteins. For example, a bead or droplet used in a sample preparation reaction for DNA sequencing can comprise one or more of the following reagents: enzymes, restriction enzymes (*e.g.,* multiple cutters), ligase, polymerase, fluorophores, oligonucleotide barcodes, adapters, buffers, nucleotides *(e.g.,* dNTPs, ddNTPs) and the like.

Additional examples of reagents include, but are not limited to: buffers, acidic solution, basic solution, temperature-sensitive enzymes, pH-sensitive enzymes, light-sensitive enzymes, metals, metal ions, magnesium chloride, sodium chloride, manganese, aqueous buffer, mild buffer, ionic buffer, inhibitor, enzyme, protein, polynucleotide, antibodies, saccharides, lipid, oil, salt, ion, detergents, ionic detergents, non-ionic detergents, and oligonucleotides.

Once the contents of the cells are released into their respective partitions, the macromolecular components (*e.g.,* macromolecular constituents of biological particles, such as RNA, DNA, or proteins) contained therein can be further processed within the partitions. In accordance with the methods and systems described herein, the macromolecular component contents of individual biological particles can be provided with unique identifiers such that, upon characterization of those macromolecular components they can be attributed as having been derived from the same biological particle or particles. The ability to attribute characteristics to individual biological particles or groups of biological particles is provided by the assignment of unique identifiers specifically to an individual biological particle or groups of biological particles. Unique identifiers, *e.g.,* in the form of nucleic acid barcodes can be assigned or associated with individual biological particles or populations of biological particles, in order to tag or label the biological particle's macromolecular components (and as a result, its characteristics) with the unique identifiers. These unique identifiers can then be used to attribute the biological particle's components and characteristics to an individual biological particle or group of biological particles. In some aspects, this is performed by co-partitioning the individual biological particle or groups of biological particles with the unique identifiers, such as described above (with reference to **FIGS. 1** or **2**).

In some cases, additional beads can be used to deliver additional reagents to a partition. In such cases, it can be advantageous to introduce different beads into a common channel or droplet generation junction, from different bead sources (*e.g.,* containing different associated reagents) through different channel inlets into such common channel or droplet generation junction. In such cases, the flow and frequency of the different beads into the channel or junction can be controlled to provide for a certain ratio of beads from each source, while ensuring a given pairing or combination of such beads into a partition with a given number of biological particles (*e.g.,* one biological particle and one bead per partition).

In some embodiments, following the generation of barcoded nucleic acid molecules according to methods disclosed herein, subsequent operations that can be performed can include generation of amplification products, purification (*e.g.,* via solid phase reversible immobilization (SPRI)), further processing (*e.g.,* shearing, ligation of functional sequences, and subsequent amplification *(e.g.,* via PCR)). These operations can occur in bulk *(e.g.,* outside the partition). In the case where a partition is a droplet in an emulsion, the emulsion can be broken and the contents of the droplet pooled for additional operations.

The methods and systems described herein can be used to greatly increase the efficiency of single cell applications and/or other applications receiving droplet-based input. For example, following the sorting of occupied cells and/or appropriately-sized cells, subsequent operations that can be performed can include generation of amplification products, purification (*e.g.,* via solid phase reversible immobilization (SPRI)), further processing (*e.g.,* shearing, ligation of functional sequences, and subsequent amplification *(e.g.,* via PCR)). These operations can occur in bulk *(e.g.,* outside the partition). In the case where a partition is a droplet in an emulsion, the emulsion can be broken and the contents of the droplet pooled for additional operations. Additional reagents that can be co-partitioned along with the barcode bearing bead can include oligonucleotides to block ribosomal RNA (rRNA) and nucleases to digest genomic DNA from cells. Alternatively, rRNA removal agents can be applied during additional processing operations. The configuration of the constructs generated by such a method can help minimize (or avoid) sequencing of the poly-T sequence during sequencing and/or sequence the 5' end of a polynucleotide sequence. The amplification products, for example, first amplification products and/or second amplification products, can be subject to sequencing for sequence analysis. In some cases, amplification can be performed using the Partial Hairpin Amplification for Sequencing (PHASE) method.

A variety of applications require the evaluation of the presence and quantification of different biological particle or organism types within a population of biological particles, including, for example, microbiome analysis and characterization, environmental testing, food safety testing, epidemiological analysis, *e.g.,* in tracing contamination or the like.

### Wells

As described herein, one or more processes can be performed in a partition, which can be a well. The well can be a well of a plurality of wells of a substrate, such as a microwell of a microwell array or plate, or the well can be a microwell or microchamber of a device *(e.g.,* microfluidic device) comprising a substrate. The well can be a well of a well array or plate, or the well can be a well or chamber of a device *(e.g.,* fluidic device). In some embodiments, a well of a fluidic device is fluidically connected to another well of the fluidic device. Accordingly, the wells or microwells can assume an "open" configuration, in which the wells or microwells are exposed to the environment (*e.g.,* contain an open surface) and are accessible on one planar face of the substrate, or the wells or microwells can assume a "closed" or "sealed" configuration, in which the microwells are not accessible on a planar face of the substrate. In some instances, the wells or microwells can be configured to toggle between "open" and "closed" configurations. For instance, an "open" microwell or set of microwells can be "closed" or "sealed" using a membrane (*e.g.,* semi-permeable membrane), an oil (*e.g.,* fluorinated oil to cover an aqueous solution), or a lid, as described elsewhere herein.

The well can have a volume of less than 1 milliliter (mL). For instance, the well can be configured to hold a volume of at most 1000 microliters (µL), at most 100 µL, at most 10 µL, at most 1 µL, at most 100 nanoliters (nL), at most 10 nL, at most 1 nL, at most 100 picoliters (pL), at most 10 (pL), or less. The well can be configured to hold a volume of about 1000 µL, about 100 µL, about 10 µL, about 1 µL, about 100 nL, about 10 nL, about 1 nL, about 100 pL, about 10 pL, etc. The well can be configured to hold a volume of at least 10 pL, at least 100 pL, at least 1 nL, at least 10 nL, at least 100 nL, at least 1 µL, at least 10 µL, at least 100 µL, at least 1000 µL, or more. The well can be configured to hold a volume in a range of volumes listed herein, for example, from about 5 nL to about 20 nL, from about 1 nL to about 100 nL, from about 500 pL to about 100 µL, etc. The well can be of a plurality of wells that have varying volumes and can be configured to hold a volume appropriate to accommodate any of the partition volumes described herein.

In some instances, a microwell array or plate comprises a single variety of microwells. In some instances, a microwell array or plate comprises a variety of microwells. For instance, the microwell array or plate can comprise one or more types of microwells within a single microwell array or plate. The types of microwells can have different dimensions (*e.g.,* length, width, diameter, depth, cross-sectional area, etc.), shapes (*e.g.,* circular, triangular, square, rectangular, pentagonal, hexagonal, heptagonal, octagonal, nonagonal, decagonal, etc.), aspect ratios, or other physical characteristics. The microwell array or plate can comprise any number of different types of microwells. For example, the microwell array or plate can comprise 1, 2, 3, 4, 5, 6, 7, 8, 9, 10, 20, 30, 40, 50, 60, 70, 80, 90, 100, 200, 300, 400, 500, 600, 700, 800, 900, 1000 or more different types of microwells. A well can have any dimension (*e.g.,* length, width, diameter, depth, cross-sectional area, volume, etc.), shape (*e.g.,* circular, triangular, square, rectangular, pentagonal, hexagonal, heptagonal, octagonal, nonagonal, decagonal, other polygonal, etc.), aspect ratios, or other physical characteristics described herein with respect to any well.

In certain instances, the microwell array or plate comprises different types of microwells that are located adjacent to one another within the array or plate. For instance, a microwell with one set of dimensions can be located adjacent to and in contact with another microwell with a different set of dimensions. Similarly, microwells of different geometries can be placed adjacent to or in contact with one another. The adjacent microwells can be configured to hold different articles; for example, one microwell can be used to contain a cell, cell bead, or other sample (*e.g.,* cellular components, nucleic acid molecules, etc.) while the adjacent microwell can be used to contain a microcapsule, droplet, bead, or other reagent. In some cases, the adjacent microwells can be configured to merge the contents held within, *e.g.,* upon application of a stimulus, or spontaneously, upon contact of the articles in each microwell.

As is described elsewhere herein, a plurality of partitions can be used in the systems, compositions, and methods described herein. For example, any suitable number of partitions *(e.g.,* wells or droplets) can be generated or otherwise provided. For example, in the case when wells are used, at least about 1,000 wells, at least about 5,000 wells, at least about 10,000 wells, at least about 50,000 wells, at least about 100,000 wells, at least about 500,000 wells, at least about 1,000,000 wells, at least about 5,000,000 wells at least about 10,000,000 wells, at least about 50,000,000 wells, at least about 100,000,000 wells, at least about 500,000,000 wells, at least about 1,000,000,000 wells, or more wells can be generated or otherwise provided. Moreover, the plurality of wells can comprise both unoccupied wells *(e.g.,* empty wells) and occupied wells.

A well can comprise any of the reagents described herein, or combinations thereof. These reagents can include, for example, barcode molecules, enzymes, adapters, and combinations thereof. The reagents can be physically separated from a sample *(e.g.,* a cell, cell bead, or cellular components, *e.g.,* proteins, nucleic acid molecules, etc.) that is placed in the well. This physical separation can be accomplished by containing the reagents within, or coupling to, a microcapsule or bead that is placed within a well. The physical separation can also be accomplished by dispensing the reagents in the well and overlaying the reagents with a layer that is, for example, dissolvable, meltable, or permeable prior to introducing the polynucleotide sample into the well. This layer can be, for example, an oil, wax, membrane *(e.g.,* semi-permeable membrane), or the like. The well can be sealed at any point, for example, after addition of the microcapsule or bead, after addition of the reagents, or after addition of either of these components. The sealing of the well can be useful for a variety of purposes, including preventing escape of beads or loaded reagents from the well, permitting select delivery of certain reagents *(e.g.,* via the use of a semi-permeable membrane), for storage of the well prior to or following further processing, etc.

Once sealed, the well can be subjected to conditions for further processing of a cell (or cells) in the well. For instance, reagents in the well can allow further processing of the cell, *e.g.,* cell lysis, as further described herein. Alternatively, the well (or wells such as those of a well-based array) comprising the cell (or cells) can be subjected to freeze-thaw cycling to process the cell (or cells), *e.g.,* cell lysis. The well containing the cell can be subjected to freezing temperatures *(e.g.,* 0°C, below 0°C, -5°C, -10°C, -15°C, -20°C, -25°C, -30°C, -35°C, -40°C, -45°C, -50°C, -55°C, - 60°C, -65°C, -70°C, -80°C, or -85°C). Freezing can be performed in a suitable manner, e.g., sub-zero freezer or a dry ice/ethanol bath. Following an initial freezing, the well (or wells) comprising the cell (or cells) can be subjected to freeze-thaw cycles to lyse the cell (or cells). In one embodiment, the initially frozen well (or wells) can be thawed to a temperature above freezing *(e.g.,* 4°C or above, 8°C or above, 12°C or above, 16°C or above, 20°C or above, room temperature, or 25°C or above). In another embodiment, the freezing can be performed for less than 10 minutes *(e.g.,* 5 minutes or 7 minutes) followed by thawing at room temperature for less than 10 minutes *(e.g.,* 5 minutes or 7 minutes). This freeze-thaw cycle can be repeated a number of times, *e.g.,* 2, 3, 4 or more times, to obtain lysis of the cell (or cells) in the well (or wells). In one embodiment, the freezing, thawing and/or freeze/thaw cycling can be performed in the absence of a lysis buffer. Additional disclosure related to freeze-thaw cycling is provided in PCT/US2019/019115, which is incorporated herein by reference in its entirety.

A well can comprise free reagents and/or reagents encapsulated in, or otherwise coupled to or associated with beads or droplets.

The wells can be provided as a part of a kit. For example, a kit can comprise instructions for use, a microwell array or device, and reagents *(e.g.,* beads). The kit can comprise any useful reagents for performing the processes described herein, *e.g.,* nucleic acid reactions, barcoding of nucleic acid molecules, sample processing *(e.g.,* for cell lysis, fixation, and/or permeabilization).

In some cases, a well comprises a bead or droplet that comprises a set of reagents that has a similar attribute *(e.g.,* a set of enzymes, a set of minerals, a set of oligonucleotides, a mixture of different barcode molecules, a mixture of identical barcode molecules). In other cases, a bead or droplet comprises a heterogeneous mixture of reagents. In some cases, the heterogeneous mixture of reagents can comprise all components necessary to perform a reaction. In some cases, such mixture can comprise all components necessary to perform a reaction, except for 1, 2, 3, 4, 5, or more components necessary to perform a reaction. In some cases, such additional components are contained within, or otherwise coupled to, a different microcapsule, droplet, or bead, or within a solution within a partition *(e.g.,* microwell) of the system.

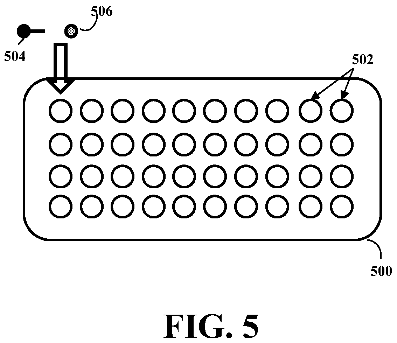

**FIG. 5** schematically illustrates a non-limiting example of a microwell array. The array can be contained within a substrate **500.** The substrate **500** comprises a plurality of wells **502.** The wells **502** can be of any size or shape, and the spacing between the wells, the number of wells per substrate, as well as the density of the wells on the substrate **500** can be modified, depending on the particular application. In one such example application, a sample molecule **506,** which can comprise a cell or cellular components *(e.g.,* nucleic acid molecules) is co-partitioned with a bead **504,** which can comprise a nucleic acid barcode molecule coupled thereto. The wells **502** can be loaded using gravity or other loading technique *(e.g.,* centrifugation, liquid handler, acoustic loading, optoelectronic, etc.). In some instances, at least one of the wells **502** contains a single sample molecule **506** *(e.g.,* cell) and a single bead **504.**

Reagents can be loaded into a well either sequentially or concurrently. In some cases, reagents are introduced to the device either before or after a particular operation. In some cases, reagents (which can be provided, in certain instances, in droplets, or beads) are introduced sequentially such that different reactions or operations occur at different steps. The reagents (or droplets, or beads) can also be loaded at operations interspersed with a reaction or operation step. For example, droplets or beads comprising reagents for fragmenting polynucleotides *(e.g.,* restriction enzymes) and/or other enzymes *(e.g.,* transposases, ligases, polymerases, etc.) can be loaded into the well or plurality of wells, followed by loading of microcapsules, droplets, or beads comprising reagents for attaching nucleic acid barcode molecules to a sample nucleic acid molecule. Reagents can be provided concurrently or sequentially with a sample, *e.g.,* a cell or cellular components *(e.g.,* organelles, proteins, nucleic acid molecules, carbohydrates, lipids, etc.). Accordingly, use of wells can be useful in performing multi-step operations or reactions.

As described elsewhere herein, the nucleic acid barcode molecules and other reagents can be contained within a bead, or droplet. These beads, or droplets can be loaded into a partition *(e.g.,* a microwell) before, after, or concurrently with the loading of a cell, such that each cell is contacted with a different bead, or droplet. This technique can be used to attach a unique nucleic acid barcode molecule to nucleic acid molecules obtained from each cell. Alternatively or in addition to, the sample nucleic acid molecules can be attached to a support. For instance, the partition *(e.g.,* microwell) can comprise a bead which has coupled thereto a plurality of nucleic acid barcode molecules. The sample nucleic acid molecules, or derivatives thereof, can couple or attach to the nucleic acid barcode molecules on the support. The resulting barcoded nucleic acid molecules can then be removed from the partition, and in some instances, pooled and sequenced. In such cases, the nucleic acid barcode sequences can be used to trace the origin of the sample nucleic acid molecule. For example, polynucleotides with identical barcodes can be determined to originate from the same cell or partition, while polynucleotides with different barcodes can be determined to originate from different cells or partitions.

The samples or reagents can be loaded in the wells or microwells using a variety of approaches. The samples *(e.g.,* a cell, cell bead, or cellular component) or reagents (as described herein) can be loaded into the well or microwell using an external force, *e.g.,* gravitational force, electrical force, magnetic force, or using mechanisms to drive the sample or reagents into the well, *e.g.,* via pressure-driven flow, centrifugation, optoelectronics, acoustic loading, electrokinetic pumping, vacuum, capillary flow, etc. In certain cases, a fluid handling system can be used to load the samples or reagents into the well. The loading of the samples or reagents can follow a Poissonian distribution or a non-Poissonian distribution, e.g., super Poisson or sub-Poisson. The geometry, spacing between wells, density, and size of the microwells can be modified to accommodate a useful sample or reagent distribution; for instance, the size and spacing of the microwells can be adjusted such that the sample or reagents can be distributed in a super-Poissonian fashion.

In one particular non-limiting example, the microwell array or plate comprises pairs of microwells, in which each pair of microwells is configured to hold a droplet (e.g., comprising a single cell) and a single bead (such as those described herein, which can, in some instances, also be encapsulated in a droplet). The droplet and the bead (or droplet containing the bead) can be loaded simultaneously or sequentially, and the droplet and the bead can be merged, *e.g.,* upon contact of the droplet and the bead, or upon application of a stimulus *(e.g.,* external force, agitation, heat, light, magnetic or electric force, etc.). In some cases, the loading of the droplet and the bead is super-Poissonian. In other examples of pairs of microwells, the wells are configured to hold two droplets comprising different reagents and/or samples, which are merged upon contact or upon application of a stimulus. In such instances, the droplet of one microwell of the pair can comprise reagents that can react with an agent in the droplet of the other microwell of the pair. For instance, one droplet can comprise reagents that are configured to release the nucleic acid barcode molecules of a bead contained in another droplet, located in the adjacent microwell. Upon merging of the droplets, the nucleic acid barcode molecules can be released from the bead into the partition *(e.g.,* the microwell or microwell pair that are in contact), and further processing can be performed *(e.g.,* barcoding, nucleic acid reactions, etc.). In cases where intact or live cells are loaded in the microwells, one of the droplets can comprise lysis reagents for lysing the cell upon droplet merging.

A droplet or bead can be partitioned into a well. The droplets can be selected or subjected to pre-processing prior to loading into a well. For instance, the droplets can comprise cells, and only certain droplets, such as those containing a single cell (or at least one cell), can be selected for use in loading of the wells. Such a pre-selection process can be useful in efficient loading of single cells, such as to obtain a non-Poissonian distribution, or to pre-filter cells for a selected characteristic prior to further partitioning in the wells. Additionally, the technique can be useful in obtaining or preventing cell doublet or multiplet formation prior to or during loading of the microwell.

In some instances, the wells can comprise nucleic acid barcode molecules attached thereto. The nucleic acid barcode molecules can be attached to a surface of the well *(e.g.,* a wall of the well). The nucleic acid barcode molecules can be attached to a droplet or bead that has been partitioned into the well. The nucleic acid barcode molecule *(e.g.,* a partition barcode sequence) of one well can differ from the nucleic acid barcode molecule of another well, which can permit identification of the contents contained with a single partition or well. In some cases, the nucleic acid barcode molecule can comprise a spatial barcode sequence that can identify a spatial coordinate of a well, such as within the well array or well plate. In some cases, the nucleic acid barcode molecule can comprise a unique molecular identifier for individual molecule identification. In some instances, the nucleic acid barcode molecules can be configured to attach to or capture a nucleic acid molecule within a sample or cell distributed in the well. For example, the nucleic acid barcode molecules can comprise a capture sequence that can be used to capture or hybridize to a nucleic acid molecule *(e.g.,* RNA, DNA) within the sample. In some instances, the nucleic acid barcode molecules can be releasable from the microwell. In some instances, the nucleic acid barcode molecule can be releasable from the bead or droplet. For instance, the nucleic acid barcode molecules can comprise a chemical crosslinker which can be cleaved upon application of a stimulus *(e.g.,* photo-, magnetic, chemical, biological, stimulus). The released nucleic acid barcode molecules, which can be hybridized or configured to hybridize to a sample nucleic acid molecule, can be collected and pooled for further processing, which can include nucleic acid processing *(e.g.,* amplification, extension, reverse transcription, etc.) and/or characterization *(e.g.,* sequencing). In some instances nucleic acid barcode molecules attached to a bead or droplet in a well can be hybridized to sample nucleic acid molecules, and the bead with the sample nucleic acid molecules hybridized thereto can be collected and pooled for further processing, which can include nucleic acid processing *(e.g.,* amplification, extension, reverse transcription, etc.) and/or characterization *(e.g.,* sequencing). In such cases, the unique partition barcode sequences can be used to identify the cell or partition from which a nucleic acid molecule originated.

Characterization of samples within a well can be performed. Such characterization can include, in non-limiting examples, imaging of the sample *(e.g.,* cell, cell bead, or cellular components) or derivatives thereof. Characterization techniques such as microscopy or imaging can be useful in measuring sample profiles in fixed spatial locations. For instance, when cells are partitioned, optionally with beads, imaging of each microwell and the contents contained therein can provide useful information on cell doublet formation *(e.g.,* frequency, spatial locations, etc.), cell-bead pair efficiency, cell viability, cell size, cell morphology, expression level of a biomarker *(e.g.,* a surface marker, a fluorescently labeled molecule therein, etc.), cell or bead loading rate, number of cell-bead pairs, etc. In some instances, imaging can be used to characterize live cells in the wells, including, but not limited to: dynamic live-cell tracking, cell-cell interactions (when two or more cells are co-partitioned), cell proliferation, etc. Alternatively or in addition to, imaging can be used to characterize a quantity of amplification products in the well.

In operation, a well can be loaded with a sample and reagents, simultaneously or sequentially. When cells or cell beads are loaded, the well can be subjected to washing, *e.g.,* to remove excess cells from the well, microwell array, or plate. Similarly, washing can be performed to remove excess beads or other reagents from the well, microwell array, or plate. In the instances where live cells are used, the cells can be lysed in the individual partitions to release the intracellular components or cellular analytes. Alternatively, the cells can be fixed or permeabilized in the individual partitions. The intracellular components or cellular analytes can couple to a support, *e.g.,* on a surface of the microwell, on a solid support *(e.g.,* bead), or they can be collected for further downstream processing. For instance, after cell lysis, the intracellular components or cellular analytes can be transferred to individual droplets or other partitions for barcoding. Alternatively, or in addition to, the intracellular components or cellular analytes *(e.g.,* nucleic acid molecules) can couple to a bead comprising a nucleic acid barcode molecule; subsequently, the bead can be collected and further processed, *e.g.,* subjected to nucleic acid reaction such as reverse transcription, amplification, or extension, and the nucleic acid molecules thereon can be further characterized, *e.g.,* via sequencing. Alternatively, or in addition to, the intracellular components or cellular analytes can be barcoded in the well *(e.g.,* using a bead comprising nucleic acid barcode molecules that are releasable or on a surface of the microwell comprising nucleic acid barcode molecules). The barcoded nucleic acid molecules or analytes can be further processed in the well, or the barcoded nucleic acid molecules or analytes can be collected from the individual partitions and subjected to further processing outside the partition. Further processing can include nucleic acid processing *(e.g.,* performing an amplification, extension) or characterization *(e.g.,* fluorescence monitoring of amplified molecules, sequencing). At any convenient or useful step, the well (or microwell array or plate) can be sealed *(e.g.,* using an oil, membrane, wax, etc.), which enables storage of the assay or selective introduction of additional reagents.

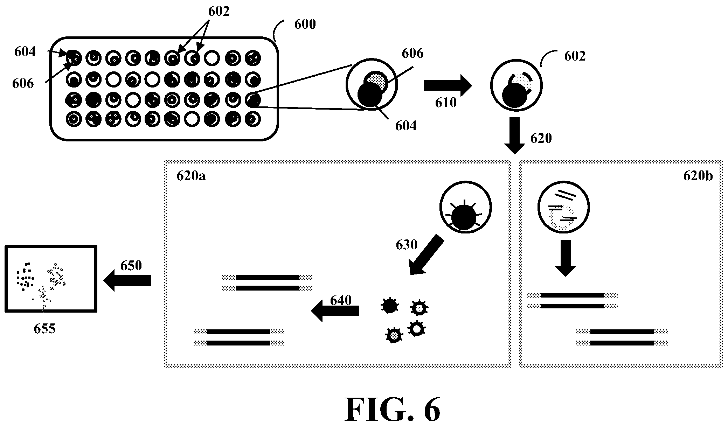

**FIG. 6** schematically shows an example workflow for processing nucleic acid molecules within a sample. A substrate **600** comprising a plurality of microwells **602** can be provided. A sample **606** which can comprise a cell, cell bead, cellular components or analytes *(e.g.,* proteins and/or nucleic acid molecules) can be co-partitioned, in a plurality of microwells **602,** with a plurality of beads **604** comprising nucleic acid barcode molecules. During process **610,** the sample **606** can be processed within the partition. For instance, in the case of live cells, the cell can be subjected to conditions sufficient to lyse the cells and release the analytes contained therein. In process **620,** the bead **604** can be further processed. By way of example, processes **620a** and **620b** schematically illustrate different workflows, depending on the properties of the bead **604.**

In **620a,** the bead comprises nucleic acid barcode molecules that are attached thereto, and sample nucleic acid molecules (*e.g*., RNA, DNA) can attach, *e.g*., via hybridization of ligation, to the nucleic acid barcode molecules. Such attachment can occur on the bead. In process **630,** the beads **604** from multiple wells **602** can be collected and pooled. Further processing can be performed in process **640.** For example, one or more nucleic acid reactions can be performed, such as reverse transcription, nucleic acid extension, amplification, ligation, transposition, etc. In some instances, adapter sequences are ligated to the nucleic acid molecules, or derivatives thereof, as described elsewhere herein. For instance, sequencing primer sequences can be appended to each end of the nucleic acid molecule. In process **650,** further characterization, such as sequencing can be performed to generate sequencing reads. The sequencing reads can yield information on individual cells or populations of cells, which can be represented visually or graphically, *e.g*., in a plot **655.**

In **620b,** the bead comprises nucleic acid barcode molecules that are releasably attached thereto, as described below. The bead can degrade or otherwise release the nucleic acid barcode molecules into the well **602;** the nucleic acid barcode molecules can then be used to barcode nucleic acid molecules within the well **602.** Further processing can be performed either inside the partition or outside the partition. For example, one or more nucleic acid reactions can be performed, such as reverse transcription, nucleic acid extension, amplification, ligation, transposition, etc. In some instances, adapter sequences are ligated to the nucleic acid molecules, or derivatives thereof, as described elsewhere herein. For instance, sequencing primer sequences can be appended to each end of the nucleic acid molecule. In process **650,** further characterization, such as sequencing can be performed to generate sequencing reads. The sequencing reads can yield information on individual cells or populations of cells, which can be represented visually or graphically, *e.g.,* in a plot **655.**

### Samples

A sample can derive from any useful source including any subject, such as a human subject. A sample can comprise material *(e.g.,* one or more biological particles) from one or more different sources, such as one or more different subjects. Multiple samples, such as multiple samples from a single subject *(e.g.,* multiple samples obtained in the same or different manners from the same or different bodily locations, and/or obtained at the same or different times *(e.g.,* seconds, minutes, hours, days, weeks, months, or years apparat)), or multiple samples from different subjects, can be obtained for analysis as described herein. For example, a first sample can be obtained from a subject at a first time and a second sample can be obtained from the subject at a second time later than the first time. The first time can be before a subject undergoes a treatment regimen or procedure *(e.g.,* to address a disease or condition), and the second time can be during or after the subject undergoes the treatment regimen or procedure. In another example, a first sample can be obtained from a first bodily location or system of a subject *(e.g.,* using a first collection technique) and a second sample can be obtained from a second bodily location or system of the subject *(e.g.,* using a second collection technique), which second bodily location or system can be different than the first bodily location or system. In another example, multiple samples can be obtained from a subject at a same time from the same or different bodily locations. Different samples, such as different subjects collected from different bodily locations of a same subject, at different times, from multiple different subjects, and/or using different collection techniques, can undergo the same or different processing *(e.g.,* as described herein). For example, a first sample can undergo a first processing protocol and a second sample can undergo a second processing protocol. In another example, a first portion of a sample can undergo a first processing protocol and a second portion of the sample can undergo a second processing protocol.

A sample can be a biological sample, such as a cell sample *(e.g.,* as described herein). A sample can include one or more biological particles, such as one or more cells and/or cellular constituents, such as one or more cell nuclei. A sample can be a tissue sample. For example, a sample can comprise a plurality of biological particles, such as a plurality of cells and/or cellular constituents. Biological particles *(e.g.,* cells or cellular constituents, such as cell nuclei) of a sample can be of a single type or a plurality of different types. For example, cells of a sample can include one or more different types or blood cells.

Cells and cellular constituents of a sample can be of any type. For example, a cell or cellular constituent can be a vertebral, mammalian, fungal, plant, bacterial, or other cell type. In some cases, the cell is a mammalian cell, such as a human cell. The cell can be, for example, a stem cell, liver cell, nerve cell, bone cell, blood cell, reproductive cell, skin cell, skeletal muscle cell, cardiac muscle cell, smooth muscle cell, hair cell, hormone-secreting cell, or glandular cell. The cell can be, for example, an erythrocyte *(e.g.,* red blood cell), a megakaryocyte *(e.g.,* platelet precursor), a monocyte *(e.g.,* white blood cell), a leukocyte, a B cell, a T cell (such as a helper, suppressor, cytotoxic, or natural killer T cell), an osteoclast, a dendritic cell, a connective tissue macrophage, an epidermal Langerhans cell, a microglial cell, a granulocyte, a hybridoma cell, a mast cell, a natural killer cell, a reticulocyte, a hematopoietic stem cell, a myoepithelial cell, a myeloid-derived suppressor cell, a platelet, a thymocyte, a satellite cell, an epithelial cell, an endothelial cell, an epididymal cell, a kidney cell, a liver cell, an adipocyte, a lipocyte, or a neuron cell. In some cases, the cell can be associated with a cancer, tumor, or neoplasm. In some cases, the cell can be associated with a fetus. In some cases, the cell can be a Jurkat cell.

### Multiplexing

The present disclosures provides methods and systems for multiplexing, and otherwise increasing throughput in, analysis. For example, a single or integrated process workflow can permit the processing, identification, and/or analysis of more or multiple analytes, more or multiple types of analytes, and/or more or multiple types of analyte characterizations. For example, in the methods and systems described herein, one or more labelling agents capable of binding to or otherwise coupling to one or more cell features can be used to characterize biological particles and/or cell features. In some instances, cell features include cell surface features. Cell surface features can include, but are not limited to, a receptor, an antigen, a surface protein, a transmembrane protein, a cluster of differentiation protein, a protein channel, a protein pump, a carrier protein, a phospholipid, a glycoprotein, a glycolipid, a cell-cell interaction protein complex, an antigen-presenting complex, a major histocompatibility complex, an engineered T-cell receptor, a T-cell receptor, a B-cell receptor, a chimeric antigen receptor, a gap junction, an adherens junction, or any combination thereof. In some instances, cell features can include intracellular analytes, such as proteins, protein modifications (e.g., phosphorylation status or other post-translational modifications), nuclear proteins, nuclear membrane proteins, or any combination thereof. A labelling agent can include, but is not limited to, a protein, a peptide, an antibody (or an epitope binding fragment thereof), a lipophilic moiety (such as cholesterol), a cell surface receptor binding molecule, a receptor ligand, a small molecule, a bi-specific antibody, a bi-specific T-cell engager, a T-cell receptor engager, a B-cell receptor engager, a pro-body, an aptamer, a monobody, an affimer, a darpin, and a protein scaffold, or any combination thereof. The labelling agents can include *(e.g.,* are attached to) a reporter oligonucleotide that is indicative of the cell surface feature to which the binding group binds. For example, the reporter oligonucleotide can comprise a barcode sequence that permits identification of the labelling agent. For example, a labelling agent that is specific to one type of cell feature *(e.g.,* a first cell surface feature) can have a first reporter oligonucleotide coupled thereto, while a labelling agent that is specific to a different cell feature *(e.g.,* a second cell surface feature) can have a different reporter oligonucleotide coupled thereto. For a description of exemplary labelling agents, reporter oligonucleotides, and methods of use, see, *e.g.,* U.S. Pat. 10,550,429; U.S. Pat. Pub. 20190177800; and U.S. Pat. Pub. 20190367969, each of which is herein entirely incorporated by reference for all purposes.

In a particular example, a library of potential cell feature labelling agents can be provided, where the respective cell feature labelling agents are associated with nucleic acid reporter molecules, such that a different reporter oligonucleotide sequence is associated with each labelling agent capable of binding to a specific cell feature. In some aspects, different members of the library can be characterized by the presence of a different oligonucleotide sequence label. For example, an antibody capable of binding to a first protein can have associated with it a first reporter oligonucleotide sequence, while an antibody capable of binding to a second protein can have a different reporter oligonucleotide sequence associated with it. The presence of the particular oligonucleotide sequence can be indicative of the presence of a particular antibody or cell feature which can be recognized or bound by the particular antibody.

Labelling agents capable of binding to or otherwise coupling to one or more biological particles can be used to characterize an biological particle as belonging to a particular set of biological particles. For example, labeling agents can be used to label a sample of cells or a group of cells. In this way, a group of cells can be labeled as different from another group of cells. In an example, a first group of cells can originate from a first sample and a second group of cells can originate from a second sample. Labelling agents can allow the first group and second group to have a different labeling agent (or reporter oligonucleotide associated with the labeling agent). This can, for example, facilitate multiplexing, where cells of the first group and cells of the second group can be labeled separately and then pooled together for downstream analysis. The downstream detection of a label can indicate analytes as belonging to a particular group.

For example, a reporter oligonucleotide can be linked to an antibody or an epitope binding fragment thereof, and labeling a biological particle can comprise subjecting the antibody-linked barcode molecule or the epitope binding fragment-linked barcode molecule to conditions suitable for binding the antibody to a molecule present on a surface of the biological particle. The binding affinity between the antibody or the epitope binding fragment thereof and the molecule present on the surface can be within a desired range to ensure that the antibody or the epitope binding fragment thereof remains bound to the molecule. For example, the binding affinity can be within a desired range to ensure that the antibody or the epitope binding fragment thereof remains bound to the molecule during various sample processing steps, such as partitioning and/or nucleic acid amplification or extension. A dissociation constant (Kd) between the antibody or an epitope binding fragment thereof and the molecule to which it binds can be less than about 100 µM, 90 µM, 80 µM, 70 µM, 60 µM, 50 µM, 40 µM, 30 µM, 20 µM, 10 µM, 9 µM, 8 µM, 7 µM, 6 µM, 5 µM, 4 µM, 3 µM, 2 µM, 1 µM, 900 nM, 800 nM, 700 nM, 600 nM, 500 nM, 400 nM, 300 nM, 200 nM, 100 nM, 90 nM, 80 nM, 70 nM, 60 nM, 50 nM, 40 nM, 30 nM, 20 nM, 10 nM, 9 nM, 8 nM, 7 nM, 6 nM, 5 nM, 4 nM, 3 nM, 2 nM, 1 nM, 900 pM, 800 pM, 700 pM, 600 pM, 500 pM, 400 pM, 300 pM, 200 pM, 100 pM, 90 pM, 80 pM, 70 pM, 60 pM, 50 pM, 40 pM, 30 pM, 20 pM, 10 pM, 9 pM, 8 pM, 7 pM, 6 pM, 5 pM, 4 pM, 3 pM, 2 pM, or 1 pM. For example, the dissociation constant can be less than about 10 µM.

In another example, a reporter oligonucleotide can be coupled to a cell-penetrating peptide (CPP), and labeling cells can comprise delivering the CPP coupled reporter oligonucleotide into a biological particle. Labeling biological particles can comprise delivering the CPP conjugated oligonucleotide into a cell and/or cell bead by the cell-penetrating peptide. A cell-penetrating peptide that can be used in the methods provided herein can comprise at least one non-functional cysteine residue, which can be either free or derivatized to form a disulfide link with an oligonucleotide that has been modified for such linkage. Non-limiting examples of cell-penetrating peptides that can be used in embodiments herein include penetratin, transportan, plsl, TAT(48-60), pVEC, MTS, and MAP. Cell-penetrating peptides useful in the methods provided herein can have the capability of inducing cell penetration for at least about 30%, 40%, 50%, 60%, 70%, 80%, 90%, 95%, 96%, 97%, 98%, 99%, or 100% of cells of a cell population. The cell-penetrating peptide can be an arginine-rich peptide transporter. The cell-penetrating peptide can be Penetratin or the Tat peptide.

In another example, a reporter oligonucleotide can be coupled to a fluorophore or dye, and labeling cells can comprise subjecting the fluorophore-linked barcode molecule to conditions suitable for binding the fluorophore to the surface of the biological particle. In some instances, fluorophores can interact strongly with lipid bilayers and labeling biological particles can comprise subj ecting the fluorophore-linked barcode molecule to conditions such that the fluorophore binds to or is inserted into a membrane of the biological particle. In some cases, the fluorophore is a watersoluble, organic fluorophore. In some instances, the fluorophore is Alexa 532 maleimide, tetramethylrhodamine-5-maleimide (TMR maleimide), BODIPY-TMR maleimide, Sulfo-Cy3 maleimide, Alexa 546 carboxylic acid/succinimidyl ester, Atto 550 maleimide, Cy3 carboxylic acid/succinimidyl ester, Cy3B carboxylic acid/succinimidyl ester, Atto 565 biotin, Sulforhodamine B, Alexa 594 maleimide, Texas Red maleimide, Alexa 633 maleimide, Abberior STAR 635P azide, Atto 647N maleimide, Atto 647 SE, or Sulfo-Cy5 maleimide. See, e.g., Hughes L D, et al. PLoS One. 2014 Feb. 4; 9(2):e87649, which is hereby incorporated by reference in its entirety for all purposes, for a description of organic fluorophores.

A reporter oligonucleotide can be coupled to a lipophilic molecule, and labeling biological particles can comprise delivering the nucleic acid barcode molecule to a membrane of the biological particle or a nuclear membrane by the lipophilic molecule. Lipophilic molecules can associate with and/or insert into lipid membranes such as cell membranes and nuclear membranes. In some cases, the insertion can be reversible. In some cases, the association between the lipophilic molecule and biological particle can be such that the biological particle retains the lipophilic molecule *(e.g.,* and associated components, such as nucleic acid barcode molecules, thereof) during subsequent processing (e.g., partitioning, cell permeabilization, amplification, pooling, etc.). The reporter nucleotide can enter into the intracellular space and/or a cell nucleus.

A reporter oligonucleotide can be part of a nucleic acid molecule comprising any number of functional sequences, as described elsewhere herein, such as a target capture sequence, a random primer sequence, and the like, and coupled to another nucleic acid molecule that is, or is derived from, the analyte.

Prior to partitioning, the cells can be incubated with the library of labelling agents, that can be labelling agents to a broad panel of different cell features, *e.g.,* receptors, proteins, etc., and which include their associated reporter oligonucleotides. Unbound labelling agents can be washed from the cells, and the cells can then be co-partitioned *(e.g.,* into droplets or wells) along with partition-specific barcode oligonucleotides *(e.g.,* attached to a support, such as a bead or gel bead) as described elsewhere herein. As a result, the partitions can include the cell or cells, as well as the bound labelling agents and their known, associated reporter oligonucleotides.

In other instances, *e.g.,* to facilitate sample multiplexing, a labelling agent that is specific to a particular cell feature can have a first plurality of the labelling agent *(e.g.,* an antibody or lipophilic moiety) coupled to a first reporter oligonucleotide and a second plurality of the labelling agent coupled to a second reporter oligonucleotide. For example, the first plurality of the labeling agent and second plurality of the labeling agent can interact with different cells, cell populations or samples, allowing a particular report oligonucleotide to indicate a particular cell population (or cell or sample) and cell feature. In this way, different samples or groups can be independently processed and subsequently combined together for pooled analysis *(e.g.,* partition-based barcoding as described elsewhere herein). See, *e.g.,* U.S. Pat. Pub. 20190323088, which is hereby entirely incorporated by reference for all purposes.

As described elsewhere herein, libraries of labelling agents can be associated with a particular cell feature as well as be used to identify analytes as originating from a particular biological particle, population, or sample. The biological particles can be incubated with a plurality of libraries and a given biological particle can comprise multiple labelling agents. For example, a cell can comprise coupled thereto a lipophilic labeling agent and an antibody. The lipophilic labeling agent can indicate that the cell is a member of a particular cell sample, whereas the antibody can indicate that the cell comprises a particular analyte. In this manner, the reporter oligonucleotides and labelling agents can allow multi-analyte, multiplexed analyses to be performed.

In some instances, these reporter oligonucleotides can comprise nucleic acid barcode sequences that permit identification of the labelling agent which the reporter oligonucleotide is coupled to. The use of oligonucleotides as the reporter can provide advantages of being able to generate significant diversity in terms of sequence, while also being readily attachable to most biomolecules, *e.g.,* antibodies, etc., as well as being readily detected, *e.g.,* using sequencing or array technologies.

Attachment (coupling) of the reporter oligonucleotides to the labelling agents can be achieved through any of a variety of direct or indirect, covalent or non-covalent associations or attachments. For example, oligonucleotides can be covalently attached to a portion of a labelling agent (such a protein, *e.g.,* an antibody or antibody fragment), *e.g.,* via a linker, using chemical conjugation techniques (*e*.*g*., Lightning-Link^{®} antibody labelling kits available from Innova Biosciences), as well as other non-covalent attachment mechanisms, *e.g.,* using biotinylated antibodies and oligonucleotides (or beads that include one or more biotinylated linker, coupled to oligonucleotides) with an avidin or streptavidin linker. Antibody and oligonucleotide biotinylation techniques are available. See, *e.g.,* Fang, et al., "Fluoride-Cleavable Biotinylation Phosphoramidite for 5'-end-Labelling and Affinity Purification of Synthetic Oligonucleotides," Nucleic Acids Res. Jan. 15, 2003; 31(2):708-715, which is entirely incorporated herein by reference for all purposes. Likewise, protein and peptide biotinylation techniques have been developed and are readily available. See, *e.g.,* U.S. Pat. No. 6,265,552, which is entirely incorporated herein by reference for all purposes. Furthermore, click reaction chemistry such as a Methyltetrazine-PEG5-NHS Ester reaction, a TCO-PEG4-NHS Ester reaction, or the like, can be used to couple reporter oligonucleotides to labelling agents. Commercially available kits, such as those from Thunderlink and Abcam, and techniques common in the art can be used to couple reporter oligonucleotides to labelling agents as appropriate. In another example, a labelling agent is indirectly *(e.g.,* via hybridization) coupled to a reporter oligonucleotide comprising a barcode sequence that identifies the label agent. For instance, the labelling agent can be directly coupled *(e.g.,* covalently bound) to a hybridization oligonucleotide that comprises a sequence that hybridizes with a sequence of the reporter oligonucleotide. Hybridization of the hybridization oligonucleotide to the reporter oligonucleotide couples the labelling agent to the reporter oligonucleotide. In some embodiments, the reporter oligonucleotides are releasable from the labelling agent, such as upon application of a stimulus. For example, the reporter oligonucleotide can be attached to the labeling agent through a labile bond *(e.g.,* chemically labile, photolabile, thermally labile, etc.) as generally described for releasing molecules from supports elsewhere herein. In some instances, the reporter oligonucleotides described herein can include one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, a sequencing primer or primer biding sequence (such as an R1, R2, or partial R1 or R2 sequence).

In some cases, the labelling agent can comprise a reporter oligonucleotide and a label. A label can be fluorophore, a radioisotope, a molecule capable of a colorimetric reaction, a magnetic particle, or any other suitable molecule or compound capable of detection. The label can be conjugated to a labelling agent (or reporter oligonucleotide) either directly or indirectly *(e.g.,* the label can be conjugated to a molecule that can bind to the labelling agent or reporter oligonucleotide). In some cases, a label is conjugated to an oligonucleotide that is complementary to a sequence of the reporter oligonucleotide, and the oligonucleotide can be allowed to hybridize to the reporter oligonucleotide.

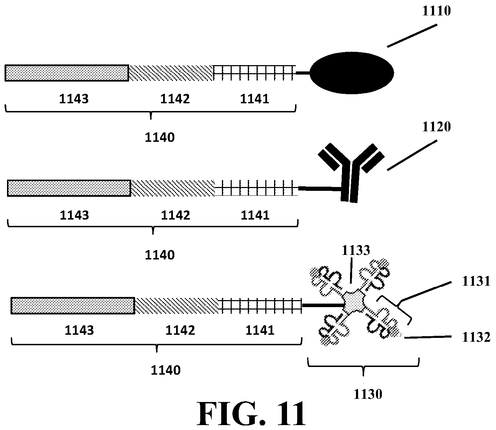

**FIG. 11** describes exemplary labelling agents **(1110, 1120, 1130)** comprising reporter oligonucleotides **(1140)** attached thereto. Labelling agent **1110** *(e.g.,* any of the labelling agents described herein) is attached (either directly, *e.g.,* covalently attached, or indirectly) to reporter oligonucleotide **1140.** Reporter oligonucleotide **1140** can comprise barcode sequence **1142** that identifies labelling agent **1110.** Reporter oligonucleotide **1140** can also comprise one or more functional sequences that can be used in subsequent processing, such as an adapter sequence, a unique molecular identifier (UMI) sequence, a sequencer specific flow cell attachment sequence (such as an P5, P7, or partial P5 or P7 sequence), a primer or primer binding sequence, or a sequencing primer or primer biding sequence (such as an R1, R2, or partial R1 or R2 sequence).

Referring to **FIG. 11****,** in some instances, reporter oligonucleotide **1140** conjugated to a labelling agent *(e.g.,* **1110, 1120, 1130)** comprises a functional sequence **1141** *(e.g.,* a primer sequence), a barcode sequence that identifies the labelling agent *(e.g.,* **1110, 1120, 1130),** and functional sequence **1143.** Functional sequence **1143** can be a reporter capture handle sequence configured to hybridize to a complementary sequence, such as a complementary sequence present on a nucleic acid barcode molecule **1190** (not shown), such as those described elsewhere herein. In some instances, nucleic acid barcode molecule **1190** is attached to a support *(e.g.,* a bead, such as a gel bead), such as those described elsewhere herein. For example, nucleic acid barcode molecule **1190** can be attached to the support via a releasable linkage *(e.g.,* comprising a labile bond), such as those described elsewhere herein. In some instances, reporter oligonucleotide **1140** comprises one or more additional functional sequences, such as those described above. In some instances, the labelling agent **1110** is a protein or polypeptide (*e.g*., an antigen or prospective antigen) comprising reporter oligonucleotide **1140.** Reporter oligonucleotide **1140** comprises barcode sequence **1142** that identifies polypeptide **1110** and can be used to infer the presence of an analyte, *e.g.,* a binding partner of polypeptide **1110** *(i.e.,* a molecule or compound to which polypeptide **1110** can bind). In some instances, the labelling agent **1110** is a lipophilic moiety (*e.g.*, cholesterol) comprising reporter oligonucleotide **1140,** where the lipophilic moiety is selected such that labelling agent **1110** integrates into a membrane of a cell or nucleus. Reporter oligonucleotide **1140** comprises barcode sequence **1142** that identifies lipophilic moiety **1110** which in some instances is used to tag cells *(e.g.,* groups of cells, cell samples, etc.) and can be used for multiplex analyses as described elsewhere herein. In some instances, the labelling agent is an antibody **1120** (or an epitope binding fragment thereof) comprising reporter oligonucleotide **1140.** Reporter oligonucleotide **1140** comprises barcode sequence **1142** that identifies antibody **1120** and can be used to infer the presence of, *e.g.,* a target of antibody **1120** *(i.e.,* a molecule or compound to which antibody **1120** binds). In other embodiments, labelling agent **1130** comprises an MHC molecule **1131** comprising peptide **1132** and reporter oligonucleotide **1140** that identifies peptide **1132.** In some instances, the MHC molecule is coupled to a support **1133.** In some instances, support **1133** can be or comprise a polypeptide, such as streptavidin, avidin, neutravidin, or a polysaccharide, such as dextran. In some embodiments, support **1133** further comprises a detectable label, e.g., a detectable label described herein, e.g., a fluorescent label. In some instances, reporter oligonucleotide **1140** can be directly or indirectly coupled to MHC labelling agent **1130** in any suitable manner. For example, reporter oligonucleotide **1140** can be coupled to MHC molecule **1131,** support **1133,** or peptide **1132.** In some embodiments, labelling agent **1130** comprises a plurality of MHC molecules described herein, *(e.g.* an MHC multimer, which can be coupled to a support *(e.g.,* **1133)).** In some embodiments, reporter oligonucleotide **1140** and MHC molecule **1130** can be attached to the polypeptide or polysaccharide of support **1133.** In some embodiments, reporter oligonucleotide **1140** and MHC molecule **1130** can be attached to the detectable label of support **1133.** In some embodiments, reporter oligonucleotide **1140** and an antigen *(e.g.,* protein, polypeptide) can be attached to polypeptide or polysaccharide of support **1133.** In some embodiments, reporter oligonucleotide **1140** and an antigen *(e.g.,* protein, polypeptide) can be attached to the detectable label of support **1133.** There are many possible configurations of Class I and/or Class II MHC multimers that can be utilized with the compositions, methods, and systems disclosed herein, *e.g.,* MHC tetramers, MHC pentamers (MHC assembled via a coiled-coil domain, *e.g.,* Pro5^{®} MHC Class I Pentamers, (ProImmune, Ltd.), MHC octamers, MHC dodecamers, MHC decorated dextran molecules *(e.g.,* MHC Dextramer^{®} (Immudex)), etc. For a description of exemplary labelling agents, including antibody and MHC-based labelling agents, reporter oligonucleotides, and methods of use, see, *e.g.,* U.S. Pat. 10,550,429 and U.S. Pat. Pub. 20190367969, each of which is herein entirely incorporated by reference for all purposes.

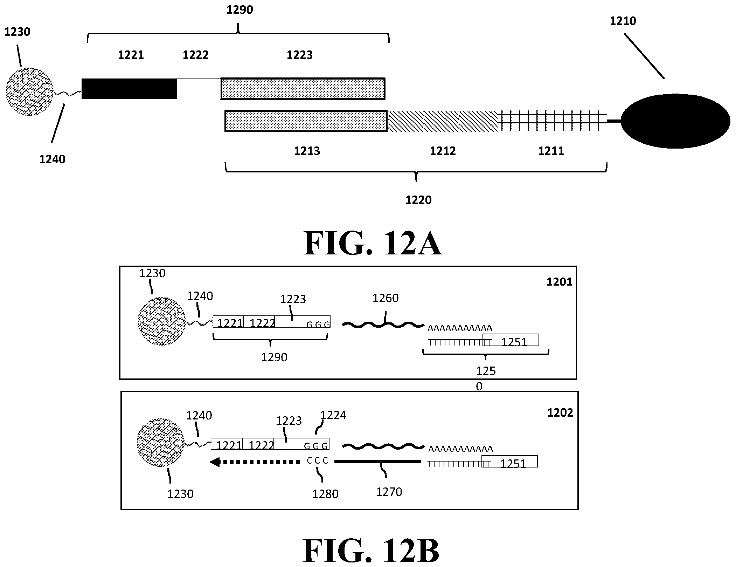

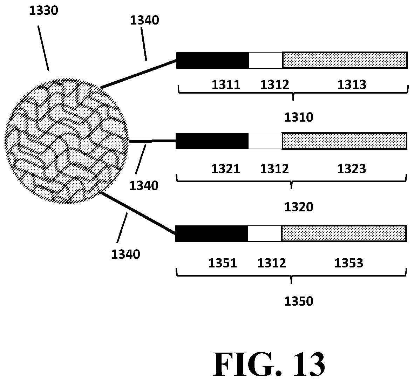

**FIG. 13** illustrates another non-limiting example of a barcode carrying bead. In some embodiments, analysis of multiple analytes *(e.g.,* RNA and one or more analytes using labelling agents described herein) can comprise nucleic acid barcode molecules as generally depicted in **FIG. 13****.** In some embodiments, nucleic acid barcode molecules **1310** and **1320** are attached to support **1330** via a releasable linkage **1340** (*e.g.*, comprising a labile bond) as described elsewhere herein. Nucleic acid barcode molecule **1310** can comprise adapter sequence **1311,** barcode sequence **1312** and capture sequence **1313.** Nucleic acid barcode molecule **1320** can comprise adapter sequence **1321,** barcode sequence **1312,** and capture sequence **1323,** wherein capture sequence **1323** comprises a different sequence than capture sequence **1313.** In some instances, adapter **1311** and adapter **1321** comprise the same sequence. In some instances, adapter **1311** and adapter **1321** comprise different sequences. Although support **1330** is shown comprising nucleic acid barcode molecules **1310** and **1320,** any suitable number of barcode molecules comprising common barcode sequence **1312** are contemplated herein. For example, in some embodiments, support **1330** further comprises nucleic acid barcode molecule **1350.** Nucleic acid barcode molecule **1350** can comprise adapter sequence **1351,** barcode sequence **1312** and capture sequence **1353,** wherein capture sequence **1353** comprises a different sequence than capture sequence **1313** and **1323.** In some instances, nucleic acid barcode molecules (*e.g*., **1310, 1320, 1350)** comprise one or more additional functional sequences, such as a UMI or other sequences described herein. The nucleic acid barcode molecules **1310, 1320** or **1350 can** interact with analytes as described elsewhere herein, for example, as depicted in **FIGS. 12A-C**.

Referring to **FIG. 12A****,** in an instance where cells are labelled with labeling agents, capture sequence **1223** can be complementary to an adapter sequence of a reporter oligonucleotide. Cells can be contacted with one or more reporter oligonucleotide **1220** conjugated labelling agents **1210** *(e.g.,* polypeptide, antibody, or others described elsewhere herein). In some cases, the cells can be further processed prior to barcoding. For example, such processing steps can include one or more washing and/or cell sorting steps. In some instances, a cell that is bound to labelling agent **1210** which is conjugated to oligonucleotide **1220** and support **1230** *(e.g.,* a bead, such as a gel bead) comprising nucleic acid barcode molecule **1290** is partitioned into a partition amongst a plurality of partitions *(e.g.,* a droplet of a droplet emulsion or a well of a microwell array). In some instances, the partition comprises at most a single cell bound to labelling agent **1210.** In some instances, reporter oligonucleotide **1220** conjugated to labelling agent **1210** *(e.g.,* polypeptide, an antibody, pMHC molecule such as an MHC multimer, etc.) comprises a first adapter sequence **1211** *(e.g.,* a primer sequence), a barcode sequence **1212** that identifies the labelling agent **1210** *(e.g.,* the polypeptide, antibody, or peptide of a pMHC molecule or complex), and an capture handle sequence **1213.** Capture handle sequence **1213** can be configured to hybridize to a complementary sequence, such as a capture sequence **1223** present on a nucleic acid barcode molecule **1290.** In some instances, oligonucleotide **1220** comprises one or more additional functional sequences, such as those described elsewhere herein.

Barcoded nucleic can be generated *(e.g.,* via a nucleic acid reaction, such as nucleic acid extension or ligation) from the constructs described in **FIGS. 12A-12C****.** For example, capture handle sequence **1213** can then be hybridized to complementary sequence, such as capture sequence **1223** to generate *(e.g.,* via a nucleic acid reaction, such as nucleic acid extension or ligation) a barcoded nucleic acid molecule comprising cell *(e.g.,* partition specific) barcode sequence **1222** (or a reverse complement thereof) and reporter barcode sequence **1212** (or a reverse complement thereof). In some embodiments, the nucleic acid barcode molecule **1290** *(e.g.,* partition-specific barcode molecule) further includes a UMI (not shown). Barcoded nucleic acid molecules can then be optionally processed as described elsewhere herein, *e.g.,* to amplify the molecules and/or append sequencing platform specific sequences to the fragments. See, *e.g.,* U.S. Pat. Pub. 2018/0105808, which is hereby entirely incorporated by reference for all purposes. Barcoded nucleic acid molecules, or derivatives generated therefrom, can then be sequenced on a suitable sequencing platform.

In some instances, analysis of multiple analytes *(e.g.,* nucleic acids and one or more analytes using labelling agents described herein) can be performed. For example, the workflow can comprise a workflow as generally depicted in any of **FIGS. 12A-12C****,** or a combination of workflows for an individual analyte, as described elsewhere herein. For example, by using a combination of the workflows as generally depicted in **FIGS. 12A-12C****,** multiple analytes can be analyzed.

In some instances, analysis of an analyte *(e.g.* a nucleic acid, a polypeptide, a carbohydrate, a lipid, etc.) comprises a workflow as generally depicted in **FIG. 12A****.** A nucleic acid barcode molecule **1290** can be co-partitioned with the one or more analytes. In some instances, nucleic acid barcode molecule **1290** is attached to a support **1230** *(e.g.,* a bead, such as a gel bead), such as those described elsewhere herein. For example, nucleic acid barcode molecule **1290** can be attached to support **1230** via a releasable linkage **1240** *(e.g.,* comprising a labile bond), such as those described elsewhere herein. Nucleic acid barcode molecule **1290** can comprise a functional sequence **1221** and optionally comprise other additional sequences, for example, a barcode sequence **1222** (e.g., common barcode, partition-specific barcode, or other functional sequences described elsewhere herein), and/or a UMI sequence (not shown). The nucleic acid barcode molecule **1290** can comprise a capture sequence **1223** that can be complementary to another nucleic acid sequence, such that it can hybridize to a particular sequence, e.g., capture handle sequence **1213.**

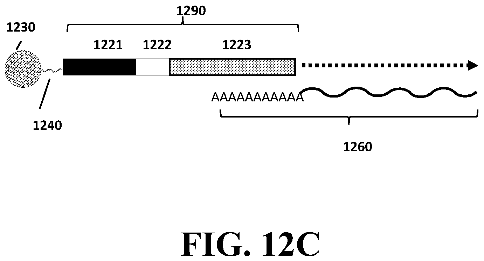

For example, capture sequence **1223** can comprise a poly-T sequence and can be used to hybridize to mRNA. Referring to **FIG. 12C****,** in some embodiments, nucleic acid barcode molecule **1290** comprises capture sequence **1223** complementary to a sequence of RNA molecule **1260** from a cell. In some instances, capture sequence **1223** comprises a sequence specific for an RNA molecule. Capture sequence **1223** can comprise a known or targeted sequence or a random sequence. In some instances, a nucleic acid extension reaction can be performed, thereby generating a barcoded nucleic acid product comprising capture sequence **1223,** the functional sequence **1221,** barcode sequence **1222,** any other functional sequence, and a sequence corresponding to the RNA molecule **1260.**

In another example, capture sequence **1223** can be complementary to an overhang sequence or an adapter sequence that has been appended to an analyte. For example, referring to **FIG. 12B****,** panel **1201,** in some embodiments, primer **1250** comprises a sequence complementary to a sequence of nucleic acid molecule **1260** (such as an RNA encoding for a BCR sequence) from a biological particle. In some instances, primer **1250** comprises one or more sequences **1251** that are not complementary to RNA molecule **1260.** Sequence **1251** can be a functional sequence as described elsewhere herein, for example, an adapter sequence, a sequencing primer sequence, or a sequence the facilitates coupling to a flow cell of a sequencer. In some instances, primer **1250** comprises a poly-T sequence. In some instances, primer **1250** comprises a sequence complementary to a target sequence in an RNA molecule. In some instances, primer **1250** comprises a sequence complementary to a region of an immune molecule, such as the constant region of a TCR or BCR sequence. Primer **1250** is hybridized to nucleic acid molecule **1260** and complementary molecule **1270** is generated (see Panel **1202).** For example, complementary molecule **1270** can be cDNA generated in a reverse transcription reaction. In some instances, an additional sequence can be appended to complementary molecule 1270. For example, the reverse transcriptase enzyme can be selected such that several non-templated bases **1280** (*e.g*., a poly-C sequence) are appended to the cDNA. In another example, a terminal transferase can also be used to append the additional sequence. Nucleic acid barcode molecule **1290** comprises a sequence **1224** complementary to the non-templated bases, and the reverse transcriptase performs a template switching reaction onto nucleic acid barcode molecule **1290** to generate a barcoded nucleic acid molecule comprising cell (*e.g*., partition specific) barcode sequence **1222** (or a reverse complement thereof) and a sequence of complementary molecule **1270** (or a portion thereof). In some instances, sequence **1223** comprises a sequence complementary to a region of an immune molecule, such as the constant region of a TCR or BCR sequence. Sequence **1223** is hybridized to nucleic acid molecule **1260** and a complementary molecule **1270** is generated. For example complementary molecule 1270 can be generated in a reverse transcription reaction generating a barcoded nucleic acid molecule comprising cell (*e.g*., partition specific) barcode sequence **1222** (or a reverse complement thereof) and a sequence of complementary molecule **1270** (or a portion thereof). Additional methods and compositions suitable for barcoding cDNA generated from mRNA transcripts including those encoding V(D)J regions of an immune cell receptor and/or barcoding methods and composition including a template switch oligonucleotide are described in International Patent Application WO2018/075693, U.S. Patent Publication No. 2018/0105808, U.S. Patent Publication No. 2015/0376609, filed June 26, 2015, and U.S. Patent Publication No. 2019/0367969, each of which applications is herein entirely incorporated by reference for all purposes.

### Computer systems

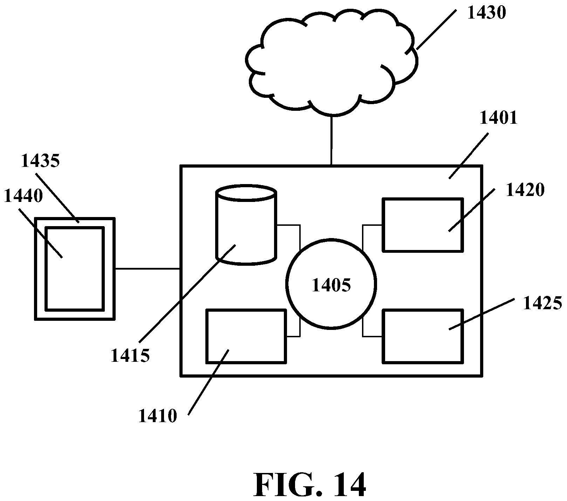

The present disclosure provides computer systems that are programmed to implement methods of the disclosure. **FIG. 14** shows a computer system **1401** that is programmed or otherwise configured to regulate various aspects of the present disclosure. The computer system **1401** can be an electronic device of a user or a computer system that is remotely located with respect to the electronic device. The electronic device can be a mobile electronic device.

The computer system **1401** includes a central processing unit (CPU, also "processor" and "computer processor" herein) **1405,** which can be a single core or multi core processor, or a plurality of processors for parallel processing. The computer system **1401** also includes memory or memory location **1410** (*e.g*., random-access memory, read-only memory, flash memory), electronic storage unit **1415** *(e.g.,* hard disk), communication interface **1420** (*e.g.*, network adapter) for communicating with one or more other systems, and peripheral devices **1425,** such as cache, other memory, data storage and/or electronic display adapters. The memory **1410,** storage unit **1415,** interface **1420** and peripheral devices **1425** are in communication with the CPU **1405** through a communication bus (solid lines), such as a motherboard. The storage unit **1415** can be a data storage unit (or data repository) for storing data. The computer system **1401** can be operatively coupled to a computer network ("network") **1430** with the aid of the communication interface **1420.** The network **1430** can be the Internet, an internet and/or extranet, or an intranet and/or extranet that is in communication with the Internet. The network **1430** in some cases is a telecommunication and/or data network. The network **1430** can include one or more computer servers, which can enable distributed computing, such as cloud computing. The network **1430,** in some cases with the aid of the computer system **1401,** can implement a peer-to-peer network, which can enable devices coupled to the computer system **1401** to behave as a client or a server.

The CPU **1405** can execute a sequence of machine-readable instructions, which can be embodied in a program or software. The instructions can be stored in a memory location, such as the memory **1410.** The instructions can be directed to the CPU **1405,** which can subsequently program or otherwise configure the CPU **1405** to implement methods of the present disclosure. Examples of operations performed by the CPU **1405** can include fetch, decode, execute, and writeback.

The CPU **1405** can be part of a circuit, such as an integrated circuit. One or more other components of the system **1401** can be included in the circuit. In some cases, the circuit is an application specific integrated circuit (ASIC).

The storage unit **1415** can store files, such as drivers, libraries and saved programs. The storage unit **1415** can store user data, *e.g.*, user preferences and user programs. The computer system **1401** in some cases can include one or more additional data storage units that are external to the computer system **1401,** such as located on a remote server that is in communication with the computer system **1401** through an intranet or the Internet.

The computer system **1401** can communicate with one or more remote computer systems through the network **1430.** For instance, the computer system **1401** can communicate with a remote computer system of a user (*e.g.*, operator). Examples of remote computer systems include personal computers (*e.g.*, portable PC), slate or tablet PC's (*e.g.*, Applet iPad, Samsung^{®} Galaxy Tab), telephones, Smart phones (*e.g.*, Apple^{®} iPhone, Android-enabled device, Blackberry^{®}), or personal digital assistants. The user can access the computer system **1401** via the network **1430.**

Methods as described herein can be implemented by way of machine (*e.g.*, computer processor) executable code stored on an electronic storage location of the computer system **1401,** such as, for example, on the memory **1410** or electronic storage unit **1415.** The machine executable or machine readable code can be provided in the form of software. During use, the code can be executed by the processor **1405.** In some cases, the code can be retrieved from the storage unit **1415** and stored on the memory **1410** for ready access by the processor **1405.** In some situations, the electronic storage unit **1415** can be precluded, and machine-executable instructions are stored on memory **1410.**

The code can be pre-compiled and configured for use with a machine having a processor adapted to execute the code, or can be compiled during runtime. The code can be supplied in a programming language that can be selected to enable the code to execute in a pre-compiled or as-compiled fashion.

Aspects of the systems and methods provided herein, such as the computer system **1401**, can be embodied in programming. Various aspects of the technology can be thought of as "products" or "articles of manufacture" typically in the form of machine (or processor) executable code and/or associated data that is carried on or embodied in a type of machine readable medium. Machine-executable code can be stored on an electronic storage unit, such as memory (*e.g.*, read-only memory, random-access memory, flash memory) or a hard disk. "Storage" type media can include any or all of the tangible memory of the computers, processors or the like, or associated modules thereof, such as various semiconductor memories, tape drives, disk drives and the like, which can provide non-transitory storage at any time for the software programming. All or portions of the software can at times be communicated through the Internet or various other telecommunication networks. Such communications, for example, can enable loading of the software from one computer or processor into another, for example, from a management server or host computer into the computer platform of an application server. Thus, another type of media that can bear the software elements includes optical, electrical and electromagnetic waves, such as used across physical interfaces between local devices, through wired and optical landline networks and over various air-links. The physical elements that carry such waves, such as wired or wireless links, optical links or the like, also can be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, can take many forms, including but not limited to, a tangible storage medium, a carrier wave medium or physical transmission medium. Non-volatile storage media include, for example, optical or magnetic disks, such as any of the storage devices in any computer(s) or the like, such as can be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media can take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer can read programming code and/or data. Many of these forms of computer readable media can be involved in carrying one or more sequences of one or more instructions to a processor for execution.

The computer system **1401** can include or be in communication with an electronic display **1435** that comprises a user interface (UI) **1440.** Examples of UIs include, without limitation, a graphical user interface (GUI) and web-based user interface.

Methods and systems of the present disclosure can be implemented by way of one or more algorithms. An algorithm can be implemented by way of software upon execution by the central processing unit **1405.**

Devices, systems, compositions and methods of the present disclosure can be used for various applications, such as, for example, processing a single analyte (*e.g.*, RNA, DNA, or protein) or multiple analytes (*e.g.*, DNA and RNA, DNA and protein, RNA and protein, or RNA, DNA and protein) from a single cell. For example, a biological particle (*e.g.*, a cell or cell bead) is partitioned in a partition (*e.g.*, droplet), and multiple analytes from the biological particle are processed for subsequent processing. The multiple analytes can be from the single cell. This can enable, for example, simultaneous proteomic, transcriptomic and genomic analysis of the cell.

### EXAMPLES

### Example 1: Feasibility Testing of Barcoding and Circularization using a Splint Oligonucleotide

To test the feasibility of a barcoding approach described herein, *e.g.*, barcoding, circularizing, and extension to generate extension products for sequencing, a shortened nucleic acid barcode molecule was generated, comprising a P5 primer, a P7 primer, and a capture sequence. The capture sequence was annealed to a test RNA molecule, and the nucleic acid barcode molecule extended using reverse transcription, thereby generating a barcoded molecule. The barcoded molecule was treated with RNase H and cleaned up, to generate single-stranded barcoded molecules, comprising, in a 5' to 3' direction: the P5 primer, the P7 primer, and a sequence complementary to the test RNA molecule.

The barcoded molecules were then hybridized to a splint molecule comprising (i) a first splint sequence complementary to the 5' end (P5 primer) of the barcoded molecule and (ii) a second splint sequence complementary to the 3' end (a portion of the test RNA molecule, or complement thereof) of the barcoded molecule. The barcoded molecules were hybridized to the splint molecule by heating at 95 degrees Celsius for 10 minutes, followed by a temperature ramp-down to 22 degrees Celsius at a gradient of 0.1 degrees C/s, then holding at 22 degrees Celsius for 1 hour. The product was stored at 4 degrees Celsius and subsequently ligated using an ACTV-derived ligase via incubation at 25 degrees Celsius for 2 hours. The circularized molecule was then amplified by PCR with the P5 and P7 primers.

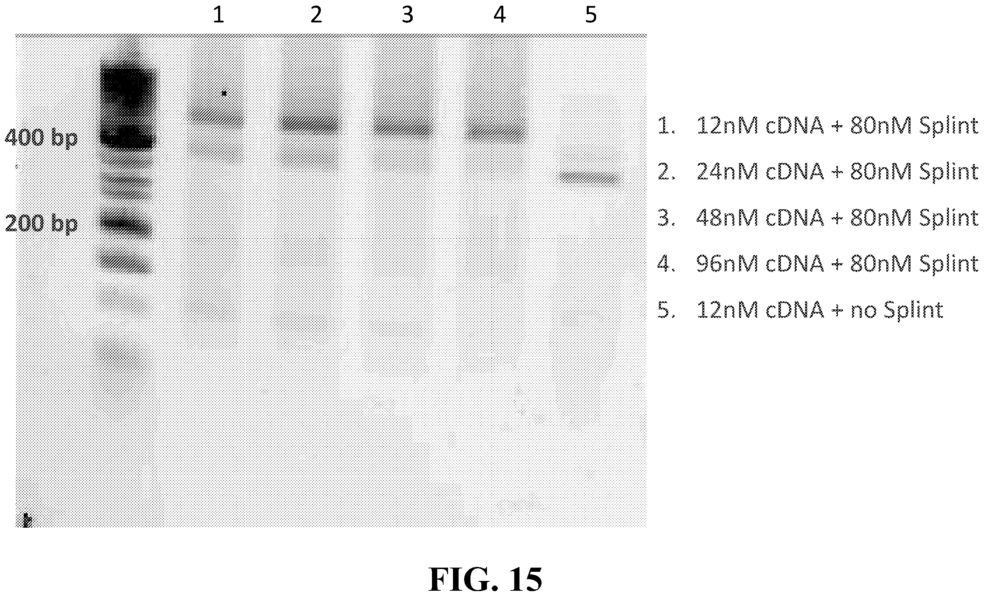

**FIG. 15** shows exemplary gel electrophoresis data of the circularized molecules that were ligated using an 80nM concentration of the splint molecule. Lanes 1-5 of the gel (excluding the DNA ladder) indicate an input cDNA concentration of 12 nM, 24 nM, 48 nM, 96 nM, and 12 nM with no splint (negative control), respectively. The expected circularized molecule was expected to have a size of ~600 base pairs. As observed in **FIG. 15****,** all of the lanes with the splint molecule exhibited a band of approximately 600 base pairs in size, and the negative control lane had a nonspecific product (less than 400 base pairs in size). These data illustrate the feasibility of splint ligation approaches for circularizing barcoded molecules.

While preferred embodiments of the present disclosure have been shown and described herein, it will be obvious to those skilled in the art that such embodiments are provided by way of example only. It is not intended that the present disclosure be limited by the specific examples provided within the specification. While the present disclosure has been described with reference to the aforementioned specification, the descriptions and illustrations of the embodiments herein are not meant to be construed in a limiting sense. Numerous variations, changes, and substitutions will now occur to those skilled in the art without departing from the present disclosure. Furthermore, it shall be understood that all aspects of the present disclosure are not limited to the specific depictions, configurations or relative proportions set forth herein which depend upon a variety of conditions and variables. It should be understood that various alternatives to the embodiments of the present disclosure described herein can be employed in practicing the present disclosure. It is therefore contemplated that the present disclosure shall also cover any such alternatives, modifications, variations or equivalents. It is intended that the following claims define the scope of the present disclosure and that methods and structures within the scope of these claims and their equivalents be covered thereby.

### NUMBERED EMBODIMENTS

1. A method, comprising:
   (a) providing (i) a first nucleic acid barcode molecule and a second nucleic acid barcode molecule, and (ii) a first nucleic acid molecule and a second nucleic acid molecule, wherein said first nucleic acid barcode molecule comprises, in a 5' to 3' direction, a first barcode sequence, a first primer sequence, and a second primer sequence, and wherein said second nucleic acid barcode molecule comprises, in a 5' to 3' direction, said second primer sequence, said first primer sequence, and a second barcode sequence;
   (b) generating (i) a first barcoded molecule using said first nucleic acid molecule and said first nucleic acid barcode molecule, wherein said first barcoded molecule comprises a first sequence complementary to at least a portion of said first nucleic acid molecule and (ii) a second barcoded molecule using said second nucleic acid molecule and said second nucleic acid barcode molecule, wherein said second barcoded molecule comprises a second sequence complementary to at least a portion of said second nucleic acid molecule;
   (c) circularizing (i) said first barcoded molecule to generate a first circularized molecule, and (ii) said second barcoded molecule to generate a second circularized molecule; and
   (d) generating (i) a first extension molecule using said first circularized molecule and a first primer molecule comprising a first primer sequence complementary to said first primer sequence, and (ii) a second extension molecule using said second circularized molecule and a second primer molecule comprising a second primer sequence complementary to said second primer sequence,
      wherein said first extension molecule comprises a sequence corresponding to said first barcode sequence 5' to said sequence corresponding to said first nucleic acid molecule, and
      wherein said second extension molecule comprises a sequence corresponding to said second barcode sequence 3' to a sequence corresponding to said second nucleic acid molecule.
2. The method of embodiment 1, wherein in (a) said first nucleic acid barcode molecule and said second nucleic acid barcode molecule are coupled to a bead.
3. The method of embodiment 1 or 2, wherein said first barcode sequence and said second barcode sequence comprise a common barcode sequence.
4. The method of embodiment 3, wherein said first nucleic acid barcode molecule comprises a first unique molecular identifier (UMI) sequence and said second nucleic acid barcode molecule comprises a second UMI sequence that is different from said first UMI sequence.
5. The method of embodiment 3 or 4, wherein said common barcode sequence is common to a bead, and wherein said bead comprises a plurality of nucleic acid barcode molecules, wherein at least a subset of said plurality of nucleic acid barcode molecules comprise said common barcode sequence.
6. The method of any one of embodiments 1-5, wherein said first nucleic acid barcode molecule comprises a first capture sequence configured to capture said first nucleic acid molecule, and wherein said second nucleic acid barcode molecule comprises a second capture sequence configured to capture said second nucleic acid molecule.
7. The method of embodiment 6, wherein said first capture sequence or said second capture sequence comprises a polyT sequence.
8. The method of embodiment 6 or 7, wherein said first capture sequence or said second capture sequence comprises a random Nmer sequence.
9. The method of any one of embodiments 6-8, wherein said first capture sequence or said second capture sequence comprises a target-specific sequence.
10. The method of any one of embodiments 6-9, wherein said first capture sequence or said second capture sequence comprises a sequence configured to couple to a reporter oligonucleotide of a feature-binding group.
11. The method of any one of embodiments 6-10, wherein (b) comprises capturing said first nucleic acid molecule using said first capture sequence and extending said first nucleic acid barcode molecule to generate said first barcoded molecule.
12. The method of embodiment 11, wherein (b) further comprises capturing said second nucleic acid molecule using said second capture sequence and extending said second nucleic acid barcode molecule to generate said second barcoded molecule.
13. The method any one of embodiments 1-12, wherein (c) is performed using a circularization enzyme.
14. The method of embodiment 13, wherein said circularization enzyme is CircLigase.
15. The method of any one of embodiments 1-12, wherein (c) is performed using a ligase.
16. The method of embodiment 15, wherein said ligase is a T4 DNA ligase, T7 DNA Ligase, SplintR, T4RNA Ligase, KOD RNA Ligase, a double stranded DNA ligase, or an Acanthocystis turfacea chlorella virus 1-isolated ligase.
17. The method of any one of embodiments 1-16, wherein (c) is performed using a splint molecule, wherein said splint molecule comprises (i) a first splint sequence complementary to a 5' end of said first barcoded molecule and (ii) a second splint sequence complementary to a 3' end of said first barcoded molecule.
18. The method of any one of embodiments 1-16, wherein (c) is performed using a splint molecule, wherein said splint molecule comprises (i) a first splint sequence complementary to a 5' end of said second barcoded molecule and (ii) a second splint sequence complementary to a 3' end of said second barcoded molecule.
19. The method of any one of the preceding embodiments, wherein said first nucleic acid barcode molecule comprises, in said 5' to 3' direction, said first barcode sequence, said first primer sequence, said second primer sequence, and a capture sequence.
20. The method of embodiment 19, wherein (d) comprises using a primer molecule to, at said second primer sequence, perform an extension reaction to generate said first extension molecule.
21. The method of embodiment 4, wherein said first nucleic acid barcode molecule comprises the first UMI sequence adjacent to said first nucleic acid barcode sequence.
22. The method of any one of embodiments 1-21, wherein said second nucleic acid barcode molecule comprises, in said 5' to 3' direction, said second primer sequence, said first primer sequence, said second barcode sequence, and a capture sequence.
23. The method of embodiment 22, wherein (d) comprises using a primer molecule to, at said first primer sequence, perform an extension reaction to generate said second extension molecule.
24. The method of embodiment 4, wherein said second nucleic acid barcode molecule comprises the second UMI sequence adjacent to said second nucleic acid barcode sequence.
25. The method of any one of embodiments 1-24, wherein (b) is performed in bulk.
26. The method of any one of embodiments 1-24, wherein (b) is performed in a partition.
27. The method of embodiment 26, further comprising, prior to (b), partitioning said first nucleic acid barcode molecule, said second nucleic acid barcode molecule, said first nucleic acid molecule, and said second nucleic acid molecule in said partition.
28. The method of embodiment 26 or 27, wherein said partition is a droplet or a well.
29. The method of any one of embodiments 26-28, wherein (c) is performed outside of said partition.
30. The method of any one of embodiments 26-28, wherein (c) is performed in said partition.
31. A method, comprising:
   (a) generating a barcoded molecule using a nucleic acid molecule and a nucleic acid barcode molecule, wherein said barcoded molecule comprises, in a 5' to 3' direction, a barcode sequence and a sequence complementary to at least a portion of said nucleic acid molecule;
   (b) circularizing said barcoded molecule to generate a circularized molecule; and
   (c) generating an extension molecule from said circularized molecule, wherein said extension molecule comprises, in a 5' to 3' direction, a first sequence corresponding to said nucleic acid molecule and a second sequence corresponding to said barcode sequence.
32. The method of embodiment 31, wherein (a) is performed in a partition.
33. The method of embodiment 32, wherein said partition is a droplet or a well.
34. The method of embodiment 32 or 33, wherein (b) is performed outside of said partition.
35. The method of embodiment 32 or 33, wherein (b) is performed in said partition.
36. The method of any one of embodiments 32-35, wherein said partition comprises a plurality of nucleic acid barcode molecules comprising said nucleic acid barcode molecule.
37. The method of embodiment 36, wherein said plurality of nucleic acid barcode molecules comprises an additional nucleic acid barcode molecule comprising an additional barcode sequence.
38. The method of embodiment 37, wherein said nucleic acid barcode molecule comprises, in a 5' to 3' direction, said barcode sequence, a first primer sequence, a second primer sequence, and said sequence complementary to at least said portion of said nucleic acid molecule.
39. The method of embodiment 37 or 38, wherein said additional nucleic acid barcode molecule comprises, in said 5' to 3' direction, said second primer sequence, said first primer sequence, said barcode sequence, and said sequence complementary to at least said portion of said nucleic acid molecule.
40. The method of any one of embodiments 37-39, wherein said barcode sequence and said additional barcode sequence are identical.
41. The method of any one of embodiments 37-40, wherein said nucleic acid barcode molecule or said additional nucleic acid barcode molecule comprises a unique molecular identifier (UMI).
42. The method of any one of embodiments 37-40, wherein both said nucleic acid barcode molecule and said additional nucleic acid barcode molecule comprise a unique molecular identifier (UMI).
43. The method of embodiment 41 or 42, wherein said UMI is disposed 5' of said barcode sequence of said nucleic acid barcode molecule or said additional barcode sequence of said additional nucleic acid barcode molecule.
44. The method of embodiment 41 or 42, wherein said UMI is disposed 3' of said barcode sequence of said nucleic acid barcode molecule or said additional barcode sequence of said additional nucleic acid barcode molecule.
45. The method of any one of embodiments 37-44, further comprising generating an additional barcoded molecule using an additional nucleic acid molecule and said additional nucleic acid barcode molecule, wherein said additional barcoded molecule comprises, in a 5' to 3' direction, said additional barcode sequence and a sequence complementary to at least a portion of said additional nucleic acid molecule.
46. The method of any one of embodiments 31-45, wherein, prior to (a), said nucleic acid barcode molecule is provided on a bead.
47. The method of embodiment 37, wherein, prior to (a), said nucleic acid barcode molecule and said additional nucleic acid barcode molecule are provided on a bead.
48. The method of any one of embodiments 31-47, wherein said nucleic acid barcode molecule comprises, at a 3' end, a capture sequence configured to hybridize to at least a portion of said nucleic acid molecule.
49. The method of embodiment 48, wherein said capture sequence comprises a polyT sequence.
50. The method of embodiment 48 or 49, wherein said capture sequence comprises a random Nmer sequence.
51. The method of any one of embodiments 48-50, wherein said capture sequence comprises a target-specific sequence.
52. The method of any one of embodiments 48-51, wherein said capture sequence comprises a sequence configured to couple to a reporter oligonucleotide of a feature-binding group.
53. The method of any one of embodiments 48-52, wherein (a) comprises capturing said nucleic acid molecule using said capture sequence, and extending said nucleic acid barcode molecule to generate said barcoded molecule.
54. The method of embodiment 53, wherein said barcoded molecule is single stranded.
55. The method of any one of embodiments 31-54, wherein (b) is performed using a single stranded circularization enzyme.
56. The method of embodiment 55, wherein said circularization enzyme is CircLigase.
57. The method of any one of embodiments 31-56, wherein (b) is performed using a ligase.
58. The method of embodiment 57, wherein said ligase is a T4 DNA ligase, T7 DNA Ligase, SplintR, Acanthocystis turfacea chlorella virus-isolated ligase, T4RNA Ligase, KOD RNA Ligase, or a double stranded DNA ligase.
59. The method embodiment 57 or 58, wherein (b) is performed using a splint molecule, wherein said splint molecule comprises (i) a first splint sequence complementary to a 5' end of said barcoded molecule and (ii) a second splint sequence complementary to a 3' end of said barcoded molecule.
60. A method, comprising:
   (a) generating a barcoded molecule using a nucleic acid molecule and a nucleic acid barcode molecule, wherein said barcoded molecule comprises a barcode sequence, and, in a 5' to 3' direction:, (i) a first primer sequence, (ii) a second primer sequence, and (iii) a sequence complementary to at least a portion of said nucleic acid molecule;
   (b) circularizing said barcoded molecule to generate a circularized molecule; and
   (c) generating an extension product from said circularized molecule, wherein said extension product comprises, said barcode sequence or complement thereof, and, in a 5' to 3' direction, a first sequence corresponding to said second primer sequence, a second sequence corresponding to said nucleic acid molecule, and a third sequence corresponding to said first primer sequence.
61. The method of embodiment 60, wherein, prior to (a), said nucleic acid barcode molecule is provided on a bead.
62. The method of embodiment 61, wherein said bead comprises a plurality of nucleic acid barcode molecules coupled thereto, including said nucleic acid barcode molecule, wherein at least a subset of said plurality of nucleic acid barcode molecules comprises a common barcode sequence.
63. The method of embodiment 62, wherein at least said subset of said plurality of nucleic acid barcode molecules further comprises a unique molecular identifier (UMI) sequence that differs among said plurality of nucleic acid barcode molecules coupled to said bead.
64. The method of any one of embodiments 60-63, wherein said nucleic acid barcode molecule comprises, at a 3' end, a capture sequence configured to hybridize to at least a portion of said nucleic acid molecule.
65. The method of embodiment 64, wherein said capture sequence comprises a polyT sequence.
66. The method of embodiment 64 or 65, wherein said capture sequence comprises a random Nmer sequence.
67. The method of any one of embodiments 64-66, wherein said capture sequence comprises a target-specific sequence.
68. The method of any one of embodiments 64-67, wherein said capture sequence comprises a sequence configured to couple to a reporter oligonucleotide of a feature-binding group.
69. The method of any one of embodiments 64-68, wherein (a) comprises capturing said nucleic acid molecule using said capture sequence, and extending said nucleic acid barcode molecule to generate said barcoded molecule.
70. The method of any one of embodiments 60-68, wherein said barcoded molecule is single stranded.
71. The method of any one of embodiments 60-70, wherein (b) is performed using a single stranded circularization enzyme.
72. The method of embodiment 71, wherein said circularization enzyme is CircLigase.
73. The method of any one of embodiments 60-72, wherein (b) is performed using a ligase.
74. The method of embodiment 73, wherein said ligase is a T4 DNA ligase, T7 DNA Ligase, SplintR, Acanthocystis turfacea chlorella virus-isolated ligase, T4RNA Ligase, KOD RNA Ligase, or a double stranded DNA ligase.
75. The method any one of embodiments 60-72, wherein (b) is performed using a splint molecule, wherein said splint molecule comprises (i) a first splint sequence complementary to a 5' end of said barcoded molecule and (ii) a second splint sequence complementary to a 3' end of said barcoded molecule.
76. The method of any one of embodiments 60-75, wherein said nucleic acid barcode molecule comprises, in said 5' to 3' direction, said barcode sequence, a unique molecular identifier (UMI) sequence, said first primer sequence, said second primer sequence, and a capture sequence.
77. The method of embodiment 76, wherein (c) comprises using a primer molecule to, at said second primer sequence, perform an extension reaction to generate said extension molecule.
78. The method of any one of embodiments 60-75, wherein said nucleic acid barcode molecule comprises, in said 5' to 3' direction, said first primer sequence, said second primer sequence, said barcode sequence, a unique molecular identifier (UMI) sequence, and a capture sequence.
79. The method of embodiment 78, wherein (c) comprises using a primer molecule to, at said second primer sequence, perform an extension reaction to generate said extension molecule.
80. The method of any one of embodiments 60-79, wherein (a) is performed in bulk.
81. The method of any one of embodiments 60-79, wherein (a) is performed in a partition.
82. The method of embodiment 81, wherein said partition is a droplet or a well.
83. The method of embodiment 81 or 82, wherein (b) is performed outside of said partition.
84. The method of embodiment 81 or 82, wherein (b) is performed in said partition.
85. A method, comprising:
   (a) generating a single-stranded barcoded nucleic acid molecule using a nucleic acid molecule and a nucleic acid barcode molecule, wherein said barcoded nucleic acid barcode molecule comprises a nucleic acid barcode sequence;
   (b) subsequent to (a), circularizing said single-stranded barcoded nucleic acid molecule by ligating a first end and a second end of said single-stranded barcoded nucleic acid molecule to generate a circularized molecule; and
   (c) generating a non-concatemeric extension molecule from said circularized molecule, wherein said non-concatemeric extension molecule comprises a first sequence corresponding to said nucleic acid molecule and a second sequence corresponding to said nucleic acid barcode sequence.
86. The method of embodiment 85, wherein, prior to (a), said nucleic acid barcode molecule is provided on a bead.
87. The method of embodiment 86, wherein said bead comprises a plurality of nucleic acid barcode molecules coupled thereto, including said nucleic acid barcode molecule, wherein at least a subset of said plurality of nucleic acid barcode molecules comprises said nucleic acid barcode sequence.
88. The method of embodiment 87, wherein at least a subset of said plurality of nucleic acid barcode molecules further comprises a unique molecular identifier (UMI) sequence that differs among said plurality of nucleic acid barcode molecules coupled to said bead.
89. The method of any one of embodiments 85-88, wherein said nucleic acid barcode molecule comprises, at a 3' end, a capture sequence configured to hybridize to at least a portion of said nucleic acid molecule.
90. The method of embodiment 89, wherein said capture sequence comprises a polyT sequence.
91. The method of embodiment 89 or 90, wherein said capture sequence comprises a random Nmer sequence.
92. The method of any one of embodiments 89-91, wherein said capture sequence comprises a target-specific sequence.
93. The method of any one of embodiments 89-92, wherein said capture sequence comprises a sequence configured to couple to a reporter oligonucleotide of a feature-binding group.
94. The method of any one of embodiments 89-93, wherein (a) comprises capturing said nucleic acid molecule using said capture sequence, and extending said nucleic acid barcode molecule to generate said single-stranded barcoded nucleic acid molecule.
95. The method of any one of embodiments 85-94, wherein (b) is performed using a single stranded circularization enzyme.
96. The method of embodiment 95, wherein said circularization enzyme is CircLigase.
97. The method of any one of embodiments 85-95, wherein (b) is performed using a ligase.
98. The method of embodiment 97, wherein said ligase is a T4 DNA ligase, T7 DNA Ligase, SplintR, Acanthocystis turfacea chlorella virus-isolated ligase, T4RNA Ligase, KOD RNA Ligase, or a double stranded DNA ligase.
99. The method of any one of embodiments 85-98, wherein (b) is performed using a splint molecule, wherein said splint molecule comprises (i) a first splint sequence complementary to a 5' end of said single-stranded barcoded molecule and (ii) a second splint sequence complementary to a 3' end of said single-stranded barcoded molecule.
100. The method of any one of embodiments 85-99, wherein said nucleic acid barcode molecule comprises, in said 5' to 3' direction, said nucleic acid barcode sequence, a first primer sequence, a second primer sequence, and a capture sequence.
101. The method of embodiment 100, wherein (c) comprises using a primer molecule to, at said second primer sequence, perform an extension reaction to generate said extension molecule.
102. The method of embodiment 100 or 101, wherein said nucleic acid barcode molecule further comprises a unique molecular identifier (UMI) sequence adjacent to said nucleic acid barcode sequence.
103. The method of embodiment 85-99, wherein said nucleic acid barcode molecule comprises, in said 5' to 3' direction, a second primer sequence, a first primer sequence, said nucleic acid barcode sequence, and a capture sequence.
104. The method of embodiment 103, wherein (c) comprises using a primer molecule to, at said first primer sequence, perform an extension reaction to generate said extension molecule.
105. The method of embodiment 103 or 104, wherein said nucleic acid barcode molecule further comprises a unique molecular identifier (UMI) sequence adjacent to said nucleic acid barcode sequence.
106. The method of any one of embodiments 85-105, wherein (a) is performed in bulk.
107. The method of any one of embodiments 85-105, wherein (a) is performed in a partition.
108. The method of embodiment 107, wherein said partition is a droplet or a well.
109. The method of embodiment 107 or 108, wherein (b) is performed outside of said partition.
110. The method of embodiment 107 or 108, wherein (b) is performed in said partition.
111. The method of any one of embodiments 85-110, wherein said single-stranded nucleic acid barcode molecule comprises a capture sequence complementary to a portion of said nucleic acid molecule.
112. The method of embodiment 111, wherein (a) comprises (i) hybridizing said nucleic acid molecule to said capture sequence and performing a nucleic acid reaction to generate a double-stranded barcoded nucleic acid molecule, and (ii) generating said single-stranded barcoded nucleic acid molecule from said double-stranded barcoded nucleic acid molecule.
113. The method of embodiment 112, wherein said nucleic acid reaction comprises reverse transcription.
114. The method of embodiment 113, wherein said reverse transcription is performed using a reverse transcriptase selected from the group consisting of thermostable group II intron reverse transcriptase (TGIRT), and Marathon reverse transcriptase.
115. The method of any one of embodiments 111-114, wherein said nucleic acid barcode molecule comprises, in said 5' to 3' direction, said barcode sequence, a first primer sequence, a second primer sequence, and said capture sequence.
116. The method of embodiment 115, wherein said nucleic acid barcode molecule comprises, adjacent to said barcode sequence, a unique molecular identifier (UMI) sequence.
117. The method of embodiment 115 or 116, wherein (c) comprises using a primer molecule to, at said second primer sequence, perform an extension reaction to generate said extension molecule.
118. The method of any one of embodiments 111-114, wherein said nucleic acid barcode molecule comprises, in said 5' to 3' direction, a first primer sequence, a second primer sequence, said barcode sequence, and said capture sequence.
119. The method of embodiment 118, wherein said nucleic acid barcode molecule comprises, adjacent to said barcode sequence, a unique molecular identifier (UMI) sequence.
120. A kit, comprising:
   a first nucleic acid barcode molecule comprising, in a 5' to 3' direction, a first barcode sequence, a first primer sequence, and a second primer sequence;
   a second nucleic acid barcode molecule comprising, in a 5' to 3' direction, said second primer sequence, said first primer sequence, and a second barcode sequence;
   a first primer molecule comprising a first sequence complementary to said first primer sequence; and
   a second primer molecule comprising a second sequence complementary to said second primer sequence.
121. The kit of embodiment 120, further comprising a bead, wherein said bead comprises said first nucleic acid barcode molecule and said second nucleic acid barcode molecule coupled thereto.
122. The kit of embodiment 121, wherein said bead comprises a plurality of first nucleic acid barcode molecules and a plurality of second nucleic acid barcode molecules, wherein said plurality of first nucleic acid barcode molecules comprises said first nucleic acid barcode molecule and comprises said first barcode sequence, wherein said plurality of second nucleic acid barcode molecules comprises said second nucleic acid barcode molecule and comprises said second barcode sequence.
123. The kit of embodiment 121 or 122, further comprising a plurality of beads, wherein said plurality of beads comprises said bead, wherein said plurality of beads comprises a plurality of first nucleic acid barcode molecules and a plurality of second nucleic acid barcode molecules coupled thereto.
124. The kit of any one of embodiments 121-123, wherein said first nucleic acid barcode molecule is releasably coupled to said bead.
125. The kit of any one of embodiments 121-124, wherein said first barcode sequence and said second barcode sequence comprises a common sequence that is common to said bead.
126. The kit of embodiment 120, further comprising a first bead coupled to said first nucleic acid barcode molecule and a second bead coupled to said second nucleic acid barcode molecule.
127. The kit of embodiment 126, further comprising a plurality of first beads and a plurality of second beads, wherein said plurality of first beads comprises (i) said first bead and (ii) a plurality of first nucleic acid barcode molecules coupled thereto, wherein said plurality of second beads comprises (i) said second bead and (ii) a plurality of second nucleic acid barcode molecules coupled thereto.
128. The kit of any one of embodiments 120-127, further comprising a plurality of first nucleic acid barcode molecules and a plurality of second nucleic acid barcode molecules, wherein said plurality of first nucleic acid barcode molecules comprises said first nucleic acid barcode molecule and comprises said first barcode sequence, wherein said plurality of second nucleic acid barcode molecules comprises said second nucleic acid barcode molecule and comprises said second barcode sequence.
129. The kit of any one of embodiments 120-127, further comprising a plurality of first primer molecules and a plurality of second primer molecules, wherein said plurality of first primer molecules comprises said first primer molecule, wherein said plurality of second primer molecules comprises said second primer molecule.
130. The kit of any one of embodiments 120-129, wherein said first barcode sequence and said second barcode sequence comprises a common sequence.
131. The kit of embodiment 130, wherein said first nucleic acid barcode molecule further comprises a first unique molecular identifier (UMI) sequence and said second nucleic acid barcode molecule further comprises a second UMI sequence different from said first UMI sequence.
132. The kit of any one of embodiments 120-131, wherein said first nucleic acid barcode molecule and said second nucleic acid barcode molecule comprise, at a 3' end, a capture sequence.
133. The kit of embodiment 132, wherein said capture sequence comprises a polyT sequence.
134. The kit of embodiment 132 or 133, wherein said capture sequence comprises a random Nmer sequence.
135. The kit of any one of embodiments 132-134, wherein said capture sequence comprises a target-specific sequence.
136. The kit of any one of embodiments 132-135, further comprising a feature-binding group comprising a reporter oligonucleotide.
137. The kit of embodiment 136, wherein said capture sequence comprises a sequence configured to couple to said reporter oligonucleotide.
138. The kit of any one of embodiments 120-137, further comprising an enzyme configured to circularize a nucleic acid molecule.
139. The kit of embodiment 138, wherein said enzyme is a single stranded circularization enzyme.
140. The kit of embodiment 139, wherein said enzyme is CircLigase.
141. The kit of any one of embodiments 120-140, further comprising a ligase.
142. The kit of embodiment 141, wherein said ligase is a T4 DNA ligase, T7 DNA Ligase, SplintR, Acanthocystis turfacea chlorella virus-isolated ligase, T4RNA Ligase, KOD RNA Ligase, or a double stranded DNA ligase.
143. The kit of any one of embodiments 120-142, further comprising a splint molecule comprising a splint sequence complementary to a sequence of said first nucleic acid barcode molecule or said second nucleic acid barcode molecule.
144. The kit of any one of embodiments 120-143, further comprising reagents for performing any one or more of: a nucleic acid extension, ligation, and amplification reaction.
145. The kit of any one of embodiments 120-144, further comprising a reverse transcriptase.
146. The kit of embodiment 145, wherein said reverse transcriptase is a thermostable group II intron reverse transcriptase (TGIRT), and Marathon reverse transcriptase.
147. The kit of any one of embodiments 120-146, wherein said first nucleic acid barcode molecule comprises, in said 5' to 3' direction, (i) a common barcode sequence and (ii) a first unique molecular identifier (UMI) sequence, said first primer sequence, said second primer sequence, and said capture sequence.
148. The kit of embodiment 147, wherein said second nucleic acid barcode molecule comprises, in said 5' to 3' direction, said second primer sequence, said first primer sequence, said common barcode sequence and a second UMI sequence different form said first UMI sequence, and said capture sequence.
149. The kit of any one of embodiments 120-148, further comprising instructions for performing any of the methods of embodiments 1-119.
150. A composition, comprising:
   a particle comprising (i) a first nucleic acid barcode molecule comprising, in a 5' to 3' direction, a first barcode sequence, a first primer sequence, and a second primer sequence; and (ii) a second nucleic acid barcode molecule comprising, in a 5' to 3' direction, said second primer sequence, said first primer sequence, and a second barcode sequence.
151. The composition of embodiment 150, wherein said particle is a bead.
152. The composition of embodiment 150 or 151, further comprising a partition, wherein said partition comprises said particle.
153. The composition of embodiment 152, wherein said partition further comprises a reverse transcriptase.
154. The composition of embodiment 153, wherein said reverse transcriptase is a thermostable group II intron reverse transcriptase (TGIRT), and Marathon reverse transcriptase.
155. The composition of any one of embodiments 152-154, wherein said partition further comprises a ligase.
156. The composition of embodiment 155, wherein said ligase is a CircLigase, T4 DNA ligase, T7 DNA Ligase, SplintR, Acanthocystis turfacea chlorella virus-isolated ligase, T4RNA Ligase, KOD RNA Ligase, or a double stranded DNA ligase.
157. The composition of any one of embodiments 152-156, wherein said partition further comprises a splint molecule comprising a splint sequence complementary to a sequence of said first nucleic acid barcode molecule.
158. The composition of any one of embodiments 152-156, wherein said partition further comprises a splint molecule comprising a splint sequence complementary to a sequence of said second nucleic acid barcode molecule.
159. The composition of any one of embodiments 152-158, wherein said partition further comprises a first primer molecule comprising a first sequence complementary to said first primer sequence and a second primer molecule comprising a second sequence complementary to said second primer sequence.
160. The composition of any one of embodiments 152-159, wherein said partition is a droplet or a well.

## Claims

1. A method, comprising:
(a) generating a barcoded molecule using a nucleic acid molecule and a nucleic acid barcode molecule, wherein said barcoded molecule comprises a barcode sequence, and, in a 5' to 3' direction: (i) a first primer sequence, (ii) a second primer sequence, and (iii) a sequence complementary to at least a portion of said nucleic acid molecule;
(b) circularizing said barcoded molecule to generate a circularized molecule; and
(c) generating an extension product from said circularized molecule, wherein said extension product comprises, said barcode sequence or complement thereof, and, in a 5' to 3' direction, a first sequence corresponding to said second primer sequence, a second sequence corresponding to said nucleic acid molecule, and a third sequence corresponding to said first primer sequence.

2. The method of claim 1, wherein, prior to (a), said nucleic acid barcode molecule is provided on a bead, optionally wherein said bead comprises a plurality of nucleic acid barcode molecules coupled thereto, including said nucleic acid barcode molecule, wherein at least a subset of said plurality of nucleic acid barcode molecules comprises a common barcode sequence, optionally wherein at least said subset of said plurality of nucleic acid barcode molecules further comprises a unique molecular identifier (UMI) sequence that differs among said plurality of nucleic acid barcode molecules coupled to said bead.

3. The method of claim 1 or claim 2, wherein said nucleic acid barcode molecule comprises, at a 3' end, a capture sequence configured to hybridize to at least a portion of said nucleic acid molecule.

4. The method of claim 3, wherein said capture sequence comprises: (i) a polyT sequence; (ii) a random Nmer sequence; (iii) a target-specific sequence; and/or (iv) a sequence configured to couple to a reporter oligonucleotide of a feature-binding group.

5. The method of claim 3 or claim 4, wherein (a) comprises capturing said nucleic acid molecule using said capture sequence, and extending said nucleic acid barcode molecule to generate said barcoded molecule.

6. The method of any one of claims 1-5, wherein said barcoded molecule is single stranded.

7. The method of any one of claims 1-6, wherein (b) is performed using a single stranded circularization enzyme, optionally wherein said circularization enzyme is CircLigase.

8. The method of any one of claims 1-7, wherein (b) is performed using a ligase, optionally wherein said ligase is a T4 DNA ligase, T7 DNA Ligase, SplintR, Acanthocystis turfacea chlorella virus-isolated ligase, T4 RNA Ligase, KOD RNA Ligase, or a double stranded DNA ligase.

9. The method any one of claims 1-8, wherein (b) is performed using a splint molecule, wherein said splint molecule comprises (i) a first splint sequence complementary to a 5' end of said barcoded molecule and (ii) a second splint sequence complementary to a 3' end of said barcoded molecule.

10. The method of any one of claims 1-9, wherein said nucleic acid barcode molecule comprises, in said 5' to 3' direction, said barcode sequence, a unique molecular identifier (UMI) sequence, said first primer sequence, said second primer sequence, and a capture sequence, optionally wherein (c) comprises using a primer molecule to, at said second primer sequence, perform an extension reaction to generate said extension molecule.

11. The method of any one of claims 1-9, wherein said nucleic acid barcode molecule comprises, in said 5' to 3' direction, said first primer sequence, said second primer sequence, said barcode sequence, a unique molecular identifier (UMI) sequence, and a capture sequence, optionally wherein (c) comprises using a primer molecule to, at said second primer sequence, perform an extension reaction to generate said extension molecule.

12. The method of any one of claims 1-11, wherein (a) is performed in bulk.

13. The method of any one of claims 1-11, wherein (a) is performed in a partition, optionally wherein said partition is a droplet or a well.

14. The method of claim 13, wherein (b) is performed outside of said partition.

15. The method of claim 13, wherein (b) is performed in said partition.
